(19) Europäisches Patentamt
European Patent Office
Office européen des brevets

(11) EP 0 888 351 B1

(12) **EUROPÄISCHE PATENTSCHRIFT**

(45) Veröffentlichungstag und Bekanntmachung des Hinweises auf die Patenterteilung:
**15.10.2003 Patentblatt 2003/42**

(21) Anmeldenummer: **97906152.0**

(22) Anmeldetag: **03.03.1997**

(51) Int Cl.$^7$: **C07D 487/04**, A61K 31/505

(86) Internationale Anmeldenummer:
**PCT/EP97/01047**

(87) Internationale Veröffentlichungsnummer:
**WO 97/032880 (12.09.1997 Gazette 1997/39)**

(54) **PYRIMIDO 5,4-d]PYRIMIDINE, DIESE VERBINDUNGEN ENTHALTENDE ARZNEIMITTEL, DEREN VERWENDUNG UND VERFAHREN ZU IHRER HERSTELLUNG**

PYRIMIDO 5,4-D]PYRIMIDINES, MEDICAMENTS CONTAINING THESE COMPOUNDS, THEIR USE AND PROCESS FOR THEIR PRODUCTION

PYRIMIDO 5,4-d]PYRIMIDINES, MEDICAMENTS CONTENANT CES COMPOSES, LEUR UTILISATION ET PROCEDE DE FABRICATION ASSOCIE

(84) Benannte Vertragsstaaten:
**AT BE CH DE DK ES FI FR GB GR IE IT LI LU MC NL PT SE**
Benannte Erstreckungsstaaten:
**LT LV SI**

(30) Priorität: **06.03.1996 DE 19608588**

(43) Veröffentlichungstag der Anmeldung:
**07.01.1999 Patentblatt 1999/01**

(73) Patentinhaber: **Dr. Karl Thomae GmbH**
**88397 Biberach (DE)**

(72) Erfinder:
- **HIMMELSBACH, Frank**
  **D-88441 Mittelbiberach (DE)**
- **DAHMANN, Georg**
  **D-88444 Ummendorf (DE)**
- **VON RÜDEN, Thomas**
  **A-2500 Baden (AT)**
- **METZ, Thomas**
  **A-1010 Wien (AT)**

(74) Vertreter: **Laudien, Dieter, Dr.**
**Boehringer Ingelheim GmbH**
**Abteilung Patente**
**55216 Ingelheim (DE)**

(56) Entgegenhaltungen:
**EP-A- 0 055 444** **DE-A- 4 431 867**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 888 351 B1

## Beschreibung

In der nicht vorveröffentlichten PCT/EP95/03482-Anmeldung werden bereits Pyrimido[5,4-d]pyrimidine der allgemeinen Formel

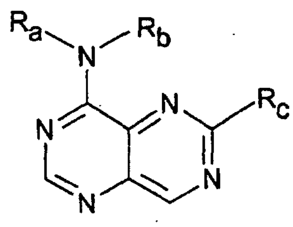

, (I)

deren Tautomeren, deren Stereoisomere und deren Salze, insbesonders deren physiologisch verträgliche Salze mit anorganischen oder organischen Säuren oder Basen, beschrieben, welche wertvolle pharmakologische Eigenschaften aufweisen, insbesondere eine Hemmwirkung auf die durch Tyrosinkinasen vermittelte Signaltransduktion, deren Verwendung zur Behandlung von Krankheiten, insbesondere von Tumorerkrankungen, und deren Herstellung.

Es wurde nun gefunden, daß weitere Pyrimido[5,4-d]pyrimidine der obigen allgemeinen Formel I die gleichen wertvollen pharmakologische Eigenschaften aufweisen.

Gegenstand der vorliegenden Erfindung sind somit die neuen Pyrimido[5,4-d]pyrimidine der obigen allgemeinen Formel I, in der

$R_a$ ein Wasserstoffatom,

$R_b$ eine 3-Chlor-phenyl-, 3-Chlor-4-fluor-phenyl-, 3-(1,1,2,2-Tetrafluorethoxy)-phenyl-, 4-Amino-3,5-dibromphenyl-, 4-Amino-3,5-dichlorphenyl-, 4-(Benzyl)phenyl-, 3-(Benzyloxy)phenyl-, 4-(Benzyloxy)phenyl-, 3-(Hydroxymethyl)-phenyl-, 4-Biphenylyl-, 3-Phenoxyphenyl-, 4-Phenoxy-phenyl-, 3-Trifluormethoxyphenyl-, 3-Cyano-phenyl-, 3-Trifluormethylphenyl-, 3,4-Difluorphenyl-, 3-Nitrophenyl-, 3-Ethinylphenyl-, 4-Amino-3-nitrophenyl-, 4-Chlor-3-nitrophenyl-, 4-(Benzyloxy)-3-chlor-phenyl-,3-Chlor-4-phenoxy-phenyl-, 3-Chlor-4-cyanophenyl- oder 4-Chlor-3-cyano-phenylgruppe

$R_c$ eine in 4-Position durch eine 4-Hydroxyphenylgruppe und zusätzlich in 2-Position durch eine Methylgruppe substituierte 1-Pyrrolidinylgruppe,

eine 1-Piperidinylgruppe, die gegebenenfalls in der 4-Position durch eine Amino-, Methylamino-, Hydroxy-, Formylamino-, Methoxycarbonylamino-, N-Methyl-N-methylsulfonyl-amino-, Aminomethyl-, Morpholino-, 1-Pyrrolidinyl-, 1-Piperazinyl, 1-Methyl-4-piperazinyl-, (1-Methyl-4-piperazinyl)-methyl, 4-Dimethylamino-1-piperidinyl-, 4-Piperidinyl- oder 1-Methyl-4-piperidinylgruppe substituiert ist,

eine 4-Amino-3-methyl-1-piperidinylgruppe,

eine 4-Amino-4-methyl-1-piperidinylgruppe,

eine in der 3-Position durch eine Aminomethyl-, Aminocarbonyloder Aminocarbonylmethyl-gruppe substituierte 1-Piperidinylgruppe,

eine 1-Azacycloheptyl- oder 4-Amino-1-azacycloheptylgruppe,

eine Morpholinogruppe,

eine in 4-Position durch eine 2-Methoxyphenyl-, 3-Methoxyphenyl- oder 4-Methoxyphenylgruppe substituierte 1-Piperazinylgruppe,

eine 1-Homopiperazinyl- oder 4-Methyl-1-homopiperazinylgruppe,

eine in 3-Position durch eine Amino- oder Acetylaminogruppe substituierte 8-Aza-bicyclo[3.2.1]-8-octylgruppe oder

eine ($R_4NR_5$)-Gruppe, in der

$R_4$ ein Wasserstoffatom, eine Methyl- oder Ethylgruppe,

$R_5$ ein Wasserstoffatom,

eine Methylgruppe, die durch eine 3-Tetrahydrofuryl-, 4-Piperidinyl-, 1-Methyl-4-piperidinyl-, 1-tert.Butyloxycarbonyl-4-piperidinyl- oder 4-Chinuclidinylgruppe substituiert ist,

eine Ethylgruppe, die in 2-Position durch eine Hydroxy-, Amino-, 4-tert.Butyloxycarbonyl-1-piperazinyl- oder 4-(Morpholinocarbonyl)-1-piperazinylgruppe substituiert ist,

eine 2,2-Dimethoxy-ethylgruppe,

eine 1-Propylgruppe, die in 2-Position durch eine Aminogruppe und gegebenenfalls zusätzlich in 2-Position durch eine Methylgruppe substituiert ist,

eine 1-Propylgruppe, die in 3-Position durch eine Aminogruppe substituiert ist,

eine 2-Propylgruppe, die in 1-Position durch eine Phenoxy-, 4-Aminophenyl-, 1-Piperidinyl- oder Diethylaminogruppe substituiert ist,

eine 2-Propylgruppe, die in 1-Position durch eine Aminogruppe und zusätzlich in 2-Position durch eine Methylgruppe substituiert ist,

eine 4-Amino-butyl- oder eine 5-Aminopentylgruppe,

eine Cyclohexylgruppe, die in 4-Position durch eine Hydroxy-, Dimethylamino-, 2-Methyl-4-piperazinyl-, 1-Piperazinyl-carbonyl-, 1-Methyl-4-piperazinyl-carbonyl-, 4-Dimethylamino-1-piperidinyl-,Carboxy-, Morpholinocarbonyl-, (1-Pyrrolidinyl)carbonyl-, Methoxycarbonyl-, Aminomethyl-, Methylamino-, Methoxycarbonylamino-, 2-(Morpholinocarbonyl)ethyl- oder 2-(1-Pyrrolidinylcarbonyl)ethyl-gruppe substituiert ist,

eine Cyclohexylmethylgruppe, die im Cyclohexylteil in 4-Position durch eine Amino-, Aminomethyl- oder Benzyloxycarbonylaminogruppe oder in 3-Position durch eine Aminomethylgruppe substituiert ist,

eine 1-Methyl-3-piperidinylgruppe,

eine 4-Piperidinylgruppe, die in der 1-Position durch eine Cyano-, Methyl-, tert.Butyloxycarbonyl-, (N,N-Dimethylamino)carbonyl- oder Methoxycarbonylgruppe substituiert ist,

eine 4-Aminobenzylgruppe,

eine 3-Chinuclidinyl-, 1-Benzyl-4-(azacycloheptyl)-, 1-tert.Butyloxycarbonyl-4-(azacycloheptyl)- oder 4-(Azacycloheptyl)gruppe darstellen,

mit der Maßgabe bedeuten, daß die Verbindungen
4-[(4-Amino-3,5-dibrom-phenyl)amino]-6-[(trans-4-hydroxy-cyclohexyl)amino]-pyrimido[5,4-d]pyrimidin,
4-[(4-Amino-3,5-dichlor-phenyl)amino]-6-[(trans-4-hydroxycyclohexyl)-amino]-pyrimido[5,4-d]pyrimidin,
4-[(4-Amino-3-nitro-phenyl)amino]-6-[(trans-4-hydroxycyclohexyl)-amino]-pyrimido[5,4-d]pyrimidin,
4-[(4-Amino-3-nitrophenyl)amino]-6-(morpholino)-pyrimido[5,4-d]pyrimidin,
4-[(4-Chlor-3-nitro-phenyl)amino]-6-[(trans-4-hydroxycyclohexyl)-amino]-pyrimido[5,4-d]pyrimidin,
sowie die Verbindungen, in denen
die $R_aNR_b$-Gruppe eine (3-Chlorphenyl)amino-, (3-Nitrophenyl)-amino- oder (3-Ethinylphenyl)aminogruppe darstellt, wenn $R_c$ gleichzeitig eine Tetrahydrofurfurylamino-, Morpholino-, 1-Methyl-4-piperidinylamino-, N-(4-Hydroxy-cyclohexyl)-N-methylamino-, 4-Hydroxy-cyclohexylamino-, 4-Dimethylamino-cyclohexylamino-, trans-4-Carboxy-cyclohexylamino-, trans-4-(1-Pyrrolidinyl)carbonyl-cyclohexylamino- oder trans-4-Morpholinocarbonyl-cyclohexylaminogruppe darstellt,
und die Verbindungen, in denen

die $R_aNR_b$-Gruppe eine (3-Chlor-4-fluor-phenyl)aminogruppe darstellt, wenn $R_c$ gleichzeitig eine 1-Methyl-3-piperidinylamino-, Tetrahydrofurfurylamino-, 3-(Methoxycarbonylamino)-1-propylamino-, N-Methyl-N-(2-hydroxyethyl)amino-, 4-Amino-1-piperidinyl-, Morpholino-, 1-Methyl-4-piperidinylamino-, 4-Hydroxy-cyclohexylamino-, 4-Dimethylamino-cyclohexylamino-, N-(4-Hydroxy-cyclohexyl)-N-methylamino-, trans-4-Carboxycyclohexylamino-, trans-4-(2-(Morpholinocarbonyl)-ethyl)-cyclohexylamino-, trans-4-(1-Pyrrolidinyl)carbonyl-cyclohexylaminooder trans-4-Morpholinocarbonyl-cyclohexylaminogruppe darstellt, ausgenommen sind,
deren Tautomeren, deren Stereoisomere und deren Salze,
diese Verbindungen enthaltende Arzneimittel, deren Verwendung zur Behandlung von Krankheiten, insbesondere von Tumorerkrankungen, und deren Herstellung.

Beispielsweise seien folgende besonders wertvolle Verbindungen der allgemeinen Formel I erwähnt:

4-[(3-Chlor-4-fluor-phenyl)amino]-6-[4-methoxycarbonylamino-1-piperidinyl]-pyrimido[5,4-d]pyrimidin,

4-[(3-Chlor-4-fluor-phenyl)amino]-6-[(3-chinuclidinyl)amino]-pyrimido[5,4-d]pyrimidin,

4-[(3-Chlor-4-fluor-phenyl)amino]-6-[4-formylamino-1-piperidinyl)-pyrimido[5,4-d]pyrimidin,

4-[(3-Chlor-4-fluor-phenyl)amino]-6-[4-(aminomethyl)-1-piperidinyl]-pyrimido[5,4-d]pyrimidin,

4-[(3-Chlor-4-fluor-phenyl)amino]-6-[1-methoxycarbonyl-4-piperidinyl-amino]-pyrimido[5,4-d]pyrimidin,

4-[(3-Chlor-4-fluor-phenyl)amino]-6-[3-amino-propylamino]-pyrimido[5,4-d]pyrimidin,

4-[(3-Chlor-4-fluor-phenyl)amino]-6-[4-amino-benzylamino]-pyrimido[5,4-d]pyrimidin,

4-[(4-Amino-3,5-dichlor-phenyl)amino]-6-[trans-4-(morpholinocarbonyl)cyclohexylamino]-pyrimido[5,4-d]pyrimidin,

4-[(4-Amino-3,5-dichlor-phenyl)amino]-6-[trans-4-(pyrrolidinocarbonyl)cyclohexylamino]-pyrimido[5,4-d]pyrimidin,

4-[(3-Chlor-4-fluor-phenyl)amino]-6-(4-(4-piperidinyl)-1-piperidinyl]-pyrimido[5,4-d]pyrimidin,

4-[(3-Chlor-4-fluor-phenyl)amino]-6-[4-(aminomethyl)-cyclohexyl-methyl-amino] -pyrimido[5,4-d]pyrimidin,

4-[(3-Chlor-4-fluor-phenyl)amino)-6-[4-chinuclidinyl-methylamino]-pyrimido[5,4-d]pyrimidin,

4-[(3-Chlor-4-fluor-phenyl)amino]-6-[2-amino-2-methyl-1-propylamino]-pyrimido[5,4-d]pyrimidin,

4-[(3-Chlor-4-fluor-phenyl)amino]-6-[N-methyl-N-(1-methyl-4-piperidinyl)-amino]-pyrimido[5,4-d]pyrimidin,

4-[(3-Chlor-4-fluor-phenyl)amino]-6-[4-piperidinyl-methyl-amino]-pyrimido[5,4-d]pyrimidin,

4-[(3-Chlor-4-fluor-phenyl)amino]-6-[4-(morpholino)-1-piperidinyl]-pyrimido[5,4-d]pyrimidin,

4-[(3-Chlor-4-fluor-phenyl)amino]-6-[4-(1-pyrrolidinyl)-1-piperidinyl]-pyrimido[5,4-d]pyrimidin,

4-[(3-Chlor-4-fluor-phenyl)amino]-6-[3-(aminomethyl)cyclohexylmethyl-amino]-pyrimido[5,4-d]pyrimidin,

4-[(3-Chlor-4-fluor-phenyl)amino]-6-[4-(1-methyl-4-piperidinyl)-1-piperidinyl)-pyrimido[5,4-d]pyrimidin,

4-[(3-Chlor-4-fluor-phenyl)amino]-6-[4-(1-methyl-4-piperazinyl)-cyclohexylamino]-pyrimido[5,4-d]pyrimidin,

4-[(3-Chlor-4-fluor-phenyl)aminol-6-[4-hydroxy-1-piperidinyl]-pyrimido[5,4-d]pyrimidin,

4-[(3-Chlor-4-fluor-phenyl)amino]-6-[(1-cyano-4-piperidinyl)-amino]-pyrimido[5,4-d]pyrimidin,

4-[(3-Chlor-4-fluor-phenyl)amino]-6-[(1-methyl-4-piperidinyl)-methylamino]-pyrimido[5,4-d]pyrimidin,

4-[(3-Chlor-4-fluor-phenyl)amino]-6-[2-(4-(morpholinocarbonyl)-1-piperazinyl)-ethylamino]-pyrimido[5,4-d]pyrimidin,

4-[(3-Chlor-4-fluor-phenyl)amino]-6-[4-amino-1-azacycloheptyl]-pyrimido[5,4-d]pyrimidin,

4-[(3-Chlor-4-fluor-phenyl)amino]-6-[4-(azacycloheptyl)-amino]-pyrimido[5,4-d]pyrimidin,

4-[(3-Chlor-4-fluor-phenyl)amino]-6-[trans-4-aminomethyl-cyclohexylamino]-pyrimido[5,4-d]pyrimidin,

4-[(3-Chlor-4-fluor-phenyl)amino]-6-[3-aminomethyl-1-piperidinyl]-pyrimido[5,4-d]pyrimidin,

4-[(3-Chlor-4-fluor-phenyl)amino]-6-[4-(1-methyl-4-piperidinyl)-1-piperidinyl]-pyrimido[5,4-d]pyrimidin,

4-[(3-Chlor-4-fluor-phenyl)amino]-6-[4-amino-4-methyl-1-piperidinyl]-pyrimido[5,4-d]pyrimidin,

4-[(3-Chlor-4-fluor-phenyl)amino]-6-[endo-3-acetylamino-8-azabicyclo[3.2.1]-8-octyl]-pyrimido[5,4-d]pyrimidin,

4-[(3-Chlor-4-fluor-phenyl)amino]-6-[4-(4-amino-1-piperidinyl)-methyl-1-piperidinyl]-pyrimido[5,4-d]pyrimidin,

4-[(4-Benzyloxy-phenyl)amino]-6-[trans-4-dimethylamino-cyclohexylamino]-pyrimido[5,4-d]pyrimidin,

(3'S)-4-[3-Chlor-phenylamino]-6-[(3'-chinuclidinyl)amino]pyrimido[5,4-d]pyrimidin

4-[(3-Chlor-4-fluor-phenyl)amino]-6-[1-dimethylaminocarbonyl-4-piperidinylamino]-pyrimido[5,4-d]pyrimidin,

(3'S)-4-[(3-Chlor-4-fluor-phenylamino]-6-[(3'-chinuclidinyl)amino]pyrimido[5,4-d]pyrimidin,

(3'R)-4-[(3-Chlor-4-fluor-phenyl)amino]-6-[(3'-chinuclidinyl)-amino]pyrimido[5,4-d]pyrimidin,

4-[(3-Chlor-4-fluor-phenyl)amino]-6-[1-methyl-4-piperidinylamino]-pyrimido[5,4-d]pyrimidin

4-[(3-Chlor-4-fluor-phenyl)amino]-6-[(4-dimethylaminocyclohexyl)amino]-pyrimido[5,4-d)pyrimidin

und deren Salze.

Die Verbindungen der allgemeinen Formel I lassen sich beispielsweise nach folgenden Verfahren herstellen: a) Umsetzung einer Verbindung der allgemeinen Formel

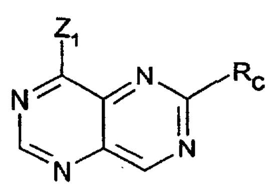

, (II)

in der

$R_c$ wie eingangs definiert ist und
$Z_1$ eine Austrittsgruppe wie ein Halogenatom, z.B. ein Chloroder Bromatom oder eine Methylsulfonyl- oder eine Hydroxygruppe darstellt, mit einem Amin der allgemeinen Formel

$$H\text{-}(R_aNR_b)\,, \qquad (III)$$

in der
$R_a$ und $R_b$ wie eingangs definiert sind.

Die Umsetzung wird zweckmäßigerweise in einem Lösungsmittel wie Isopropanol, Butanol, Tetrahydrofuran, Dioxan, Toluol, Chlorbenzol, Dimethylformamid, Dimethylsulfoxid, Ethylenglycolmonomethylether, Ethylenglycoldiethylether oder Sulfolan gegebenenfalls in Gegenwart einer anorganischen Base, z.B. Natriumcarbonat oder Kaliumhydroxid, oder einer tertiären organischen Base, z.B. Triethylamin oder Pyridin, wobei letztere gleichzeitig auch als Lösungsmittel dienen können, und gegebenenfalls in Gegenwart eines Reaktionsbeschleunigers wie eines Kupfersalzes, eines entsprechenden Amin-hydrohalogenids oder Alkalihalogenids bei Temperaturen zwischen 0 und 200°C, vorzugsweise jedoch bei Temperaturen zwischen 60 und 150°C, durchgeführt. Die Umsetzung kann jedoch auch ohne Lösungsmittel oder in einem Überschuß der eingesetzten Verbindung der allgemeinen Formel III durchgeführt werden.

Bedeuted $Z_1$ eine Hydroxygruppe, so wird die Umsetzung zweckmäßigerweise in Gegenwart von Hexamethyldisilazan, vorzugsweise ohne weitere Lösungsmittel und gegebenenfalls in Gegenwart eines Reaktionsbeschleunigers wie einer organischen Säure wie z.B. Toluolsulfonsäure bei Temperaturen zwischen 0 und 200°C, vorzugsweise jedoch bei Temperaturen zwischen 60 und 180°C, durchgeführt.

b) Zur Herstellung von Verbindungen der allgemeinen Formel I, in der $R_c$ einen der für $R_c$ eingangs erwähnten über ein Sauerstoff- oder Stickstoffatom mit dem Pyrimido[5,4-d]pyrimidin verknüpften Reste darstellt:

Umsetzung einer Verbindung der allgemeinen Formel

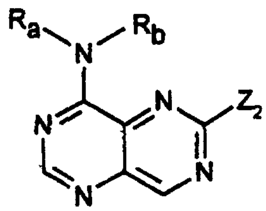
, (IV)

in der

$R_a$ und $R_b$ wie eingangs definiert sind und

$Z_2$ eine Austrittsgruppe wie ein Halogenatom, eine substituierte Hydroxy-, Mercapto-, Sulfinyl- oder Sulfonylgruppe wie ein Chlor- oder Bromatom, eine Methoxy-, Ethoxy-, Phenoxy-, Methylsulfinyl-, Ethylsulfinyl-, Methylsulfonyl- oder Ethylsulfonylgruppe darstellt, mit einer Verbindung der allgemeinen Formel

$$H - R_c ,\qquad (V)$$

in der

$R_c$ die für $R_c$ eingangs erwähnten über ein Sauerstoff- oder Stickstoffatom mit dem Pyrimido[5,4-d]pyrimidin verknüpften Reste darstellt.

Die Umsetzung wird zweckmäßigerweise in einem Lösungsmittel wie Isopropanol, Butanol, Tetrahydrofuran, Dioxan, Toluol, Chlorbenzol, Dimethylformamid, Dimethylsulfoxid, Ethylenglycolmonomethylether, Ethylenglycoldiethylether oder Sulfolan gegebenenfalls in Gegenwart einer anorganischen Base, z.B. Natriumcarbonat oder Kaliumhydroxid, oder einer tertiären organischen Base, z.B. Triethylamin oder Pyridin, wobei letztere gleichzeitig auch als Lösungsmittel dienen können, und gegebenenfalls in Gegenwart eines Reaktionsbeschleunigers wie eines Kupfersalzes, eines entsprechenden Amin-hydrohalogenids oder Alkalihalogenids bei Temperaturen zwischen 0 und 150°C, vorzugsweise jedoch bei Temperaturen zwischen 20 und 120°C, durchgeführt. Die Umsetzung kann jedoch auch ohne Lösungsmittel oder in einem Überschuß der eingesetzten Verbindung der allgemeinen Formel V durchgeführt werden.

Mit einem Alkohol der allgemeinen Formel V wird die Umsetzung vorzugsweise in einem entsprechenden Alkohol und gegebenenfalls in Gegenwart einer organischen oder anorganischen Base wie mit dem entsprechenden Alkalimetallalkoholat bei Temperaturen zwischen 0 und 100°C durchgeführt.

Erhält man erfindungsgemäß eine Verbindung der allgemeinen Formel I, die eine Amino-, Alkylamino- oder Iminogruppe enthält, so kann diese mittels Acylierung oder Sulfonylierung in eine entsprechende Acyl- oder Sulfonylverbindung der allgemeinen Formel I übergeführt werden oder

eine Verbindung der allgemeinen Formel I, die eine Amino-, Alkylamino- oder Iminogruppe enthält, so kann diese mittels Alkylierung oder reduktiver Alkylierung in eine entsprechende Alkylverbindung der allgemeinen Formel I übergeführt werden oder

eine Verbindung der allgemeinen Formel I, die eine Carboxygruppe enthält, so kann diese mittels Veresterung in einen entsprechenden Ester der allgemeinen Formel I übergeführt werden oder

eine Verbindung der allgemeinen Formel I, die eine Carboxyoder Estergruppe enthält, so kann diese mittels Amidierung in ein entsprechendes Amid der allgemeinen Formel I übergeführt werden oder

eine Verbindung der allgemeinen Formel I, die eine primäre oder sekundäre Hydroxygruppe enthält, so kann diese mittels Oxidation in eine entsprechende Carbonylverbindung der allgemeinen Formel I übergeführt werden.

Die nachträgliche Veresterung wird gegebenenfalls in einem Lösungsmittel oder Lösungsmittelgemisch wie Methylenchlorid, Dimethylformamid, Benzol, Toluol, Chlorbenzol, Tetrahydrofuran, Benzol/Tetrahydrofuran oder Dioxan oder besonders vorteilhaft in einem entsprechenden Alkohol gegebenenfalls in Gegenwart einer Säure wie Salzsäure oder in Gegenwart eines wasserentziehenden Mittels, z.B. in Gegenwart von Chlorameisensäureisobutylester, Thionylchlorid, Trimethylchlorsilan, Schwefelsäure, Methansulfonsäure, p-Toluolsulfonsäure, Phosphortrichlorid, Phosphorpentoxid, N.N'-Dicyclohexylcarbodiimid, N,N'-Dicyclohexylcarbodiimid/N-Hydroxysuccinimid oder 1-Hydroxy-benztriazol und gegebenenfalls zusätzlich in Gegenwart von 4-Dimethylamino-pyridin, N.N'-Carbonyldiimidazol oder Triphenyl-phosphin/Tetrachlorkohlenstoff, zweckmäßigerweise bei Temperaturen zwischen 0 und 150°C, vorzugsweise bei Temperaturen zwischen 0 und 80°C, durchgeführt.

Die nachträgliche Acylierung oder Sulfonylierung wird gegebenenfalls in einem Lösungsmittel oder Lösungsmittelgemisch wie Methylenchlorid, Dimethylformamid, Benzol, Toluol, Chlorbenzol, Tetrahydrofuran, Benzol/Tetrahydrofuran oder Dioxan mit einem entsprechenden Acyl- oder Sulfonylderivat gegebenenfalls in Gegenwart einer tertiären organischen Base oder in Gegenwart einer anorganischen Base oder in Gegenwart eines wasserentziehenden Mittels, z.B. in Gegenwart von Chlorameisensäureisobutylester, Thionylchlorid, Trimethylchlorsilan, Schwefelsäure, Methansulfonsäure, p-Toluolsulfonsäure, Phosphortrichlorid, Phosphorpentoxid, N,N'-Dicyclohexylcarbodiimid, N.N'-Dicyclohexylcarbodiimid/N-Hydroxysuccinimid oder 1-Hydroxy-benztriazol und gegebenenfalls zusätzlich in Gegenwart von 4-Dimethylamino-pyridin, N,N'-Carbonyldiimidazol oder Triphenylphosphin/Tetrachlorkohlenstoff, zweckmäßigerweise bei Temperaturen zwischen 0 und 150°C, vorzugsweise bei Temperaturen zwischen 0 und 80°C, durchgeführt.

Die nachträgliche Alkylierung wird gegebenenfalls in einem Lösungsmittel oder Lösungsmittelgemisch wie Methylenchlorid, Dimethylformamid, Benzol, Toluol, Chlorbenzol, Tetrahydrofuran, Benzol/Tetrahydrofuran oder Dioxan mit einem Alkylierungsmittel wie einem entsprechenden Halogenid oder Sulfonsäureester, z.B. mit Methyljodid, Ethylbromid, Dimethylsulfat oder Benzylchlorid, gegebenenfalls in Gegenwart einer tertiären organischen Base oder in Gegenwart einer anorganischen Base zweckmäßigerweise bei Temperaturen zwischen 0 und 150°C, vorzugsweise bei Temperaturen zwischen 0 und 100°C, durchgeführt.

Die nachträgliche reduktive Alkylierung wird mit einer entsprechenden Carbonylverbindung wie Formaldehyd, Acetaldehyd, Propionaldehyd, Aceton oder Butyraldehyd in Gegenwart eines komplexen Metallhydrids wie Natriumborhydrid, Lithiumborhydrid oder Natriumcyanoborhydrid zweckmäßigerweise bei einem pH-Wert von 6-7 und bei Raumtemperatur oder in Gegenwart eines Hydrierungskatalysators, z.B. mit Wasserstoff in Gegenwart von Palladium/Kohle, bei einem Wasserstoffdruck von 1 bis 5 bar durchgeführt. Die Methylierung wird jedoch vorzugsweise in Gegenwart von Ameisensäure als Reduktionsmittel bei erhöhten Temperaturen, z.B. bei Temperaturen zwischen 60 und 120°C, durchgeführt.

Die nachträgliche Amidierung wird durch Umsetzung eines entsprechenden reaktionsfähigen Carbonsäurederivates mit einem entsprechenden Amin gegebenenfalls in einem Lösungsmittel oder Lösungsmittelgemisch wie Methylenchlorid, Dimethylformamid, Benzol, Toluol, Chlorbenzol, Tetrahydrofuran, Benzol/Tetrahydrofuran oder Dioxan, wobei das eingesetzte Amin gleichzeitig als Lösungsmittel dienen kann, gegebenenfalls in Gegenwart einer tertiären organischen Base oder in Gegenwart einer anorganischen Base oder mit einer entsprechenden Carbonsäure in Gegenwart eines wasserentziehenden Mittels, z.B. in Gegenwart von Chlorameisensäureisobutylester, Thionylchlorid, Trimethylchlorsilan, Schwefelsäure, Methansulfonsäure, p-Toluolsulfonsäure, Phosphortrichlorid, Phosphorpentoxid, O-(Benzotriazol-1-yl)-N,N,N',N'-tetramethyluronium-tetrafluoroborat, N,N'-Dicyclohexylcarbodiimid, N.N'-Dicyclohexylcarbodiimid/N-Hydroxysuccinimid oder 1-Hydroxy-benztriazol und gegebenenfalls zusätzlich in Gegenwart von 4-Dimethylamino-pyridin, N.N'-Carbonyldiimidazol oder Triphenylphosphin/Tetrachlorkohlenstoff, zweckmäßigerweise bei Temperaturen zwischen 0 und 150°C, vorzugsweise bei Temperaturen zwischen 0 und 80°C, durchgeführt.

Die nachträgliche Oxidation wird gegebenenfalls in einem Lösungsmittel wie Methylenchlorid, Wasser, Dimethylformamid, Benzol, Chlorbenzol, Tetrahydrofuran oder Dioxan mit einem Oxidationsmittel wie Chromschwefelsäure, Chromtrioxid und Pyridin, Pyridiniumdichromat, Pyridiniumchlorochromat, Oxalylchlorid/Dimethylsulfoxid/Triethylamin, Tetra-n-propylperruthenat/N-Methylmorpholin-N-oxid, Rutheniumtrichlorid/Natriummetaperiodat oder Dess-Martin-Reagenz zweckmäßigerweise bei Temperaturen zwischen -80 und 100°C, vorzugsweise bei Temperaturen zwischen -80°C und Raumtemperatur, durchgeführt.

Bei den vorstehend beschriebenen Umsetzungen können gegebenenfalls vorhandene reaktive Gruppen wie Hydroxy-, Carboxy-, Amino-, Alkylamino- oder Iminogruppen während der Umsetzung durch übliche Schutzgruppen geschützt werden, welche nach der Umsetzung wieder abgespalten werden.

Beispielsweise kommt als Schutzrest für eine Hydroxygruppe die Trimethylsilyl-, Acetyl-, Benzoyl-, Methyl-,

Ethyl-, tert.Butyl-, Trityl-, Benzyl- oder Tetrahydropyranylgruppe,
als Schutzreste für eine Carboxygruppe die Trimethylsilyl-, Methyl-, Ethyl-, tert.Butyl-, Benzyl- oder Tetrahydropyranylgruppe,
als Schutzreste für eine Phosphonogruppe eine Alkylgruppe wie die Methyl-, Ethyl-, Isopropyl- oder n-Butylgruppe, die Phenyloder Benzylgruppe,
als Schutzreste für eine Amino-, Alkylamino- oder Iminogruppe die Formyl-, Acetyl-, Trifluoracetyl-, Ethoxycarbonyl-, tert.-Butoxycarbonyl-, Benzyloxycarbonyl-, Benzyl-, Methoxybenzyl- oder 2,4-Dimethoxybenzylgruppe und für die Aminogruppe zusätzlich die Phthalylgruppe und
als Schutzreste für das Stickstoffatom einer 1-Aza-bicycloalkylgruppe wie der Chinuclidinylgruppe die Benzylgruppe oder Boran in Betracht.

Die gegebenenfalls anschließende Abspaltung eines verwendeten Schutzrestes erfolgt beispielsweise hydrolytisch in einem wässrigen Lösungsmittel, z.B. in Wasser, Isopropanol/Wasser, Essigsäure/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Natriumhydroxid oder Kaliumhydroxid oder aprotisch, z.B. in Gegenwart von Jodtrimethylsilan, bei Temperaturen zwischen 0 und 120°C, vorzugsweise bei Temperaturen zwischen 10 und 100°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem geeigneten Lösungsmittel wie Methanol, Ethanol, Essigsäureethylester oder Eisessig gegebenenfalls unter Zusatz einer Säure wie Salzsäure bei Temperaturen zwischen 0 und 100°C, vorzugsweise jedoch bei Temperaturen zwischen 20 und 60°C, und bei einem Wasserstoffdruck von 1 bis 7 bar, vorzugsweise jedoch von 3 bis 5 bar. Die Abspaltung eines 2,4-Dimethoxybenzylrestes erfolgt jedoch vorzugsweise in Trifluoressigsäure in Gegenwart von Anisol.

Die Abspaltung eines tert.-Butyl- oder tert.-Butyloxycarbonylrestes erfolgt vorzugsweise durch Behandlung mit einer Säure wie Trifluoressigsäure oder Salzsäure oder durch Behandlung mit Jodtrimethylsilan gegebenenfalls unter Verwendung eines Lösungsmittels wie Methylenchlorid, Dioxan, Methanol oder Diethylether.

Die Abspaltung eines Trifluoracetylrestes erfolgt vorzugsweise durch Behandlung mit einer Säure wie Salzsäure gegebenenfalls in Gegenwart eines Lösungsmittels wie Essigsäure bei Temperaturen zwischen 50 und 120°C oder durch Behandlung mit Natronlauge gegebenenfalls in Gegenwart eines Lösungsmittels wie Tetrahydrofuran bei Temperaturen zwischen 0 und 50°C.

Die Abspaltung eines Phthalylrestes erf olgt vorzugsweise in Gegenwart von Hydrazin oder eines primären Amins wie Methylamin, Ethylamin oder n-Butylamin in einem Lösungsmittel wie Methanol, Ethanol, Isopropanol, Toluol/Wasser oder Dioxan bei Temperaturen zwischen 20 und 50°C.

Die Spaltung des Komplexes einer 1-Aza-bicycloalkylgruppe wie der Chinuclidinylgruppe mit Boran erfolgt vorzugsweise durch Behandlung mit einer Säure wie Salzsäure und gegebenenfalls in Gegenwart eines Lösungsmittels wie Methanol, Ethanol, Essigsäure oder Dioxan bei Temperaturen zwischen 0°C und der Siedetemperatur des Reaktionsgemisches. Bei dieser Umsetzung kann eine gegebenenfalls vorhandene Estergruppe gleichzeitig in die entsprechende Carboxygruppe übergeführt werden.

Ferner können die erhaltenen Verbindungen der allgemeinen Formel I, wie bereits eingangs erwähnt wurde, in ihre Enantiomeren und/oder Diastereomeren aufgetrennt werden. So können beispielsweise cis-/trans-Gemische in ihre cis- und trans-Isomere, und Verbindungen mit mindestens einem optisch aktiven Kohlenstoffatom in ihre Enantiomeren aufgetrennt werden.

So lassen sich beispielsweise die erhaltenen cis-/trans-Gemische durch Chromatographie in ihre cis- und trans-Isomeren, die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel I mit mindestens 2 asymmetrischen Kohlenstoffatomen auf Grund ihrer physikalischchemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-o-Tolylweinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)-oder (-)-Menthyloxycarbonyl in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure oder Maleinsäure in Betracht.

Außerdem lassen sich die so erhaltenen neuen Verbindungen der Formel I, falls diese eine Carboxy-, Phosphono-, O-Alkylphosphono-, Sulfo- oder 5-Tetrazolylgruppe enthalten, gewünschtenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Arginin, Cyclohexylamin, Ethanolamin, Diethanolamin und Triethanolamin in Betracht.

Die als Ausgangsstoffe verwendeten Verbindungen der allgemeinen

Formeln II bis V sind teilweise literaturbekannt oder man erhält diese nach an sich literaturbekannten Verfahren (siehe

Beispiele I bis XLVIII).

Wie bereits eingangs erwähnt, weisen die erfindungsgemäßen

Verbindungen der allgemeinen Formel I und ihre physiologisch verträglichen Salze wertvolle pharmakologische Eigenschaften auf, insbesondere eine spezifische Hemmwirkung auf die durch den Epidermal Growth Factor-Rezeptor (EGF-R) vermittelte Signaltransduktion, wobei diese beispielsweise durch eine Inhibition der Ligandenbindung, der Rezeptordimerisierung oder der

Tyrosinkinase selbst bewirkt werden kann. Außerdem ist es möglich, daß die Signalübertragung an weiter abwärtsliegenden Komponenten blockiert wird.

Die biologischen Eigenschaften der neuen Verbindungen wurden wie folgt geprüft:

Die Hemmung der EGF-R vermittelten Signalübertragung kann z.B. mit Zellen nachgewiesen werden, die humanen EGF-R exprimieren und deren Überleben und Proliferation von Stimulierung durch EGF bzw. TGF-alpha abhängt. Hier wurde eine Interleukin-3-(IL-3) abhängige Zellinie murinen Ursprungs verwendet, die derart genetisch verändert wurde, daß sie funktionellen humanen EGF-R exprimiert. Die Proliferation dieser F/L-HERc genannten Zellen kann daher entweder durch murines IL-3 oder durch EGF stimuliert werden (siehe von Rüden, T. et al. in EMBO J. 7, 2749-2756 (1988) und Pierce, J. H. et al. in Science 239, 628-631 (1988)).

Als Ausgangsmaterial für die F/L-HERc Zellen diente die Zelllinie FDC-$P_1$, deren Herstellung von Dexter, T. M. et al. in J. Exp. Med. 152, 1036-1047 (1980) beschrieben wurde. Alternativ können aber auch andere Wachstumsfaktor-abhängige Zellen verwendet werden (siehe beispielsweise Pierce, J. H. et al. in Science 239, 628-631 (1988), Shibuya, H. et al. in Cell 70, 57-67 (1992) und Alexander, W. S. et al. in EMBO J. 10, 3683-3691 (1991)). Zur Expression der humanen EGF-R cDNA (siehe Ullrich, A. et al. in Nature 309, 418-425 (1984)) wurden rekombinante Retroviren verwendet, wie in von Rüden, T. et al., EMBO J. 7, 2749-2756 (1988) beschrieben, mit dem Unterschied, daß zur Expression der EGF-R cDNA der retrovirale Vektor LXSN (siehe Miller, A. D. et al. in BioTechniques 7, 980-990 (1989)) eingesetzt wurde und als Verpackungszelle die Linie GP+E86 (siehe Markowitz, D. et al. in J. Virol. 62, 1120-1124 (1988)) diente.

Der Test wurde wie folgt durchgeführt:

F/L-HERc Zellen wurden in RPMI/1640 Medium (BioWhittaker), supplementiert mit 10 % foetalem Rinderserum (FCS, Boehringer Mannheim), 2 mM Glutamin (BioWhittaker), Standardantibiotika und 20 ng/ml humanem EGF (Promega), bei 37°C und 5% $CO_2$ kultiviert. Zur Untersuchung der inhibitorischen Aktivität der erfindungsgemäßen Verbindungen wurden 1,5 x $10^4$ Zellen pro Vertiefung in Triplikaten in 96-Loch-Platten in obigem Medium (200µl) kultiviert, wobei die Proliferation der Zellen entweder mit EGF (20 ng/ml) oder murinem IL-3 stimuliert wurde. Als Quelle für IL-3 dienten Kulturüberstände der Zellinie X63/0 mIL-3 (siehe Karasuyama, H. et al.in Eur. J. Immunol. 18, 97-104 (1988)). Die erfindungsgemäßen Verbindungen wurden in 100% Dimethylsulfoxid (DMSO) gelöst und in verschiedenen Verdünnungen den Kulturen zugefügt, wobei die maximale DMSO Konzentration 1% betrug. Die Kulturen wurden für 48 Stunden bei 37°C inkubiert.

Zur Bestimmung der inhibitorischen Aktivität der erfindungsgemäßen Verbindungen wurde die relative Zellzahl mit dem Cell Titer 96™ $AQ_{ueous}$ Non-Radioactive Cell Proliferation Assay (Promega) in O.D. Einheiten gemessen. Die relative Zellzahl wurde in Prozent der Kontrolle (F/L-HERc Zellen ohne Inhibitor) berechnet und die Wirkstoffkonzentration, die die Proliferation der Zellen zu 50% hemmt ($IC_{50}$), abgeleitet. Hierbei wurden folgende Ergebnisse erhalten:

| Verbindung (Beispiel Nr.) | Hemmung der EGF-abhängigen Proliferation $IC_{50}$ [nM] | Hemmung der IL-3-abhängigen Proliferation $IC_{50}$ [µM] |
|---|---|---|
| 1 | 200 | > 10 |
| 1(1) | 34 | > 10 |
| 1(2) | 44 | > 10 |

(fortgesetzt)

| Verbindung (Beispiel Nr.) | Hemmung der EGF-abhängigen Proliferation $IC_{50}$ [nM] | Hemmung der IL-3-abhängigen Proliferation $IC_{50}$ [µM] |
|---|---|---|
| 1(3) | 67 | > 1 |
| 1(4) | 15 | > 10 |
| 1(5) | 21 | > 10 |
| 1(6) | 123 | > 10 |
| 1(7) | 8 | > 1 |
| 1(8) | 3 | > 10 |
| 1(9) | 18 | > 10 |
| 1(10) | 17 | > 10 |
| 1(11) | 75 | > 10 |
| 1(12) | 375 | > 10 |
| 1(13) | 150 | > 10 |
| 1(14) | 28 | > 10 |
| 1(15) | 15 | > 10 |
| 1(16) | 20 | > 1 |
| 1(17) | 1 | > 1 |
| 1(18) | 3 | > 1 |
| 1(19) | 83 | > 1 |
| 1(20) | 225 | > 1 |
| 1(21) | 300 | > 1 |
| 1(22) | 43 | > 1 |
| 1(23) | 63 | > 1 |
| 1(24) | 9 | > 1 |
| 1(25) | 28 | > 20 |
| 1(26) | 200 | > 20 |
| 1(27) | 40 | > 10 |
| 1(28) | 14 | 10 |
| 1(29) | 13 | > 20 |
| 1(30) | 125 | > 20 |
| 1(31) | 103 | 10 |
| 1(32) | > 1000 | > 20 |
| 1(33) | > 1000 | > 20 |
| 1(35) | 240 | > 1 |
| 1(36) | 140 | > 1 |
| 1(38) | 51 | > 1 |
| 1(41) | 200 | > 1 |
| 1(42) | > 100 | > 1 |
| 1(46) | 2 | > 1 |

(fortgesetzt)

| Verbindung (Beispiel Nr.) | Hemmung der EGF-abhängigen Proliferation $IC_{50}$ [nM] | Hemmung der IL-3-abhängigen Proliferation $IC_{50}$ [µM] |
|---|---|---|
| 1(47) | > 100 | > 1 |
| 1(48) | 36 | > 1 |
| 1(49) | > 1000 | > 1 |
| 1(50) | 3 | > 1 |
| 1(51) | 9 | > 1 |
| 1(52) | 10 | > 1 |
| 1(53) | > 100 | > 1 |
| 1(54) | 10 | > 1 |
| 1(55) | 40 | > 1 |
| 1(56) | 40 | > 1 |
| 1(57) | 88 | >10 |
| 1(58) | 4 | > 1 |
| 1(59) | 1 | > 1 |
| 1 (60) | 120 | > 10 |
| 1 (61) | 50 | > 1 |
| 1(62) | 4 | > 10 |
| 1(63) | > 10000 | > 10 |
| 1(64) | 150 | > 10 |

Die erfindungsgemäßen Verbindungen inhibieren auch die EGFstimulierte Proliferation der menschlichen Tumorzellinie KB, die von einem oralen epidermoiden Karzinom stammt und den EGF-Rezeptor überexprimiert (z.B. Aboud-Pirak, E. et al, J. Natl. Cancer. Inst. 80, 1605-11 (1988)). KH-Zellen (bezogen von ATCC) wurden in DMEM (BioWhittaker) in Anwesenheit von 10% FCS (Boehringer Mannheim), 50µM beta-Mercaptoethanol und Standardantibiotika passagiert. Als Indikator für die EGF/TGF-alpha-stimulierte Zellproliferation wurde die EGF-induzierte DNA-Synthese durch Messung des Einbaus radioaktiv markierten Thymidins bestimmt. Dazu wurden die Zellen zweimal gewaschen und 1500 Zellen pro Vertiefung einer 96-Loch-Platte in 200µl IMDM (BioWhittaker) ohne Serum in Anwesenheit von 50µM beta-Mercaptoethanol, Standardantibiotika, TGF-alpha [10ng/ml] oder EGF [20ng/ml] und von verschiedenen Konzentrationen der erfindungsgemäßen Substanzen ausplattiert (Triplikate, maximale DMSO-Konzentration 1%, siehe Proliferations-Test mit F/L-HBRc-Zellen). Nach 60 Stunden wurde für etwa 16 - 18h [$^3$H]-Thymidin (0.1µCi in 10µl) zugegeben. Die anschließende Messung des Thymidin-Einbaus ergab für die Verbindungen 4, 7, 8, 16, 17, 18, 19, 20, 21 und 22 des Beispiels 1 $IC_{50}$-Werte von 0.01 - 1 µM für die Hemmung der BGF/TGF-alpha-stimulierten KB-Zell-Proliferation.

Die erfindungsgemäßen Verbindungen der allgemeinen Formel I hemmen somit die Signaltransduktion durch Tyrosinkinasen, wie am Beispiel des humanen EGF-Rezeptors gezeigt wurde, und sind daher nützlich zur Behandlung pathophysiologischer Prozesse, die durch Überfunktion von Tyrosinkinasen hervorgerufen werden. Das sind z.B. benigne oder maligne Tumoren, insbesondere Tumoren epithelialen und neuroepithelialen Ursprungs, Metastasierung sowie die abnorme Proliferation vaskulärer Endothelzellen (Neoangiogenese).

Außerdem können die Verbindungen der allgemeinen Formel I und deren physiologisch verträglichen Salze zur Behandlung anderer Krankheiten verwendet werden, die durch aberrante Funktion von Tyrosinkinasen verursacht werden, wie z.B. epidermaler Hyperproliferation (Psoriasis), inflammatorischer Prozesse, Erkrankungen des Immunsystems, Hyperproliferation hämatopoetischer Zellen etc..

Auf Grund ihrer biologischen Eigenschaften können die erfindungsgemäßen Verbindungen allein oder in Kombination mit anderen pharmakologisch wirksamen Verbindungen angewendet werden, beispielsweise in der Tumortherapie in Monotherapie oder in Kombination mit anderen Anti-Tumor Therapeutika, beispielsweise in Kombination mit Topoisomerase-Inhibitoren (z.B. Etoposide), Mitoseinhibitoren (z.B. Vinblastin), mit Nukleinsäuren interagierenden

Verbindungen (z.B. cis-Platin, Cyclophosphamid, Adriamycin), Hormon-Antagonisten (z.B. Tamoxifen), Inhibitoren metabolischer Prozesse (z.B. 5-FU etc.), Zytokinen (z.B. Interferonen), Antikörpern etc. Diese Kombinationen können entweder simultan oder sequentiell verabreicht werden.

Bei der pharmazeutischen Anwendung werden die erfindungsgemäßen Verbindungen in der Regel bei warmblütigen Wirbeltieren, insbesondere beim Menschen, in Dosierungen von 0,01-100 mg/kg Körpergewicht, vorzugsweise bei 0,1-15 mg/kg verwendet. Zur Verabreichung werden diese mit einem oder mehreren üblichen inerten Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyäthylenglykol, Propylenglykol, Stearylalkohol, Carboxymethyl-cellulose oder fetthaltigen Substanze wie Hartfett oder deren geeigneten Gemischen in übliche galenische Zubereitungen wie Tabletten, Dragées, Kapseln, Pulver, Suspensionen, Lösungen, Sprays oder Zäpfchen eingearbeitet.

Die nachfolgenden Beispiele sollen die vorliegende Erfindung näher erläutern ohne diese zu beschränken:

Beispiel I

4-Hydroxy-6-methylsulfinyl-pyrimido[5,4-d]pyrimidin und 4-Hydroxy-6-methylsulfonyl-pyrimido[5,4-d]pyrimidin

2,0 g 4-Hydroxy-6-methylthio-pyrimido[5,4-d]pyrimidin und 8 g 3-Chlorperoxybenzoesäure (Gehalt: 50 %) werden in 50 ml Methylenchlorid 3 Stunden kräftig gerührt. Der Niederschlag wird abgesaugt, mit Essigester gewaschen und getrocknet.
Ausbeute: 2,2 g
$R_f$-Wert: 0,27 und 0,50 (Kieselgel; Methylenchlorid/Essigester/Methanol = 10:4:3)

Beispiel II

4-Hydroxy-6-(morpholino)-pyrimido[5,4-d]pyrimidin

16 g eines Gemisches aus 4-Hydroxy-6-methylsulfinyl-pyrimido[5,4-d]pyrimidin und 4-Hydroxy-6-methylsulfonyl-pyrimido[5,4-d]pyrimidin werden in 25 ml Morpholin 4 Stunden auf 135°C (Badtemperatur) erhitzt. Nach dem Abkühlen wird eingeengt, der Rückstand wird mit Wasser verrieben, der Feststoff abgesaugt, mit Wasser gewaschen und getrocknet.
Ausbeute: 7,8 g,
Schmelzpunkt: >240°C
$R_f$-Wert: 0,60 (Kieselgel; Methylenchlorid/Essigester/Methanol = 10:4:3)

Beispiel III

4-Chlor-6-(morpholino)-pyrimido[5,4-d]pyrimidin

7,8 g 4-Hydroxy-6-(morpholino)-pyrimido[5,4-d]pyrimidin werden mit 100 ml Thionylchlorid unter Zusatz von 4 Tropfen Dimethylformamid 1,5 Stunden unter Rückfluß erhitzt. Das Reaktionsgemisch wird eingeengt, mit Methylenchlorid versetzt und nochmals eingeengt. Der Rückstand wird dann zwischen Methylenchlorid und einer wäßrigen Kaliumcarbonatlösung verteilt. Die wäßrige Phase wird noch zweimal mit Methylenchlorid extrahiert, die vereinigten organischen Phasen werden über Magnesiumsulfat getrocknet und eingeengt. Der Rückstand wird mit Diethylether verrieben und abgesaugt.
Ausbeute: 8,0 g (90% der Theorie), Schmelzpunkt: 238-240°C (Zers.)
$R_f$-Wert: 0,60 (Kieselgel; Petrolether/Essigester = 2:1)

| | | | |
|---|---|---|---|
| Ber. | C 63,49 | H 4,76 | N 27,28 |
| Gef. | 63,39 | 4,80 | 27,00 |

Analog Beispiel III wird folgende Verbindung erhalten:

(1) 4-Chlor-6-methylthio-pyrimido[5,4-d]pyrimidin Schmelzpunkt: 90-92°C
$R_f$-Wert: 0,63 (Kieselgel; Petrolether/Essigester = 7:3)

Beispiel IV

5-Amino-2-methylthio-pyrimidin-4-carbonsäure

131,4 g 5-Brom-2-methylthio-pyrimidin-4-carbonsäure, 860 ml konz. wäßriges Ammoniak und 2,42 g Kupfer (II)sulfat, gelöst in 34 ml Wasser, werden in einem Druckgefäß 4 Stunden bei 95°C geschüttelt. Nach dem Abkühlen wird der Niederschlag abgesaugt. Der Niederschlag wird in 600 ml heißem Wasser gelöst und die Lösung über Aktivkohle filtriert. Das Filtrat wird im Eisbad abgekühlt und mit konzentrierter Salzsäure auf einen pH von 3 gebracht. Der Niederschlag wird abgesaugt und durch Lösen in verdünnter Natronlauge und Ausfällen mit Salzsäure gereinigt. Ausbeute: 54,6 g (56% der Theorie),
Schmelzpunkt: 187°C
$R_f$-Wert: 0,35 (Kieselgel; Essigester/Methanol = 2:1)

Beispiel V

4-Hydroxy-6-methylthio-pyrimido[5,4-d]pyrimidin

25 g 5-Amino-2-methylthio-pyrimidin-4-carbonsäure und 150 ml Formamid werden in einem Ölbad gerührt, wobei innerhalb einer halben Stunde die Ölbadtemperatur auf 180°C gesteigert wird. Es wird noch 1,5 Stunden bei dieser Temperatur gerührt. Dann wird das Reaktionsgemisch heiß auf 750 ml eines Eis/Wasser-Gemisches gegeben. Nach 2 Stunden wird abgesaugt, mit Wasser gewaschen und getrocknet.
Schmelzpunkt: >240°C
$R_f$-Wert: 0,63 (Kieselgel; Methylenchlorid/Essigester/Methanol = 10:4:3)

Beispiel VI

4-Hydroxy-6-methylthio-pyrimido[5,4-d]pyrimidin

Eine Mischung von 69 g 5-Amino-2-methylthio-pyrimidin-4-carbonsäure, 155 g Formamidinacetat und 300 ml Ethoxyethanol wird 2 Stunden zum Sieden erhitzt. Dann wird das Reaktionsgemisch auf 10°C abgekühlt, mit 250 ml Wasser versetzt und eine Stunde bei 10°C stehen gelassen. Dann wird abgesaugt, mit Wasser gewaschen und getrocknet.
Ausbeute: 59 g (82% der Theorie),
Schmelzpunkt: >240°C
$R_f$-Wert: 0,63 (Kieselgel; Methylenchlorid/Essigester/Methanol = 10:4:3)

Beispiel VII

3-(Aminocarbonylmethyl)-piperidin

16,8 g Ethyl-3-pyridylacetat und 1 l methanolische Ammoniaklösung werden in einem Druckgefäß 40 Stunden bei 100°C geschüttelt. Nach dem Abkühlen wird eingeengt, der Niederschlag mit Ether verrieben und abgesaugt. 12,7 g des so erhaltenen 3-(Aminocarbonylmethyl)-pyridins werden mit 250 ml Ethanol und 3 g Rhodium/Platin-Katalysator 2 Stunden bei 50°C unter 3 bar Wasserstoffdruck hydriert. Nach dem Abkühlen wird filtriert, eingeengt, der Niederschlag mit Ether verrieben und abgesaugt. Ausbeute 13,3 g (96% der Theorie),
$R_f$-Wert: 0,15 (Kieselgel; Methylenchlorid/Methanol/konz. Ammoniak = 16:4:1)

Beispiel VIII

3-(4-Amino-phenyl)-propionsäure

Eine Mischung von 155 g 4-Nitro-zimtsäure in 1 l Methanol wird mit 15 g Palladium auf Kohle (10 %) und 30 ml Wasser versetzt und bei Raumtemperatur und 3 bar hydriert, bis nach ca. 2 Stunden kein Wasserstoff mehr aufgenommen wird. Nach Filtration und verdampfen des Lösungsmittels im Rotationsverdampfer wird zur Entfernung der Wasser-Reste zweimal mit je 300 ml Toluol versetzt und das Lösungsmittel im Rotationsverdampfer abdestilliert.
Ausbeute: 132 g (100% der Theorie),
Schmelzpunkt: 124-128°C.

Beispiel IX

3-(trans-4-Acetylamino-cyclohexyl)-propionsäure

Eine Mischung von 397 g 3-(4-Amino-phenyl)-propionsäure, 125 g Natriumhydroxid und 160 g Raney-Nickel in 5,7 l Wasser wird bei 170°C und 100 bar hydriert, bis nach ca. 30 Stunden kein Wasserstoff mehr aufgenommen wird. Nach Filtration und Waschen des Rückstands mit Wasser erhält man als Filtrat 6,3 l einer farblosen Lösung, die mit einer Lösung von 192 g Natriumhydroxid in 400 ml Wasser und danach tropfenweise innerhalb 35 Minuten mit 454 ml Acetanhydrid versetzt wird. Nach fünf Stunden wird vom Niederschlag abfiltriert, das Filtrat durch Zugabe konzentrierter Salzsäure auf pH4 gebracht und drei Stunden bei 0°C gerührt. Dann wird abgesaugt, mit 250 ml Eiswasser nachgewaschen und bei 70°C getrocknet.
Ausbeute: 216 g (42% der Theorie),
Schmelzpunkt: 193-196°C

Beispiel X

3-(trans-4-Amino-cyclohexyl)-propionsäuremethylester-hydrochlorid

Eine Mischung von 185 g 3-(trans-4-Acetylamino-cyclohexyl)-propionsäure, 500 ml Wasser und 500 ml konzentrierte Salzsäure wird 68 Stunden zum Sieden erhitzt. Dann wird im Rotationsverdampfer zur Trockne eingedampft, fünfmal mit je 300 ml einer Methanol/Toluol (2:1) Mischung versetzt und jeweils erneut eingedampft. Der Rückstand wird mit 450 ml einer Aceton/tert.Butylmethylether (1:2) Mischung verrührt, abgesaugt und im Vakuum über Natriumhydroxid getrocknet. Zur Vervollständigung der Veresterung wird in 1 1 Methanol gelöst und unter Eiskühlung tropfenweise mit 50 ml Thionylchlorid versetzt. Nach 30 Minuten wird das Lösungsmittel im Rotationsverdampfer abdestilliert, der Rückstand mit 300 ml Methanol versetzt und erneut eingedampft. Der Rückstand wird mit 450 ml einer Aceton/tert.Butylmethylether (1:2) Mischung verrührt, abgesaugt und getrocknet.
Ausbeute: 178 g (92% der Theorie),
Schmelzpunkt: 196-198°C

Beispiel XI

4-Amino-2,6-dibromanilin

20 g 2,6-Dibrom-4-nitroanilin werden in 250 ml Ethanol, 250 ml Essigsäureethylester, 100 ml Dimethylformamid und 90 ml Methylenchlorid aufgenommen, mit 3,3 g 5%igem feuchtem Platin/Aktivkohle-Katalysator versetzt und eine Stunde bei Raumtemperatur und 50 psi hydriert. Dann wird das Lösungsmittel im Rotationsverdampfer abdestilliert, der Rückstand mit Diethylether verrieben, abgesaugt und mehrfach mit Petrolether nachgewaschen. Ausbeute: 8 g (45% der Theorie),
Schmelzpunkt: 127-132°C
$R_f$-Wert: 0,59 (Kieselgel; Petrolether/Essigester = 10:5)

Beispiel XII

4-Amino-1-tert.butyloxycarbonyl-piperidin

10 g 4-Amino-piperidin werden bei 0°C in 120 ml eines Dioxan/-Wasser (1:1) Gemisches mit 22 g Di-tert. butyldicarbonat und 14 ml Triethylamin versetzt und 12 Stunden bei Raumtemperatur gerührt. Dann wird das Dioxan im Rotationsverdampfer abdestilliert und die wäßrige Phase sechsmal mit Essigester extrahiert. Die vereinigten organischen Phasen werden über Magnesiumsulfat getrocknet und das Lösungsmittel im Rotationsverdampfer abdestilliert. Der Rückstand kristallisiert langsam.
Ausbeute: 16 g (80% der Theorie),
Schmelzpunkt: 47-52°C
$R_f$-Wert: 0,69 (Aluminiumoxid; Methylenchlorid/Methanol = 9:1)
Analog Beispiel XII werden folgende Verbindungen erhalten:

(1) 1-tert.Butyloxycarbonylamino-2-methyl-2-propylamin Hergestellt aus 1,2-Diamino-2-methylpropan.
Gelbes Öl
$R_f$-Wert: 0,45 (Aluminiumoxid; Methylenchlorid/Methanol = 20:1)

(2) 3-Aminocarbonyl-1-tert.butyloxycarbonyl-piperidin Schmelzpunkt: 172-177°C
$R_f$-Wert: 0,50 (Aluminiumoxid; Methylenchlorid/Methanol = 10:0,8)

Beispiel XIII

4-Aminomethyl-1-tert.butyloxycarbonyl-piperidin

10 g 4-Aminomethyl-piperidin werden bei 0°C in 120 ml eines Dioxan/Wasser (1:1) Gemisches mit 19 g Di-tert.butyldicarbonat und 12 ml Triethylamin versetzt und 12 Stunden bei Raumtemperatur gerührt. Dann wird das Dioxan im Rotationsverdampfer abdestilliert und die wäßrige Phase sechsmal mit Essigester extrahiert. Die vereinigten organischen Phasen werden über Magnesiumsulfat getrocknet und das Lösungsmittel im Rotationsverdampfer abdestilliert. Der Rückstand wird durch Chromatographie über eine Aluminiumoxidsäule mit Methylenchlorid/Methanol = 50:1 gereinigt.
Ausbeute: 7,4 g (39% der Theorie),
$R_f$-Wert: 0,48 (Aluminiumoxid; Methylenchlorid/Methanol = 10:1)
Analog Beispiel XIII wird folgende Verbindung erhalten:

(1) 4-Amino-1-tert.-butyloxycarbanyl-azacycloheptan farbloses Öl
$R_f$-Wert: 0,36 (Aluminiumoxid; Methylenchlorid/Methanol = 9:1)

Beispiel XIV

3-Aminomethyl-piperidin

Zu 8 g Lithiumaluminiumhydrid in 250 ml Tetrahydrofuran werden bei Raumtemperatur unter Rühren portionsweise 7 g Piperidin-3-carbonsäureamid in 30 ml Tetrahydrofuran getropft. Danach wird noch 10 Stunden zum Sieden erhitzt. Man kühlt auf 0°C und tropft vorsichtig solange 10%ige Kalilauge hinzu, bis. sich ein weißer Niederschlag gebildet hat. Man dekantiert und wäscht den Niederschlag viermal durch Zugabe von je 50 ml Tetrahydrofuran und Abdekantieren. Die organischen Phasen werden vereinigt, das Lösungsmittel im Rotationsverdampfer abdestilliert und der Rückstand an Aluminiumoxid mit einem Methylenchlorid/Methanol (16:1) Gemisch säulenchromatographisch gereinigt.
Ausbeute: 4,8 g (77% der Theorie) als farbloses Öl.
Analog Beispiel XIV wird folgende Verbindung erhalten:

(1) 4-Aminomethyl-chinuclidin
Farbloses Öl
$R_f$-Wert: ca. 0,3 (zieht Schweifspur; Aluminiumoxid; Methylenchlorid/Methanol = 7:3)
Massenspektrum: $M^+$ = 140
Hergestellt aus 4-Cyano-chinuclidin (siehe Beispiel 3 der EP-A-0,213,337).

Beispiel XV

trans-4-tert.Butyloxycarbonylaminomethyl-cyclohexancarbonsäure

4,6 g trans-4-Aminomethyl-cyclohexancarbonsäure werden in 65 ml 1N Natronlauge gelöst und mit 6,6 g Di-tert.butyldicarbonat in 50 ml Tetrahydrofuran versetzt. Nach 12 Stunden wird sechsmal mit Essigester extrahiert. Die vereinigten organischen Phasen werden nacheinander mit einer 2N Citronensäurelösung und gesättigter Natriumchloridlösung gewaschen, über Magnesiumsulfat getrocknet und das Lösungsmittel im Rotationsverdampfer abdestilliert. Der Rückstand wird bei 0,1 torr getrocknet.
Ausbeute: 6,5 g (87% der Theorie),
Schmelzpunkt: 137-140°C

Beispiel XVI

trans-4-(tert.Butyloxycarbonylaminomethyl)-benzyloxycarbonylamino-cyclohexan

5,5 g trans-4-tert.Butyloxycarbonylaminomethyl-cyclohexancarbonsäure werden in 250 ml Dioxan gelöst, mit 6,5 ml Triethylamin und 5, 6 ml Diphenylphosphorylazid versetzt und 1,5 Stunden auf 130°C erhitzt. Dann werden 8,7

ml Benzylalkohol hinzugegeben und weitere 14 Stunden zum Sieden erhitzt. Nach dem Abkühlen wird das Dioxan im Rotationsverdampfer abdestilliert, der Rückstand in Essigester aufgenommen, mit gesättigter Natriumchloridlösung gewaschen, über Magnesiumsulfat getrocknet und das Lösungsmittel im Rotationsverdampfer abdestilliert. Der Rückstand wird mit Petrolether/Ether (5:1) verrieben, abgesaugt und getrocknet.
Ausbeute: 6,6 g (86% der Theorie),
Schmelzpunkt: 117-122°C

Beispiel XVII

trans-4-(Aminomethyl)-benzyloxycarbonylamino-cyclohexan

1,3 g trans-4-(tert.Butyloxycarbonylaminomethyl)-benzyloxycarbonylamino-cyclohexan werden in 50 ml Methylenchlorid gelöst und mit 5 ml Trifluoressigsäure versetzt. Nach 1 Stunde werden 33 ml 2N Natronlauge zugetropft. Nach der Phasentrennung wird die wäßrige Phase noch dreimal mit Methylenchlorid extrahiert, die vereinigten organischen Phasen werden über Magnesiumsulfat getrocknet und das Lösungsmittel im Rotationsverdampfer abdestilliert. Der Rückstand wird ohne weitere Reinigung eingesetzt.
Ausbeute: 870 mg (93% der Theorie) eines farblosen Wachses,
$R_f$-Wert: 0,15 (Aluminiumoxid; Methylenchlorid/Methanol = 20:1)

Beispiel XVIII

trans-4-Amino-(tert.butyloxycarbonylaminomethyl)-cyclohexan

1,4 g trans-4-(tert.Butyloxycarbonylaminomethyl)-benzyloxycarbonylamino-cyclohexan werden in 30 ml Methanol gelöst, mit 0,3 g Palladium/Aktivkohle-Katalysator versetzt und eine Stunde bei Raumtemperatur und 50 psi hydriert. Dann wird filtriert und das Lösungsmittel im Rotationsverdampfer abdestilliert. Der Rückstand wird ohne weitere Reinigung eingesetzt.
Ausbeute: 1,02 g (100% der Theorie) eines farblosen Wachses,
$R_f$-Wert: 0,28 (Aluminiumoxid; Methylenchlorid/Methanol = 10:1)

Beispiel XIX

1-Benzyl-azacycloheptan-4-on-oxim

7,2 g 1-Benzyl-azacycloheptan-4-on werden bei 60°C zu einer Lösung von 3,1 g Hydroxylaminhydrochlorid und 2,9 g Natriumacetat in 30 ml Wasser gegeben. Nach 2 Stunden wird abgekühlt und mit 2N Natronlauge alkalisch gestellt. Die Mischung wird viermal mit Diethylether extrahiert, mit gesättigter Natriumchloridlösung gewaschen, über Magnesiumsulfat getrocknet und das Lösungsmittel im Rotationsverdampfer abdestilliert. Der Rückstand wird mit Petrolether verrrieben, abgesaugt und ohne weitere Reinigung eingesetzt.
Ausbeute: 5,9 g (90% der Theorie),
Schmelzpunkt: 73-76°C
$R_f$-Wert: 0,16 (Aluminiumoxid; Petrolether/Essigester = 1:1)

Beispiel XX

4-Amino-1-benzyl-azacycloheptan

2,18 g 1-Benzyl-azacycloheptan-4-on-oxim werden in 30 ml Tetrahydrofuran und 2 ml konzentrierter Ammoniaklösung gelöst, mit 0,9 g Raney-Nickel versetzt und 12 Stunden bei Raumtemperatur und 50 psi hydriert. Dann wird filtriert und das Lösungsmittel im Rotationsverdampfer abdestilliert. Der Rückstand wird mit Wasser versetzt, die Mischung dreimal mit Methylenchlorid extrahiert, über Magnesiumsulfat getrocknet und das Lösungsmittel im Rotationsverdampfer abdestilliert. Der Rückstand wird an Aluminiumoxid mit einem Methylenchlorid/Methanol (30:1) Gemisch säulenchromatographisch gereinigt.
Ausbeute: 1,55 g (76% der Theorie) als farbloses Öl,
$R_f$-Wert: 0,43 (Aluminiumoxid; Methylenchlorid/Methanol = 9:1)

Beispiel XXI

4-Amino-azacycloheptan

13,4 g 4-Amino-1-benzyl-azacycloheptan werden in 200 ml Methanol gelöst, mit 10 g Palladiumdihydroxid versetzt und eine Stunde bei Raumtemperatur und 50 psi hydriert. Dann wird filtriert und das Lösungsmittel im Rotationsverdampfer abdestilliert. Der Rückstand wird bei 0,7 torr destilliert.
Ausbeute: 7,6 g (100% der Theorie) als farbloses Öl,
Kp.: 43°C bei 0,7 torr.

Beispiel XXII

4-(N-Methyl-N-acetylamino)-aminocyclohexan

2,5 g 4-(N-Methyl-N-acetylamino)-anilin werden in 50 ml Methanol und 15 ml 1N Salzsäure gelöst, mit 2 g Rhodium/Aktivkohle versetzt und acht Stunden bei 70°C und 50 psi hydriert. Dann wird filtriert und das Lösungsmittel im Rotationsverdampfer abdestilliert. Der Rückstand wird mit 50%iger Natronlauge alkalisch gestellt, die Mischung dreimal mit Methylenchlorid extrahiert, über Magnesiumsulfat getrocknet und das Lösungsmittel im Rotationsverdampfer abdestilliert. Der Rückstand wird an Kieselgel mit einem Methylenchlorid/Essigester/Methanol (10:4:3) Gemisch säulenchromatographisch gereinigt.
Ausbeute: 0,7 g (28% der Theorie) als farbloses Öl,
$R_f$-Wert: 0,40 (Aluminiumoxid; Methylenchlorid/Essigester/Methanol = 10:4:3)

Beispiel XXIII

4-Tetrahydropyranon-oxim

Zu einer Mischung von 5.2 g Hydroxylamin-hydrochlorid und 4.8 g Natriumacetat in 50 ml Wasser wird bei 60°C unter Rühren 5.0 g 4-Tetrahydropyranon getropft. Nach einer weiteren Stunde bei 60°C wird abkühlen gelassen und die Lösung dreimal mit je 50 ml Ether extrahiert. Dann werden die vereinigten organischen Phasen über Natriumsulfat getrocknet, das Solvens im Rotationsverdampfer abdestilliert und der Rückstand ohne weitere Reinigung in die nächste Reaktion eingesetzt.
Ausbeute: 4,2 g (74% der Theorie),
Schmelzpunkt: 50-52°C
$R_f$-Wert: 0,30 (Kieselgel; Petrolether/Essigester = 1:1)

Beispiel XXIV

4-Amino-tetrahydropyran

4.2 g 4-Tetrahydropyranon-oxim werden in 100 ml Ethanol gelöst und nach Zugabe von 0.5 g Palladium auf Kohle (10%) in einer Parr-Apparatur 2.5 Stunden bei 90°C und 5 bar Wasserstoffdruck hydriert. Nach dem Abkühlen wird das Solvens im Rotationsverdampfer abdestilliert und der Rückstand ohne weitere Reinigung weiter verwendet.
Ausbeute: 0.7 g (19% der Theorie) eines farblosen Öls,
$R_f$-Wert: 0,45 (Kieselgel; Methylenchlorid/Essigester/Methanol = 10:4:2)

Beispiel XXV

1-(2-Benzylidenamino-ethyl)-piperazin

12,9 g 1-(2-Amino-ethyl)-piperazin und 10,6 g Benzaldehyd werden in 125 ml Toluol am Wasserabscheider zum Sieden erhitzt, bis sich nach ca. 4 Stunden kein Wasser mehr abscheidet. Nach dem Abkühlen wird das Solvens im Rotationsverdampfer abdestilliert und der Rückstand ohne weitere Reinigung weiter verwendet.
Ausbeute: 21.7 g (100% der Theorie) eines leicht bräunlichen Öls,
$R_f$-Wert: 0,70 (Kieselgel; Methylenchlorid/Methanol = 10:1)

Beispiel XXVI

N-Benzyl-3-methyl-4-piperidon-3-carbonsäureethylester

8,88 g N-Benzyl-4-piperidon-3-carbonsäureethylester in 30 ml Tetrahydrofuran werden unter Eiskühlung mit 4,2 g Kalium-tert.butylat und 2,1 g Methyliodid versetzt. Nach 12 Stunden bei Raumtemperatur wird filtriert, der Rückstand mit Tetrahydrofuran gewaschen und die vereinigten Filtrate im Rotationsverdampfer vom Solvens befreit. Das verbleibende Ö1 wird an Kieselgel mit Methylenchlorid säulenchromatographisch gereinigt.
Ausbeute: 3,41 g (36% der Theorie) eines gelben Öls,
$R_f$-Wert: 0,80 (Kieselgel; Methylenchlorid/Methanol = 50:1)

Beispiel XXVII

N-Benzyl-3-methyl-4-piperidon

3,29 g N-Benzyl-3-methyl-4-piperidon-3-carbonsäureethylester werden in 20 ml 6N Salzsäure acht Stunden zum Sieden erhitzt. Nach dem Abkühlen wird mit 200 ml Methylenchlorid versetzt und unter Eiskühlung mit 15%iger Natronlauge alkalisch gestellt. Die Lösung wird dreimal mit je 50 ml Methylenchlorid extrahiert. Dann werden die vereinigten organischen Phasen über Natriumsulfat getrocknet, das Solvens im Rotationsverdampfer abdestilliert und der Rückstand ohne weitere Reinigung weiter verwendet.
Ausbeute: 2,26 g (93% der Theorie) eines leicht braunen Öls,
$R_f$-Wert: 0,70 (Kieselgel; Methylenchlorid/Methanol = 20:1)

Beispiel XXVIII

cis- und trans-1-Benzyl-4-benzylamino-3-methyl-piperidin

9,94 g N-Benzyl-3-methyl-4-piperidon, 5,4 ml Benzylamin, 90 ml Toluol und 10 g Molsieb (4A) werden 12 Stunden bei Raumtemperatur gerührt, filtriert und das Solvens im Rotationsverdampfer abdestilliert. Der Rückstand wird in 50 ml Methanol gelöst und unter Eiskühlung mit 0,55 g Natriumboranat versetzt. Nach 5 Stunden bei Raumtemperatur wird das Solvens im Rotationsverdampfer abdestilliert, der Rückstand mit Methylenchlorid und Eiswasser versetzt und die Mischung mit Citronensäure auf pH 5 gestellt. Die Phasen werden nach gutem Schütteln getrennt, die wäßrige wird dann mit Natronlauge alkalisch gestellt und dreimal mit Methylenchlorid extrahiert. Dann werden die vereinigten organischen Phasen über Natriumsulfat getrocknet, das Solvens im Rotationsverdampfer abdestilliert und der Rückstand an Kieselgel mit einem Methylenchlorid/Methanol/konz.Ammoniak (erst 98:2:0,8, dann 95:5:0,8) Gemisch säulenchromatographisch gereinigt. Dabei werden die Isomeren getrennt. Neben 3 g einer Mischung der beiden Isomeren erhält man:
Ausbeute: 4,0 g cis-Isomer (27% der Theorie) eines gelben Öls,
$R_f$-Wert: 0,90 (Kieselgel; Methylenchlorid/Methanol/konz.Ammoniak = 98:2:2)
Ausbeute: 1,5 g trans-Isomer (10% der Theorie) eines gelben Öls,
$R_f$-Wert: 0,45 (Kieselgel; Methylenchlorid/Methanol/konz.Ammoniak = 98:2:2)

Beispiel XXIX

cis-4-Amino-3-methyl-piperidin

3,8 g cis-1-Benzyl-4-benzylamino-3-methyl-piperidin werden in 30 ml Methanol und 25 ml 1N Salzsäure gelöst, mit 1,5 g Palladium-dihydroxid versetzt und 10 Stunden bei 50°C und 3 bar hydriert. Dann wird filtriert und das Lösungsmittel im Rotationsverdampfer abdestilliert. Der Rückstand wird mit 15%iger Natronlauge versetzt und dreimal mit Diethylether extrahiert. Dann werden die vereinigten organischen Phasen über Natriumsulfat getrocknet und das Solvens im Rotationsverdampfer abdestilliert. Der Rückstand wird ohne weitere Reinigung weiter verwendet.
Ausbeute: 1,1 g (72% der Theorie) als farbloses Öl.
Analog Beispiel XXIX wird folgende Verbindung erhalten:

(1) trans-4-Amino-3-methyl-piperidin
Hergestellt aus trans-1-Benzyl-4-benzylamino-3-methylpiperidin

Beispiel XXX

4-[(3-Chlor-4-fluor-phenyl)amino]-6-methylthio-pyrimido[5,4-d]-pyrimidin

3,0 g 4-Chlor-6-methylthio-pyrimido[5,4-d]pyrimidin, 3,8 g 3-Chlor-4-fluor-anilin und 10 ml Dioxan werden 2 Stunden auf 80°C erhitzt. Nach dem Abkühlen wird das Reaktionsgemisch eingeengt und erst mit Wasser, dann mit Diethylether verrieben, abgesaugt und getrocknet.
Ausbeute: 4,0 g (91 % der Theorie),
Schmelzpunkt: 144-148°C
$R_f$-Wert: 0,50 (Kieselgel; Petrolether/Essigester = 1:1)

Beispiel XXXI

4-[(3-Chlor-4-fluor-phenyl)amino]-6-methylthio-pyrimido[5,4-d]-pyrimidin

148 g 4-Hydroxy-6-methylthio-pyrimido[5,4-d]pyrimidin, 286 ml Hexamethyldisilazan, 333 g 3-Chlor-4-fluor-anilin und 15 g p-Toluolsulfonsäure werden 23 Stunden auf 140°C erhitzt. Nach dem Abkühlen wird das Reaktionsgemisch mit 41 Methanol versetzt und eine Stunde auf 100°C erhitzt. Das Methanol wird abdestilliert und der Rückstand dreimal mit Diethylether verrieben und abgesaugt.
Ausbeute: 202 g (82 % der Theorie),
Schmelzpunkt: 144-148°C
$R_f$-Wert: 0,50 (Kieselgel; Petrolether/Essigester = 1:1)

Analog den Beispielen XXX und XXXI werden folgende Verbindungen erhalten:

(1) 4-[(3-Methylphenyl)amino]-6-methylthio-pyrimido[5,4-d]-pyrimidin
Schmelzpunkt: 118-120°C
$R_f$-Wert: 0,55 (Kieselgel; Petrolether/Essigester = 2:1)

(2) 4-[(4-Amino-3,5-dichlorphenyl)amino]-6-methylthio-pyrimido[5,4-d]-pyrimidin
Schmelzpunkt: 195-197°C
$R_f$-Wert: 0,50 (Kieselgel; Petrolether/Essigester = 1:1)

(3) 4-[(4-Amino-3-brom-5-chlorphenyl)amino)-6-methylthio-pyrimido[5,4-d]-pyrimidin
Schmelzpunkt: 210-212°C
$R_f$-Wert: 0,31 (Kieselgel; Petrolether/Essigester = 1:1)

(4) 4-[(4-Amino-3,5-dibromphenyl)amino]-6-methylthio-pyrimido[5,4-d)-pyrimidin
Schmelzpunkt: 245-247°C
$R_f$-Wert: 0,35 (Kieselgel; Petrolether/Essigester = 1:1)

(5) 4-[(3-(1,1,2,2-Tetrafluorethoxy)phenyl)amino)-6-methylthiopyrimido[5,4-d]-pyrimidin
Schmelzpunkt: 129-130°C
$R_f$-Wert: 0,55 (Kieselgel; Petrolether/Essigester = 2:1)

(6) 4-[(4-Benzyl-phenyl)amino)-6-methylthio-pyrimido[5,4-d)-pyrimidin
Schmelzpunkt: 197-199°C
$R_f$-Wert: 0,65 (Kieselgel; Petrolether/Essigester = 2:1)

(7) 4-[(3-Benzyloxy-phenyl)amino]-6-methylthio-pyrimido[5,4-d]pyrimidin
Schmelzpunkt: 144-146°C
$R_f$-Wert: 0,80 (Kieselgel; Petrolether)

(8) 4-[(3-Hydroxymethyl-phenyl)amino]-6-methylthio-pyrimido-[5,4-d]-pyrimidin
Schmelzpunkt: 164-166°C
$R_f$-Wert: 0,55 (Kieselgel; Petrolether/Essigester = 2:1)

(9) 4-[4-Biphenylylamino]-6-methylthio-pyrimido[5,4-d]pyrimidin
Schmelzpunkt: 200-202°C

$R_f$-Wert: 0,45 (Kieselgel; Petrolether/Essigester = 1:1)

(10) 4-[3-Phenoxy-phenylamino]-6-methylthio-pyrimido[5,4-d]-pyrimidin
Schmelzpunkt: 140-142°C
$R_f$-Wert: 0,40 (Kieselgel; Petrolether/Essigester = 2:1)

(11) 4-[3-Ethinyl-phenylamino]-6-methylthio-pyrimido[5,4-d]-pyrimidin
Schmelzpunkt: 160-163°C
$R_f$-Wert: 0,39 (Kieselgel; Petrolether/Essigester = 2:1)

(12) 4-[3,4-Difluor-phenylamino]-6-methylthio-pyrimido[5,4-d]-pyrimidin
Schmelzpunkt: 175-178°C
$R_f$-Wert: 0,45 (Kieselgel; Petrolether/Essigester = 10:6)

(13) 4-[3-Cyano-phenylamino)-6-methylthio-pyrimido[5,4-d)-pyrimidin
Schmelzpunkt: 240-244°C
$R_f$-Wert: 0,52 (Kieselgel; Petrolether/Essigester = 10:6)

(14) 4-[3-Trifluormethoxy-phenylamino]-6-methylthio-pyrimido-[5,4-d]pyrimidin
Schmelzpunkt: 114-116°C
$R_f$-Wert: 0,56 (Kieselgel; Petrolether/Essigester = 10:5)

(15) 4-[3-Nitro-phenylamino]-6-methylthio-pyrimido[5,4-d]-pyrimidin
Schmelzpunkt: 194-197°C
$R_f$-Wert: 0,30 (Kieselgel; Petrolether/Essigester = 10:5)

(16) 4-[4-Phenoxy-phenylamino]-6-methylthio-pyrimido[5,4-d]-pyrimidin
Schmelzpunkt: 191-192°C
$R_f$-Wert: 0,50 (Kieselgel; Petrolether/Essigester = 10:5)

(17) 4-[4-Benzyloxy-phenylamino]-6-methylthio-pyrimido[5.4-d]-pyrimidin
Schmelzpunkt: 163°C
$R_f$-Wert: 0,44 (Kieselgel; Petrolether/Essigester = 10:5)

(18) 4-[3-Chlor-4-phenoxy-phenylamino]-6-methylthio-pyrimido-[5,4-d]pyrimidin
Schmelzpunkt: 169-170°C
$R_f$-Wert: 0,39 (Kieselgel; Petrolether/Essigester = 10:5)

(19) 4-[4-Benzyloxy-3-chlor-phenylamino]-6-methylthio-pyrimido-[5,4-d]pyrimidin
Schmelzpunkt: 144-146°C
$R_f$-Wert: 0,55 (Aluminiumoxid; Petrolether/Essigester = 20:1)

Beispiel XXXII

4-[(3-Chlor-4-fluor-phenyl)amino]-6-methylsulfinyl-pyrimido-[5,4-d]pyrimidin und 4-[(3-Chlor-4-fluor-phenyl)amino]-6-methylsulfonylpyrimido[5,4-d]pyrimidin

4,0 g 4-[(3-Chlor-4-fluor-phenyl)amino]-6-methylthio-pyrimido[5,4-d]pyrimidin werden in 100 ml Methylenchlorid und 5 ml Methanol gelöst und bei Raumtemperatur portionsweise mit 8,0 g 3-Chlorperoxybenzoesäure (50%ig) versetzt. Nach zwei Stunden wird zweimal mit Natriumbicarbonatlösung gewaschen, über Magnesiumsulfat getrocknet und eingeengt. Die Titelverbindungen werden als 1:1-Gemisch erhalten und ohne weitere Trennung weiter eingesetzt.
Ausbeute: 4,2 g,
Schmelzpunkt des Gemisches: 170°C (Zersetzung)
$R_f$-Werte: 0,10 und 0,28 (Kieselgel; Petrolether/Essigester = 1:1)

Beispiel XXXIII

4-[(3-Chlor-4-fluor-phenyl)amino]-6-methylsulfinyl-pyrimido-[5,4-d]pyrimidin und 4-[(3-,Chlor-4-fluor-phenyl)amino]-6-methylsulfonylpyrimido[5,4-d]pyrimidin

39,2 g 4-[(3-Chlor-4-fluor-phenyl)amino]-6-methylthio-pyrimido[5,4-d]pyrimidin werden in 350 ml Eisessig gelöst und bei Raumtemperatur portionsweise innerhalb vier Stunden mit 37 g Natriumperborat versetzt. Nach 24 Stunden wird auf 11 Wasser gegossen, der Niederschlag abgesaugt und zweimal mit Wasser, einmal mit Natriumbicarbonatlösung und noch zweimal mit Wasser gewaschen und getrocknet. Das Produkt ist eine 10:1-Mischung aus Sulfoxid- und Sulfonyl-Verbindung und wird ohne weitere Reinigung weiter eingesetzt.
Ausbeute: 38 g,
$R_f$-Wert: 0,10 und 0,28 (Kieselgel; Petrolether/Essigester = 1:1)
Schmelzpunkt des Gemisches: 140-145°C (Zersetzung)
Analog den Beispielen XXXII und XXXIII werden folgende Verbindungen erhalten:

(1) 4-[(3-Methylphenyl)amino]-6-methylsulfinyl-pyrimido[5,4-d]-pyrimidin und 4-[(3-Methylphenyl)amino]-6-methylsulfonyl-pyrimido[5,4-d]pyrimidin
$R_f$-Wert: 0,38 und 0,54 (Kieselgel; Petrolether/Essigester/Methanol = 10:10:1)

(2) 4-[(4-Amino-3,5-dichlorphenyl)amino]-6-methylsulfinylpyrimido[5,4-d]pyrimidin und 4-[(4-Amino-3,5-dichlorphenyl)-amino]-6-methylsulfonyl-pyrimido[5,4-d]pyrimidin
$R_f$-Wert: 0,40 und 0,51 (Kieselgel; Petrolether/Essigester/Methanol = 10:10:2)

(3) 4-[(4-Amino-3-brom-5-chlorphenyl)amino]-6-methylsulfinylpyrimido[5,4-d]pyrimidin und 4-[(4-Amino-3-brom-5-chlorphenyl)-amino]-6-methylsulfonyl-pyrimido[5,4-d]pyrimidin
$R_f$-Wert: 0,28 und 0,40 (Kieselgel; Petrolether/Essigester/Methanol = 10:10:1)

(4) 4-[(4-Amino-3,5-dibromphenyl)amino]-6-methylsulfinyl-pyrimido[5,4-d]pyrimidin und 4-[(4-Amino-4,5-dibromphenyl)amino]-6-methylsulfonyl-pyrimido[5,4-d]pyrimidin
$R_f$-Wert: 0,51 und 0,68 (Kieselgel; Petrolether/Essigester/Methanol = 10:10:2)

(5) 4-[(3-(1,1,2,2-Tetrafluorethoxy)phenyl)amino]-6-methylsulfinyl-pyrimido[5,4-d]pyrimidin und 4-[(3-(1,1,2,2-Tetrafluorethoxy)phenyl)amino]-6-methylsulfonyl-pyrimido[5,4-d]pyrimidin
$R_f$-Wert: 0,32 und 0,80 (Kieselgel; Petrolether/Essigester/Methanol = 10:10:1)

(6) 4-[(4-Benzyl-phenyl)amino]-6-methylsulfinyl-pyrimido-[5,4-d]pyrimidin und 4-[(4-Benzyl-phenyl)amino]-6-methylsulfonyl-pyrimido[5,4-d]pyrimidin
$R_f$-Wert: 0,20 und 0,58 (Kieselgel; Petrolether/Essigester/Methanol = 10:10:1)

(7) 4-[(3-Benzyloxy-phenyl)amino]-6-methylsulfinyl-pyrimido-[5,4-d]pyrimidin und 4-[(3-Benzyloxy-phenyl)amino]-6-methylsulfonyl-pyrimido[5,4-d]pyrimidin
$R_f$-Wert: 0,40 (Kieselgel; Petrolether/Essigester = 2:1)

(8) 4-[(3-Hydroxymethyl-phenyl)amino]-6-methylsulfinyl-pyrimido[5,4-d]pyrimidin und 4-[(3-Hydroxymethyl-phenyl)amino]-6-methylsulfonyl-pyrimido[5,4-d]pyrimidin
$R_f$-Wert: 0,15 und 0,40 (Kieselgel; Petrolether/Essigester/Methanol = 10:10:1)

(9) 4-[4-Biphenylylamino]-6-methylsulfinyl-pyrimido[5,4-d]pyrimidin und 4-[4-Biphenylylamino]-6-methylsulfonyl-pyrimido-[5,4-d]pyrimidin
$R_f$-Wert: 0,38 (Kieselgel; Petrolether/Essigester/Methanol = 10:10:1)

(10) 4-[3-Phenoxy-phenylamino]-6-methylsulfinyl-pyrimido-[5,4-d]pyrimidin und 4-[3-Phenoxy-phenylamino]-6-methylsulfonyl-pyrimido[5,4-d]pyrimidin
$R_f$-Wert: 0,31 und 0,48 (Kieselgel; Petrolether/Essigester/Methanol = 10:10:1)

(11) 4-[3-Ethinyl-phenylamino]-6-methylsulfinyl-pyrimido-[5,4-d]pyrimidin und 4-[3-Ethinyl-phenylamino]-6-methylsulfonyl-pyrimido[5,4-d]pyrimidin
Schmelzpunkt: 160-163°C

$R_f$-Wert: 0,31 und 0,38 (Kieselgel; Petrolether/Essigester/Methanol = 10:10:1)

(12) 4-[3,4-Difluorphenylamino]-6-methylsulfinyl-pyrimido-[5,4-d]pyrimidin und 4-[3,4-Difluorphenylamino]-6-methylsulfonyl-pyrimido[5,4-d]pyrimidin
Schmelzpunkt: 169-173°C
$R_f$-Wert: 0,46 (Kieselgel; Petrolether/Essigester/Methanol = 10:10:2)

(13) 4-[3-Cyano-phenylamino]-6-methylsulfinyl-pyrimido[5,4-d]-pyrimidin und 4-[3-Cyano-phenylamino]-6-methylsulfonyl-pyrimido[5,4-d]-pyrimidin
Schmelzpunkt: 222-225°C
$R_f$-Wert: 0,38 und 0,47 (Kieselgel; Petrolether/Essigester/Methanol = 10:8:2)

(14) 4-[3-Trifluormethoxy-phenylamino]-6-methylsulfinyl-pyrimido[5,4-d]pyrimidin und 4-[3-Trifluormethoxy-phenylamino]-6-methylsulfonyl-pyrimido[5,4-d]pyrimidin
Schmelzpunkt: 95-100°C
$R_f$-Wert: 0,28 und 0,48 (Aluminiumoxid; Petrolether/Essigester/Methanol = 10:10:1)

(15) 4-[3-Nitro-phenylamino]-6-methylsulfinyl-pyrimido[5,4-d]-pyrimidin und 4-[3-Nitro-phenylamino]-6-methylsulfonyl-pyrimido[5,4-d]-pyrimidin
Schmelzpunkt: 71°C (Zers.)
$R_f$-Wert: 0,44 und 0,53 (Kieselgel; Petrolether/Essigester/-Methanol = 10:10:2)

(16) 4-[4-Phenoxy-phenylamino]-6-methylsulfinyl-pyrimido-[5,4-d]pyrimidin und 4-[4-Phenoxy-phenylamino]-6-methylsulfonyl-pyrimido[5,4-d]pyrimidin
$R_f$-Wert: 0,28 und 0,41 (Kieselgel; Petrolether/Essigester/Methanol = 10:10:1)

(17) 4-[4-Benzyloxy-phenylamino]-6-methylsulfinyl-pyrimido-[5,4-d]pyrimidin und 4-[4-Benzyloxy-phenylamino]-6-methylsulfonyl-pyrimido[5,4-d]-pyrimidin
Schmelzpunkt: 122-130°C
$R_f$-Wert: 0,35 und 0,48 (Kieselgel; Petrolether/Essigester/Methanol = 10:8:2)

(18) 4-[3-Chlor-4-phenoxy-phenylamino]-6-methylsulfinyl-pyrimido[5,4-d]-pyrimidin und 4-[3-Chlor-4-phenoxy-pheaylamino]-6-methylsulfonyl-pyrimido[5,4-d]pyrimidin
Schmelzpunkt: 189 - 190°C
$R_f$-Wert: 0,55 und 0,70 (Kieselgel; Petrolether/Essigester/Methanol = 10:10:2)

(19) 4-[4-Benzyloxy-3-chlor-phenylamino]-6-methylsulfinylpyrimido[5,4-d]pyrimidin und 4-[4-Benzyloxy-3-chlor-phenylamino]-6-methylsulfonyl-pyrimido[5,4-d]pyrimidin
Schmelzpunkt: 148-150°C
$R_f$-Wert: 0,28 und 0,50 (Kieselgel; Petrolether/Essigester/Methanol = 10:10:2)

Beispiel XXXIV

4-Hydroxymethyl-piperidin

Zu 1,5 g Lithiumaluminiumhydrid in 50 ml Tetrahydrofuran wird eine Lösung von 3,1 g Piperidin-4-carbonsaureethylester in 50 ml Tetrahydrofuran bei Raumtemperatur getropft. Dann wird eine Stunde lang zum Sieden erhitzt. Nach dem Abkühlen auf 0°C werden 10 ml einer 10%igen Kaliumhydroxid-Lösung hinzugegeben. Der Niederschlag wird abfiltriert und noch zweimal mit je 20 ml Tetrahydrofuran gewaschen. Die vereinigten organischen Phasen werden eingedamnpft, der Rückstand wird an Aluminiumoxid mit einem Essigester/Methanol/konz.Ammoniak-Gemisch (10:5:0,05) säulenchromatographisch gereinigt.
Ausbeute: 2.2 g (100 % der Theorie) als gelbes Öl
$R_f$-Wert: 0,50 (Aluminiumoxid; Essigester/Methanol/konz.Ammoniak = 10:5:0,05)

Beispiel XXXV

endo-8-Benzyl-3-benzylamino-8-aza-bicyclo[3.2.1]octan

Zu einer Mischung aus 17,8 g N-Benzyl-tropinon, 8,9 g Benzylamin, 4,8 ml Eisessig und 300 ml absolutem Tetrahydrofuran gibt man bei Raumtemperatur portionsweise 23 g Natriumtriacetoxyborhydrid und rührt 12 Stunden. Dann wird das Solvens im Rotationsverdampfer abdestilliert, der Rückstand mit Natriumhydrogencarbonat-Lösung versetzt und die Mischung dreimal mit Essigester extrahiert. Dann werden die vereinigten organischen Phasen über Natriumsulfat getrocknet und das Solvens im Rotationsverdampfer abdestilliert. Jeweils 7 g des Rückstands von insgesamt 25 g werden an Kieselgel mit Methylenchlorid/Methanol/konz.Ammoniak (30:1:0,1) säulenchromatographisch gereinigt. Ausbeute: 3,1 g (43 % der Theorie),
$R_f$-Wert: 0,31 (Kieselgel; Methylenchlorid/Methanol/konz.Ammoniak = 9:1:0,1)
Schmelzpunkt: 49-51°C

Analog Beispiel XXXV wird folgende Verbindung erhalten:

(1) 8-Dimethylamino-1,4-dioxaspiro[4.5]decan
Hergestellt aus 1,4-Dioxaspiro[4.5]decan-8-on und Dimethylamin. Hellbraunes Öl,
$R_f$-Wert: 0,28 (Kieselgel; Methylenchlorid/Methanol/konz.Ammoniak = 9:1:0,1)

Beispiel XXXVI

endo-3-Amino-8-azabicyclo[3.2.1]octan

3,1 g endo-8-Benzyl-3-benzylamino-8-aza-bicyclo[3.2.1]octan werden in 100 ml Methanol gelöst, mit 3 g Palladiumhydroxid auf Kohle versetzt und bei Raumtemperatur und 3 bar hydriert, bis kein Wasserstoff mehr aufgenommen wird. Dann wird vom Katalysator abfiltriert, das Solvens im Rotationsverdampfer abdestilliert und das verbleibende Öl im Vakuum getrocknet. Das Produkt wird ohne weitere Reinigung verwendet.
Ausbeute: 1,28 g (100 % der Theorie),
$R_f$-Wert: 0,5 (Aluminiumoxid; Methylenchlorid/Methanol/konz.-Ammoniak = 6:1:0,1)

Analog Beispiel XXXVI werden folgende Verbindungen erhalten:

(1) exo-3-Amino-8-azabicyclo[3.2.1]octan
Hergestellt aus der Verbindung des Beispiels XXXVIII.
$R_f$-Wert: 0,17 (Aluminiumoxid; Methylenchlorid/Methanol/konz.-Ammoniak = 15:1:0,1)

(2) 4-Amino-4-methyl-piperidin
Hergestellt aus der Verbindung des Beispiels XL.
$R_f$-Wert: 0,50 (Aluminiumoxid; Methylenchlorid/Methanol/konz.-Ammoniak = 9:1:0,1)

Beispiel XXXVII

N-Benzyl-tropinon-oxim

Zu einer Mischung aus 4,9 g Hydroxylamin-hydrochlorid und 4,24 g Natriumacetat in 80 ml Wasser werden bei 60°C 10 g N-Benzyl-tropinon gegeben und die Mischung noch 2 Stunden bei 60°C gerührt. Dann wird mit wasserfreiem Kaliumcarbonat alkalisch gestellt und die Mischung dreimal mit Methylenchlorid extrahiert. Dann werden die vereinigten organischen Phasen über Natriumsulfat getrocknet und das Solvens im Rotationsverdampfer abdestilliert. Zu dem verbleibenden Öl werden zum Kristallisieren einige ml Ether gegeben; der Feststoff wird mit Petrolether verrieben und abgesaugt.
Ausbeute: 8,7 g (80 % der Theorie),
Scchmelzpunkt: 117-119°C
$R_f$-Wert: 0,19 (Aluminiumoxid; Petrolether/Essigester = 1:1)

Beispiel XXXVIII

exo-3-Amino-8-azabicyclo[3.2.1]octan

Eine Lösung aus 5,35 g N-Benzyl-tropinon-oxim in 100 ml Amylalkohol wird zum Sieden erhitzt. 3,2 g Natrium

werden innerhalb 30 Minuten portionsweise zugegeben, dann wird noch zwei weitere Stunden zum Sieden erhitzt. Nach dem Abkühlen wird unter Eiskühlung mit halbkonzentrirter Salzsäure sauer gestellt und zweimal mit Essigester extrahiert. Die wäßrige Phase wird dann mit 20%iger Natronlauge alkalisch gestellt und dreimal mit Essigester extrahiert. Dann werden die vereinigten organischen Phasen über Kaliumcarbonat getrocknet und das Solvens im Rotationsverdampfer abdestilliert. Der Rückstand wird an Aluminiumoxid mit einem Petrolether/Essigester/Methanol (10:5:1) Gemisch säulenchromatographisch gereinigt.
Ausbeute: 2,72 g (55 % der Theorie) als schwach gelbes Öl,
$R_f$-Wert: 0,31 (Kieselgel; Methylenchlorid/Methanol/konz.Ammoniak = 4:1:0,1)

Beispiel XXXIX

4-Acetylamino-1-benzyl-4-methyl-piperidin

Zu einer Lösung aus 38,8 g 1-Benzyl-4-hydroxy-4-methyl-piperidin in 218 ml Acetonitril werden 190 ml konzentrierte Schwefelsäure innerhalb 2 Stunden unter Rühren zugetropft, wobei die Innentemperatur durch Eisbadkühlung unter 30°C gehalten wird. Dann wird noch 12 Stunden bei Raumtemperatur gerührt. Danach wird auf Eis gegossen und unter Kühlung mit 50%iger Kalilauge auf pH 10 gestellt. Die Mischung wird dreimal mit Methylenchlorid extrahiert. Dann werden die vereinigten organischen Phasen über Natriumsulfat getrocknet und das Solvens im Rotationsverdampfer abdestilliert. Der Rückstand wird mit Petrolether verrieben, abgesaugt und getrocknet und ohne weitere Reinigung in der nächsten Reaktion eingesetzt.
Ausbeute: 39,4 g (84 % der Theorie) gallertartige Masse,
$R_f$-Wert: 0,40 (Kieselgel; Methylenchlorid/Methanol = 9:1)

Beispiel XL

4-Amino-1-benzyl-4-methyl-piperidin

39,4 g 4-Acetylamino-1-benzyl-4-methyl-piperidin werden in 400 ml konzentrierter Salzsäure 3 Tage lang zum Sieden erhitzt. Danach wird im Rotationsverdampfer auf halbes Volumen eingedampft und unter Kühlung mit 50%iger Natronlauge auf pH 12 gestellt. Die Mischung wird dreimal mit Methylenchlorid extrahiert. Dann werden die vereinigten organischen Phasen über Natriumsulfat getrocknet und das Solvens im Rotationsverdampfer abdestilliert. Der Rückstand wird ohne weitere Reinigung in der nächsten Reaktion eingesetzt.
Ausbeute: 25 g (76 % der Theorie) braunes Öl,
$R_f$-Wert: 0,45 (Kieselgel; Methylenchlorid/Methanol/konz.Ammoniak = 9:2:0,1)

Beispiel XLI

3-Chlor-4-phenoxy-nitrobenzol

10 g 3-Chlor-4-fluor-nitrobenzol, 7,8 g wasserfreies Kaliumcarbonat, 6,5 g Phenol und 50 ml Dimethylformamid werden 5 Stunden lang auf 135°C erhitzt. Danach wird von festen Bestandteilen abgesaugt und die Lösung im Rotationsverdampfer eingedampft. Der Rückstand wird in Methylenchlorid gelöst und mit 2N Natronlauge extrahiert. Dann wird die organische Phase über Natriumsulfat getrocknet und das Solvens im Rotationsverdampfer abdestilliert. Der Rückstand wird ohne weitere Reinigung in der nächsten Reaktion eingesetzt.
Ausbeute: 14,7 g (100 % der Theorie) hellbraunes Öl,
$R_f$-Wert: 0,50 (Kieselgel; Petrolether/Essigester = 5:1)
Analog Beispiel XLI wird folgende Verbindung erhalten:

(1) 4-Benzyloxy-3-chlor-nitrobenzol
$R_f$-Wert: 0,63 (Kieselgel; Petrolether/Essigester = 5:2) Schmelzpunkt: 120-122°C

Beispiel XLII

3-Chlor-4-phenoxy-anilin

4,0 g 3-Chlor-4-phenoxy-nitrobenzol werden in 40 ml Essigester gelöst, mit 0,4 g Platinoxid versetzt und bei 18°C und 1 bar hydriert, bis das Edukt verbraucht ist. Dann wird vom Katalysator abfiltriert, das Solvens im Rotationsverdampfer abdestilliert und das verbleibende Öl im Vakuum getrocknet. Das Produkt wird ohne weitere Reinigung

verwendet.
Ausbeute: 3,6 g (100 % der Theorie),
$R_f$-Wert: 0,6 (Aluminiumoxid; Petrolether/Essigester = 2:1)
Analog Beispiel XLII wird folgende Verbindung erhalten:

(1) 4-Benzyloxy-3-chlor-anilin
$R_f$-Wert: 0,70 (Aluminiumoxid; Petrolether/Essigester = 2:1)

Beispiel XLIII

4-Dimethylamino-cyclohexanon

45,3 g 8-Dimethylamino-1,4-dioxaspiro[4.5]decan (Verbindung 1 des Beispiels XXXV) werden in 400 ml 2N Salzsäure 3 Tage lang bei Raumtemperatur gerührt. Dann wird die Lösung dreimal mit Diethylether extrahiert, die wäßrige Phase mit Kaliumcarbonat gesättigt und fünfmal mit Essigester extrahiert. Dann werden die vereinigten organischen Phasen über Natriumsulfat getrocknet und das Solvens im Rotationsverdampfer abdestilliert. Der Rückstand wird ohne weitere Reinigung in der nächsten Reaktion eingesetzt.
Ausbeute: 30,6 g (88 % der Theorie) hellbraunes Öl,
$R_f$-Wert: 0,53 (Kieselgel; Methylenchlorid/Methanol/konz.Ammoniak = 10:2:0,1)

Beispiel XLIV

cis/trans-N-Benzyl-N-(4-dimethylamino-cyclohexyl)amin

Eine Mischung aus 67,8 g 4-Dimethylamino-cyclohexanon, 53 ml Benzylamin, 79 g Molsieb 4A und 500 ml absolutem Toluol wird bei Raumtemperatur 12 Stunden gerührt. Dann wird vom Molsieb abfiltriert und das Filtrat im Rotationsverdampfer eingedampft. Der Rückstand wird in 400 ml Methanol gelöst, dann gibt man bei Raumtemperatur portionsweise 5,5 g Natriumborhydrid hinzu und rührt 5 Stunden. Dann wird das Solvens im Rotationsverdampfer abdestilliert, der Rückstand mit 300 ml Methylenchlorid und 200 ml Eiswasser versetzt, die Mischung mit Zitronensäure auf pH 5 und dann mit Natronlauge auf pH 10 gestellt. Nach der Phasentrennung wird die Mischung dreimal mit Methylenchlorid extrahiert, dann werden die vereinigten organischen Phasen mit gesättigter Natriumchlorid-Lösung gewaschen, über Natriumsulfat getrocknet und das Solvens im Rotationsverdampfer abdestilliert.
Ausbeute: 107 g (95 % der Theorie) eines braunen Öls, das laut NMR-Spektren etwa ein 60/40-cis/trans-Gemisch ist.
$R_f$-Wert: 0,27 (trans) und 0,37 (cis)(Kieselgel; Methylenchlorid/Methanol/konz.Ammoniak = 10:2:0,1)

Beispiel XLV

trans-N-Benzyl-N-(4-dimethylamino-cyclohexyl)amin-fumarat

Zur Lösung von 93,7 g der Verbindung des Beispiels XLIV in 200 ml absolutem Tetrahydrofuran wird bei 50°C eine Lösung von 25,5 g Fumarsäure in 720 ml absolutem Tetrahydrofuran schnell zugegeben und die Mischung noch weitere 3 Stunden gerührt. Der ausgefallene Feststoff wird abgesaugt und mit Tetrahydrofuran nachgewaschen und dann in 1,5 l Isopropanol aufgeschlämmt und zum Sieden erhitzt. Die Mischung wird heiß filtriert, der Rückstand mit Ether verrieben und erneut abgesaugt.
Ausbeute: 66,9 g (70 % der Theorie) eines grauen Feststoffs, der laut NMR-Spektren zu mindestens 95% aus der trans-Verbindung besteht.
Schmelzpunkt: 209-215°C

Beispiel XLVI

trans-4-Dimethylamino-cyclohexylamin

12 g des unter Beispiel XLV erhaltenen Fumarats werden in 2N Natronlauge aufgenommen und die Lösung dreimal mit Methylenchlorid extrahiert, dann werden die vereinigten organischen Phasen mit gesättigter Natriumchlorid-Lösung gewaschen, über Natriumsulfat getrocknet und das Solvens im Rotationsverdampfer abdestilliert. Der Rückstand wird in 150 ml Methanol gelöst, mit 4,5 g Palladiumhydroxid auf Kohle versetzt und bei Raumtemperatur und 3 bar hydriert, bis kein Wasserstoff mehr aufgenommen wird. Dann wird vom Katalysator abfiltriert, das Solvens im Rotationsverdampfer abdestilliert und das verbleibende Öl im Vakuum getrocknet. Das Produkt wird ohne weitere

Reinigung verwendet.
Ausbeute: 4,26 g (86 % der Theorie) eines braunen Öls,

Beispiel XLVII

3-Amino-1-tert.butyloxycarbonyl-piperidin

1,0 g 3-Aminocarbonyl-1-tert.butyloxycarbonyl-piperidin (Verbindung 2 des Beispiels XII) werden unter Eiskühlung portionsweise zu 9 ml frisch hergestellter Natriumhypobromit-Lösung gegeben. Nach 3 Stunden bei Raumtemperatur gibt man zu der Lösung gesättigte Natriumsulfit-Lösung, bis eine Trübung entsteht. Man sättigt mit Kaliumcarbonat und extrahiert dreimal mit Essigester, dann werden die vereinigten organischen Phasen über Natriumsulfat getrocknet und das Solvens im Rotationsverdampfer abdestilliert.
Ausbeute: 693 mg (79 % der Theorie) eines farblosen Öls,
$R_f$-Wert: 0,44 (Aluminiumoxid; Methylenchlorid/Methanol = 10:0,6)

Beispiel 1

4-[(3-Chlor-4-fluor-phenyl)amino]-6-[(trans-4-methoxycarbonylcyclohexyl)amino]-pyrimido[5,4-d]pyrimidin

Zu 0,7 g eines Gemisches aus 4-[(3-Chlor-4-fluor-phenyl)amino]-6-methylsulfinyl-pyrimido[5,4-d]pyrimidin und 4-[(3-Chlor-4-fluor-phenyl)amino]-6-methylsulfonyl-pyrimido[5,4-d]pyrimidin in 15 ml Dimethylformamid werden 1,9 g trans-4-Amino-cyclohexancarbonsäuremethylester und 1,8 ml N-Ethyl-diisopropylamin gegeben und die Mischung zwei Stunden auf 80°C erhitzt. Das Reaktionsgemisch wird eingedampft, mit Wasser versetzt und der Feststoff abgesaugt. Das Rohprodukt wird durch Chromatographie über eine Kieselgelsäule mit Petrolether/Essigester (6:10) gereinigt.
Ausbeute: 0,69 g (80% der Theorie),
Schmelzpunkt: 204-206°C
$R_f$-Wert: 0,44 (Kieselgel; Petrolether/Essigester = 6:10)
Analog Beispiel 1 werden folgende Verbindungen erhalten:

(1) 4-[(3-Chlor-4-fluor-phenyl)amino]-6-[4-methoxycarbonylamino-1-piperidinyl)-pyrimido[5,4-d]pyrimidin
Hergestellt aus den Verbindungen des Beispiels XXXII durch Umsetzung mit 4-Aminopiperidin und nachfolgende Umsetzung mit Chlorameisensäuremethylester.
Schmelzpunkt: 195-197°C
$R_f$-Wert: 0,50 (Kieselgel; Petrolether/Essigester/Methanol = 10:10:1)

(2) 4-[(3-Chlor-4-fluor-phenyl)amino]-6-[1-tert.butyloxycarbonyl-4-piperidinyl-amino] -pyrimido [5,4-d] pyrimidin
Hergestellt aus den Verbindungen der Beispiele XXXII und XII. Schmelzpunkt: 196-200°C (Zersetzung)
$R_f$-Wert: 0,50 (Kieselgel; Petrolether/Essigester/Methanol = 10:8:1)

(3) 4-[(4-Amino-3,5-dibromphenyl)amino]-6-[4-amino-1-piperidinyl]-pyrimido[5,4-d]pyrimidin
Schmelzpunkt: 204-206°C
$R_f$-Wert: 0,71 (Aluminiumoxid; Methylenchlorid/Methanol = 10:1)

(4) (3'RS)-4-[(3-Chlor-4-fluor-phenyl)amino]-6-[(3'-chinuclidinyl)amino]pyrimido[5,4-d]pyrimidin
Schmelzpunkt: 201-203°C
$R_f$-Wert: 0,23 (Aluminiumoxid; Petrolether/Essigester/Methanol = 10:5:2)

(5) 4-[(3-Chlor-4-fluor-phenyl)amino]-6-[4-formylamino-1-piperidinyl]-pyrimido[5,4-d]pyrimidin
Hergestellt aus den Verbindungen des Beispiels XXXII durch Umsetzung mit 4-Aminopiperidin und nachfolgende Umsetzung mit Ameisensäuremethylester.
Schmelzpunkt: 201-203°C
$R_f$-Wert: 0,24 (Kieselgel; Petrolether/Essigester/Methanol = 10:5:2)

(6) 4-[(3-Chlor-4-fluor-phenyl)amino]-6-[2-amino-ethylamino]-pyrimido[5,4-d]pyrimidin
Schmelzpunkt: 173-175°C
$R_f$-Wert: 0,41 (Aluminiumoxid; Methylenchlorid/Methanol = 70:1)

(7) 4-[(3-Chlor-4-fluor-phenyl)amino]-6-[4-(aminomethyl)-2-piperidinyl]-pyrimido[5,4-d]pyrimidin
Schmelzpunkt: 165°C
$R_f$-Wert: 0,48 (Aluminiumoxid; Methylenchlorid/Methanol = 15:1)

(8) 4-[(3-Chlor-4-fluor-phenyl)amino]-6-[1-methoxycarbonyl-4-piperidinyl-amino]-pyrimido[5,4-d]pyrimidin
Hergestellt aus 4-[(3-Chlor-4-fluor-phenyl)amino]-6-[1-tert.butyloxycarbonyl-4-piperidinyl-amino]-pyrimido[5,4-d] pyrimidin durch Umsetzung mit Trifluoressigsäure und nachfolgende Umsetzung mit Chlorameisensäuremethylester.
Schmelzpunkt: 213-215°C
$R_f$-Wert: 0,19 (Kieselgel; Petrolether/Essigester/Methanol = 20:10:1)

(9) 4-[(3-Chlor-4-fluor-phenyl)aminol-6-[3-amino-propylamino]-pyrimido[5,4-d]pyrimidin
Schmelzpunkt: 177-179°C
$R_f$-Wert: 0,50 (Aluminiumoxid; Methylenchlorid/Methanol = 7:1)

(10) 4-[(3-Chlor-4-fluor-phenyl)amino]-6-[4-amino-butylamino]-pyrimido[5,4-d]pyrimidin
Schmelzpunkt: 160-162°C
$R_f$-Wert: 0,60 (Aluminiumoxid; Methylenchlorid/Methanol = 7:1)

(11) 4-[(3-Chlor-4-fluor-phenyl)amino]-6-[2-amino-1-propylamino]-pyrimido[5,4-d]pyrimidin
Schmelzpunkt: 168-170°C
$R_f$-Wert: 0,20 (Kieselgel ; Petrolether/Essigester/Methanol/konz. Ammoniak = 10:10:3:0.05)

(12) 4-[(3-Chlor-4-fluor-phenyl)amino]-6-[4-methyl-1-homopiperazinyl]-pyrimido[5,4-d]pyrimidin
Schmelzpunkt: 122-124°C
$R_f$-Wert: 0,60 (Aluminiumoxid; Petrolether/Essigester/Methanol = 40:40:1)

| | | | |
|---|---|---|---|
| Ber. | C 55,74 | H 4,93 | N 25,27 |
| Gef. | 55,99 | 5,12 | 25,13 |

(13) 4-[(3-Chlor-4-fluor-phenyl)amino]-6-[1-homopiperazinyl]-pyrimido[5,4-d]pyrimidin
Schmelzpunkt: >300°C
$R_f$-Wert: 0,40 (Kieselgel; petrolether/Essigester/Methanol = 10:10:3)

(14) 4-[(3-Chlor-4-fluor-phenyl)amino]-6-[4-amino-benzylamino]-pyrimido[5,4-d]pyrimidin
Schmelzpunkt: 218-220°C
$R_f$-Wert: 0,58 (Aluminiumoxid; Petrolether/Essigester/Methanol = 10:10:2)

(15) 4-[(4-Amino-3,5-dichlor-phenyl)amino3-6-[(trans-4-methoxycarbonyl-cyclohexyl)amino]-pyrimido[5,4-d]pyrimidin Schmelzpunkt: 219-224°C
$R_f$-Wert: 0,39 (Kieselgel; Petrolether/Essigester = 8:10)

(16) 4-[(4-Amino-3,5-dichlor-phenyl)amino]-6-[(trans-4-carboxycyclohexyl)amino]-pyrimido[5,4-d]pyrimidin
Hergestellt aus 4-[(4-Amino-3,5-dichlor-phenyl)amino]-6-[(trans-4-methoxycarbonyl-cyclohexyl)amino]-pyrimido [5,4-d]-pyrimidin durch Verseifung mit Natronlauge.
Schmelzpunkt: 339°C
$R_f$-Wert: 0,37 (Kieselgel; Petrolether/Essigester/Methanol = 10:10:3)

(17) 4-[(4-Amino-3,5-dichlor-phenyl)amino]-6-[trans-4-(morpholinocarbonyl)cyclohexylamino]-pyrimido[5,4-d]pyrimidin Hergestellt aus 4-[(4-Amino-3,5-dichlor-phenyl)amino]-6-[(trans-4-carboxy-cyclohexyl)amino]-pyrimido [5,4-d] pyrimidin durch Umsetzung mit O-(Benzotriazol-1-yl)-N,N,N',N'-tetramethyluronium-tetrafluoroborat, Triethylamin und Morpholin. Schmelzpunkt: 204-207°C
$R_f$-Wert: 0,53 (Kieselgel; Petrolether/Essigester/Methanol = 10:10:4)

(18) 4-[(4-Amino-3,5-dichlor-phenyl)amino]-6-[trans-4-(pyrrolidinocarbonyl)cyclohexylaminol-pyrimido[-5,4-d]pyrimidin Hergestellt aus 4-[(4-Amino-3,5-dichlor-phenyl)amino]-6-[(trans-4-carboxy-cyclohexyl)amino]-pyrimido [5,4-d]pyrimidin durch Umsetzung mit O-(Benzotriazol-1-yl)-N,N,N',N'-tetramethyluronium-tetrafluoroborat,

Triethylamin und Pyrrolidin.
Schmelzpunkt: 228-230°C
$R_f$-Wert: 0,43 (Kieselgel; Petrolether/Essigester/Methanol = 10:10:4)

| | | | | |
|---|---|---|---|---|
| Ber. | C 55,09 | H 5,23 | N 22,35 | Cl 14,14 |
| Gef. | 55,07 | 5,19 | 22,33 | 14,22 |

(19) 4-[(3-Chlor-4-fluor-phenyl)amino]-6-[4-(4-piperidinyl)-1-piperidinyl]-pyrimido[5,4-d]pyrimidin
Schmelzpunkt: 170°C (Zersetzung)
$R_f$-Wert: 0,45 (Aluminiumoxid; Methylenchlorid/Essigester/Methanol/konz.Ammoniak = 10:4:1:0.05)

(20) 4-[(3-Chlor-4-fluor-phenyl)amino)-6-[1-(1-piperidinyl)-2-propyl-amino]-pyrimido[5,4-d]pyrimidin
Schmelzpunkt: 188-190°C
$R_f$-Wert: 0,38 (Aluminiumoxid; Petrolether/Essigester = 1:1)

(21) 4-[(3-Chlor-4-fluor-phenyl)amino]-6-[1-(N,N-diethylamino)-2-propyl-amino]-pyrimido[5,4-d]pyrimidin
Schmelzpunkt: 193-195°C
$R_f$-Wert: 0,55 (Aluminiumoxid; Petrolether/Essigester = 1:1)

(22) 4-[(3-Chlor-4-fluor-phenyl)amino]-6-[4-(aminomethyl)-cyclohexyl-methyl-amino]-pyrimido[5,4-d]pyrimidin
Schmelzpunkt: 155-160°C
$R_f$-Wert: 0,60 (Aluminiumoxid; Methylenchlorid/Essigester/Methanol/konz.Ammoniak = 10:5:2:0.05)

(23) 4-[(3-Chlor-4-fluor-phenyl)amino]-6-[N-ethyl-N-(2-hydroxyethyl)amino]-pyrimido[5,4-d]pyrimidin
Schmelzpunkt: 194-196°C
$R_f$-Wert: 0,45 (Kieselgel; Petrolether/Essigester/Methanol = 10:10:1)

(24) 4-[(3-Chlor-4-fluor-phenyl)amino]-6-[4-chinuclidinylmethyl-amino]-pyrimido[5,4-d]pyrimidin
Hergestellt aus den Verbindungen der Beispiele XXXII und XIV (1) .
Schmelzpunkt: 221-223°C
$R_f$-Wert: 0,53 (Aluminiumoxid; Methylenchlorid/Methanol = 20:1)

(25) 4-[(3-Chlor-4-fluor-phenyl)amino]-6-[2,2-dimethoxyethylamino]-pyrimido[5,4-d]pyrimidin
Schmelzpunkt: 169-171°C
$R_f$-Wert: 0,47 (Kieselgel; Petrolether/Essigester/Methanol = 10:8:1)

(26) 4-[(3-Chlor-4-fluor-phenyl)aminol-,6-[(1-tert.butyloxycarbonyl-4-piperidinyl)-methyl-amino)-pyrimido[5,4-d]-pyrimidin
Hergestellt aus den Verbindungen der Beispiele XXXII und XIII. Schmelzpunkt: 210-213°C
$R_f$-Wert: 0,38 (Kieselgel; Petrolether/Essigester/Methanol = 10:5:1)

| | | | | |
|---|---|---|---|---|
| Ber. | C 56,61 | H 5,58 | N 20,09 | Cl 7,26 |
| Gef. | 56,68 | 5,52 | 19,80 | 7,33 |

(27) 4-[(3-Chlor-4-fluor-phenyl)amino]-6-[2-amino-2-methyl-1-propylamino]-pyrimido[5,4-d]pyrimidin
Schmelzpunkt: 205-207°C
$R_f$-Wert: 0,52 (Aluminiumoxid; Methylenchlorid/Methanol = 10:1)

(28) 4-[(3-Chlor-4-fluor-phenyl)amino]-6-[N-methyl-N-(1-methyl-4-piperidinyl) -amino] -pyrimido [5,4-d]pyrimidin
Schmelzpunkt: 197-199°C
$R_f$-Wert: 0,56 (Aluminiumoxid; Petrolether/Essigester/Methanol = 3:10:0,2)

(29) 4-[(3-Chlor-4-fluor-phenyl)amino]-6-[4-piperidinyl-methylamino]-pyrimido[5,4-d]pyrimidin
Hergestellt aus 4-[(3-Chlor-4-fluor-ghenyl)amino]-6-[(1-tert.butyloxycarbonyl-4-piperidinyl)-methyl-amino]-pyrimido[5,4-d]-pyrimidin durch Umsetzung mit Trifluoressigsäure.
Schmelzpunkt: 204-206°C
$R_f$-Wert: 0,42 (Aluminiumoxid; Methylenchlorid/Methanol = 10:1)

(30) 4-[(3-Chlor-4-fluor-phenyl)amino]-6-[4-(morpholino)-1-piperidinyl]-pyrimido[5,4-d]pyrimidin
Hergestellt aus den Verbindungen des Beispiels XXXII durch Umsetzung mit 4-Hydroxypiperidin, Oxidation mit Dess-Martin-Reagenz und anschließende reduktive Aminierung mit Morpholin und Natriumcyanoborhydrid.
Schmelzpunkt: 222-224°C
$R_f$-Wert: 0,40 (Kieselgel; Petrolether/Essigester/Methanol = 10:10:2)

(31) 4-[(3-Chlor-4-fluor-phenyl)amino]-6-[4-(1-pyrrolidinyl)-1-piperidinyl]-pyrimido[5,4-d]pyrimidin
Hergestellt aus 4-[(3-Chlor-4-fluor-phenyl)amino]-6-[4-hydroxy-1-piperidinyl]-pyrimido[5,4-d]pyrimidin durch Oxidation mit Dess-Martin-Reagenz und anschließende reduktive Aminierung mit Pyrrolidin und Natriumcyanoborhydrid.
Schmelzpunkt: 202-204°C
$R_f$-Wert: 0,58 (Aluminiumoxid; Petrolether/Essigester/Methanol = 10:10:1)

(32) 4-[(3-Chlor-4-fluor-phenyl)amino]-6-[4-(3-methoxyphenyl)-1-piperazinyl]-pyrimido[5,4-d]pyrimidin
Schmelzpunkt: 185-188°C
$R_f$-Wert: 0,63 (Kieselgel; Petrolether/Essigester/Methanol = 10:10:0,5)

(33) 4-[(3-Chlor-4-fluor-phenyl)amino]-6-[4-(2-methoxyphenyl)-1-piperazinyl]-pyrimido[5,4-d]pyrimidin
Schmelzpunkt: 150-152°C
$R_f$-Wert: 0,48 (Kieselgel; Petrolether/Essigester = 1:2)

(34) 4-[(3-Chlor-4-fluor-phenyl)amino]-6-[3-aminocarbonyl-1-piperidinyl]-pyrimido[5,4-d]pyrimidin
Schmelzpunkt: 264-267°C
$R_f$-Wert: 0,50 (Kieselgel; Petrolether/Essigester/Methanol = 10:8:3)

(35) 4-[(3-Chlor-4-fluor-phenyl)amino]-6-[2-(4-tert.butyloxycarbonyl-1-piperazinyl-)ethylamino]-pyrimido[5,4-d]pyrimidin Hergestellt aus den Verbindungen des Beispiels XXXII durch Umsetzung mit 1-(2-Aminoethyl)piperazin und anschließende Umsetzung mit Di-tert.-butyldicarbonat und Triethylamin. Schmelzpunkt: sintert bei 100-106°C
$R_f$-Wert: 0,42 (Kieselgel; Petrolether/Essigester/Methanol = 10:10:2)

(36) 4-[(3-Chlor-4-fluor-phenyl)amino]-6-[4-(4-hydroxyphenyl)-2-methyl-1-pyrrolidinyl]-pyrimido[5,4-d]pyrimidin
Schmelzpunkt: 223-228°C
$R_f$-Wert: 0,56 (Kieselgel; Petrolether/Essigester/Methanol = 10:5:1)

| | | | | |
|---|---|---|---|---|
| Ber. | C 61,27 | H 4,47 | N 18,64 | Cl 7,86 |
| Gef. | 61,14 | 4,42 | 18,36 | 7,81 |

(37) 4-[(3-Chlor-4-fluor-phenyl)amino]-6-[1-phenoxy-2-propylamino]-pyrimido[5,4-d]pyrimidin
Schmelzpunkt: 163-167°C
$R_f$-Wert: 0,53 (Kieselgel; Petrolether/Essigester/Methanol = 10:5:1)

| | | | | |
|---|---|---|---|---|
| Ber. | C 59,37 | H 4,27 | N 19,78 | Cl 8,34 |
| Gef. | 59,07 | 4,37 | 19,29 | 8,24 |

(38) 4-[(3-Chlor-4-fluor-phenyl)amino]-6-[3-(aminomethyl)cyclohexylmethyl-amino]-pyrimido[5,4-d]pyrimidin
Schmelzpunkt: 145-148°C
$R_f$-Wert: 0,48 (Aluminiumoxid; Methylenchlorid/Methanol = 10:1)

(39) 4-[(3-Chlor-4-fluor-phenyl)amino]-6-[1-(4-aminophenyl)-2-propyl-amino]-pyrimido[5,4-d]pyrimidin
Schmelzpunkt: 172-176°C
$R_f$-Wert: 0,59 (Kieselgel; Petrolether/Essigester/Methanol = 10:10:3)

(40) 4-[(3-Chlor-4-fluor-phenyl)amino]-6-[4-(4-methoxyphenyl)-1-piperazinyl]-pyrimido[5,4-d]pyrimidin
Schmelzpunkt: 176-178°C
$R_f$-wert: 0,50 (Kieselgel; Petrolether/Essigester = 1:1)

(41) 4-[(3-Chlor-4-fluor-phenyl)amino]-6-[4-(1-methyl-4-piperidinyl)-1-piperidinyl]-pyrimido[5,4-d]pyrimidin

Hergestellt aus 4-[(3-Chlor-4-fluor-phenyl)amino]-6-[4-(4-piperidinyl)-1-piperidinyl]-pyrimido[5,4-d]pyrimidin durch reduktive Aminierung mit Formaldehyd und Natriumcyanoborhydrid. Schmelzpunkt: 159-162°C
$R_f$-Wert: 0,58 (Aluminiumoxid; Petrolether/Essigester/Methanol = 10:10:1)

(42) 4-[(3-Chlor-4-fluor-phenyl)amino]-6-[1-benzyl-4-(1-azacycloheptyl)-amino]-pyrimido[5,4-d] pyrimidin
Hergestellt aus den Verbindungen der Beispiele XXXII und XX. Schmelzpunkt: 147-149°C
$R_f$-Wert: 0,43 (Kieselgel; Petrolether/Essigester/Methanol = 10:10:3)

| | | | | |
|---|---|---|---|---|
| Ber. | C 62,82 | H 5,27 | N 20,51 | Cl 7,42 |
| Gef. | 63,04 | 5,29 | 20,24 | 7,42 |

(43) 4-[(3-Tetrafluorethoxy-phenyl)amino]-6-[trans-4-hydroxycyclohexylamino]-pyrimido[5,4-d]pyrimidin
Schmelzpunkt: 218-220°C
$R_f$-Wert: 0,37 (Kieselgel; Petrolether/Essigester/Methanol = 10:10:1)

(44) 4-[(4-Benzyl-phenyl)amino]-6-[trans-4-hydroxy-cyclohexylamino]-pyrimido[5,4-d]pyrimidin
Schmelzpunkt: 232-234°C
$R_f$-Wert: 0,45 (Kieselgel; Petrolether/Essigester/Methanol = 10:10:1)

(45) 4-[(3-Benzyloxy-phenyl)amino]-6-[trans-4-hydroxy-cyclohexylamino]-pyrimido[5,4-d]pyrimidin
Schmelzpunkt: 227-230°C
$R_f$-Wert: 0,30 (Kieselgel; Petrolether/Essigester/Methanol = 10:10:1)

| | | | |
|---|---|---|---|
| Ber. | C 67,85 | H 5,92 | N 18,99 |
| Gef. | 67,22 | 5,98 | 18,35 |

(46) 4-[(3-Chlor-4-fluor-phenyl)amino]-6-[4-(1-methyl-4-piperazinyl)-cyclohexylamino]-pyrimido[5,4-d]pyrimidin
Hergestellt aus den Verbindungen des Beispiels XXXII durch Umsetzung mit 4-Aminocyclohexanol, Oxidation mit Dess-Martin-Reagenz und anschließende reduktive Aminierung mit 1-Methylpiperazin und Natriumcyanoborhydrid.
Schmelzpunkt: 194-196°C
$R_f$-Wert: 0,58 (Aluminiumoxid; Methylenchlorid/Essigester/Methanol = 10:5:1)

(47) 4-[(3-Hydroxymethyl-phenyl)amino]-6-[trans-4-hydroxycyclohexylamino]-pyrimido[5,4-d]pyrimidin
Schmelzpunkt: 255-257°C
$R_f$-Wert: 0,30 (Kieselgel; Petrolether/Essigester/Methanol = 10:10:3)

(48) 4-[(3-Chlor-4-fluor-phenyl)amino]-6-[4-hydroxy-1-piperidinyl]-pyrimido[5,4-d]pyrimidin
Schmelzpunkt: 243-246°C
$R_f$-Wert: 0,45 (Aluminiumoxid; Methylenchlorid/Essigester/Methanol = 10:4:1)

(49) 4-[(3-Chlor-4-fluor-phenyl)amino]-6-[1-piperidinyl]-pyrimido[5,4-d]pyrimidin
Schmelzpunkt: 196-198°C
$R_f$-Wert: 0,52 (Kieselgel; Petrolether/Essigester = 10:5)

(50) 4-[(3-Chlor-4-fluor-phenyl)amino)-6-[(1-cyano-4-piperidinyl)amino]-pyrimido[5,4-d]pyrimidin
Hergestellt aus 4-[(3-Chlor-4-fluor-phenyl)amino]-6-[1-tert.butyloxycarbonyl-4-piperidinyl-amino)-pyrimido[5,4-d] pyrimidin durch Umsetzung mit Trifluoressigsäure und anschließende Umsetzung mit Bromcyan.
Schmelzpunkt: 245-247°C
$R_f$-Wert: 0,59 (Kieselgel; Petrolether/Essigester/Methanol = 10:10:3)

(51) 4-[(3-Chlor-4-fluor-phenyl)amino]-6-[(1-methyl-4-piperidinyl)-methylamino]-pyrimido[5,4-d]pyrimidin
Hergestellt aus 4-[(3-Chlor-4-fluor-phenyl)amino]-6-[4-piperidinyl-methyl-amino]-pyrimido[5,4-d]pyrimidin durch Umsetzung mit Trifluoressigsäure und anschließende reduktive Aminierung mit Formaldehyd und Natriumcyanoborhydrid.
Schmelzpunkt: 198-201°C
$R_f$-Wert: 0,65 (Aluminiumoxid; Methylenchlorid/Methanol = 20:1)

(52) 4-[(3-Chlor-4-fluor-phenyl)amino]-6-[2-(4-(morpholinocarbonyl)-1-piperazinyl)-ethylamino]-pyrimido[5,4-d] pyrimidin Hergestellt aus den Verbindungen des Beispiels XXXII durch Umsetzung mit 1-(2-Aminoethyl)piperazin und anschließende Umsetzung mit 4-Morpholincarbonylchlorid und Triethylamin. Schmelzpunkt: 163-168°C (sintert ab 98°C)
$R_f$-Wert: 0,58 (Aluminiumoxid; Methylenchlorid/Methanol = 10:0,4)

(53) 4-[(3-Chlor-4-fluor-phenyl)amino]-6-amino-pyrimido-[5,4-d]pyrimidin
Schmelzpunkt: 300-301°C
$R_f$-Wert: 0,41 (Aluminiumoxid; Methylenchlorid/Methanol = 20:1)

(54) 4-[(3-Chlor-4-fluor-phenyl)amino)-6-[5-aminopentyl-amino)-pyrimido[5,4-d]pyrimidin
Schmelzpunkt: 253-155°C
$R_f$-Wert: 0,52 (Aluminiumoxid; Methylenchlorid/Methanol = 5:1)

(55) 4-[(3-Chlor-4-fluor-phenyl)amino]-6-[1-azacycloheptyl]-pyrimido[5,4-d]pyrimidin
Schmelzpunkt: 87-90°C
$R_f$-Wert: 0,60 (Kieselgel; Methylenchlorid/Methanol = 25:1)

(56) 4-[(3-Chlor-4-fluor-phenyl)amino]-6-[4-amino-1-azacycloheptyl]-pyrimido[5,4-d]pyrimidin
Hergestellt aus den Verbindungen der Beispiele XXXII und XXI. Schmelzpunkt: 122-124°C
$R_f$-Wert: 0,59 (Kieselgel; Methylenchlorid/Methanol/konz. Ammoniak = 80:20:2)

(57) 4-[(3-Chlor-4-fluor-phenyl)amino]-6-[1-tert.butyloxycarbonyl-4-(azacycloheptyl)-amino]-pyrimido[5,4-d]pyrimidin Hergestellt aus den Verbindungen der Beispiele XXXII und XIII. Schmelzpunkt: 175-177°C
$R_f$-Wert: 0,33 (Kieselgel; Petrolether/Essigester/Methanol = 10:5:1)

| | | | | |
|---|---|---|---|---|
| Ber. | C 56,61 | H 5,58 | N 20,09 | Cl 7,27 |
| Gef. | 56,89 | 5,58 | 19,81 | 7,37 |

(58) 4-[(3-Chlor-4-fluor-phenyl)amino]-6-[4-(azacycloheptyl)-amino]-pyrimido[5,4-d]pyrimidin
Hergestellt aus 4-[(3-Chlor-4-fluor-phenyl)amino]-6-[1-tert.-butyloxycarbonyl-4-(azacycloheptyl)-amino]-pyrimido [5,4-d]pyrimidin durch Umsetzung mit Trifluoressigsäure.
Schmelzpunkt: 266-268°C
$R_f$-Wert: 0,23 (Kieselgel; Methylenchlorid/Methanol/konz. Ammoniak = 70:30:2)

(59) 4-(3-Chlor-4-fluor-phenyl)amino]-6-[trans-4-aminomethylcyclohexylamino]-pyrimido[5,4-d]pyrimidin
Hergestellt aus den Verbindungen der Beispiele XXXII und XVIII und anschließende Umsetzung mit Trifluoressigsäure. Schmelzpunkt: 170-173°C
$R_f$-Wert: 0,37 (Aluminiumoxid; Methylenchlorid/Methanol = 10:1)

(60) 4-[(3-Chlor-4-fluor-phenyl)amino]-6-[trans-4-(benzyloxycarbonylamino)-cyclohexylmethyl-amino]-pyrimido [5,4-d]pyrimidin
Hergestellt aus den Verbindungen der Beispiele XXXII und XVII. Schmelzpunkt: 176-179°C
$R_f$-Wert: 0,47 (Kieselgel; Petrolether/Essigester/Methanol = 10:8:2)

| | | | | |
|---|---|---|---|---|
| Ber. | C 60,50 | H 5,08 | N 18,29 | Cl 6,61 |
| Gef. | 60,59 | 5,13 | 18,13 | 6,75 |

(61) 4-[(3-Chlor-4-fluor-phenyl)amino]-6-[3-aminomethyl-1-piperidinyl]-pyrimido[5,4-d]pyrimidin
Hergestellt aus den Verbindungen der Beispiele XXXII und XIV. Schmelzpunkt: 132-137°C
$R_f$-Wert: 0,67 (Kieselgel; Methylenchlorid/Methanol/konz. Ammoniak = 10:2:0.05)

(62) 4-[(4-Amino-2,5-dichlor-phenyl)amino]-6-[trans-4-hydroxycyclohexylamino]-pyrimido[5,4-d]pyrimidin
Schmelzpunkt: 268-270°C
$R_f$-Wert: 0,43 (Kieselgel; Petrolether/Essigester/Methanol = 10:10:2)

(63) 4-[4-Biphenylylamino]-6-(trans-4-hydroxy-cyclohexylamino]-pyrimido[5,4-d]pyrimidin

Schmelzpunkt: 246-248°C
$R_f$-Wert: 0,37 (Kieselgel; Petrolether/Essigester/Methanol = 10:10:3)

(64) 4-[3-Phenoxy-phenylamino]-6-[trans-4-hydroxy-cyclohexylamino]-pyrimido[5,4-d]pyrimidin
Schmelzpunkt: 204-206°C
$R_f$-Wert: 0,35 (Kieselgel; Petrolether/Essigester/Methanol = 10:10:2)

(65) 4-[(3-Chlor-4-fluor-phenyl)amino]-6-[(trans-4-aminocyclohexyl)methylamino]-pyrimido[5,4-d]pyrimidin
Hergestellt aus 4-[(3-Chlor-4-fluor-phenyl)amino]-6-[trans-4-(benzyloxycarbonylamino)-cyclohexylmethyl-amino)-pyrimido-[5,4-d]pyrimidin durch katalytische Hydrierung.
Schmelzpunkt: 166-169°C
$R_f$-Wert: 0,53 (Aluminiumoxid; Methylenchlorid/Methanol/konz. Ammoniak = 10:1:0,05)
Massenspektrum: $M^+$ = 401/403 (Cl)

(66) 4-[(3-Chlor-4-fluor-phenyl)amino]-6-[3-(aminocarbonylmethyl)-1-piperidinyl]-pyrimido[5,4-d]pyrimidin
Hergestellt aus den Verbindungen der Beispiele XXXII und VII. Schmelzpunkt: 247-248°C
$R_f$-Wert: 0,38 (Kieselgel; Petrolether/Essigester/Methanol = 10:8:2)
Massenspektrum: $M^+$ = 415/417 (Cl)

(67) 4-[(3-Chlor-4-fluor-phenyl)amino]-6-[trans-4-(2-(1-pyrrolidinyl-carbonyl)ethyl)-cyclohexylamino]-pyrimido [5,4-d]pyrimidin
Hergestellt aus den Verbindungen des Beispiels XXXII und X, Verseifung mit Natronlauge und Methanol und anschließende Umsetzung mit O-(Benzotriazol-1-yl)-N,N,N',N'-tetramethyluroniumtetrafluoroborat, Triethylamin und Pyrrolidin.
Schmelzpunkt: 187-192°C
$R_f$-Wert: 0,49 (Kieselgel; Petrolether/Essigester/Methanol = 10:8:3)

| | | | | |
|---|---|---|---|---|
| Ber. | C 60,30 | H 5,87 | N 19,69 | Cl 7,12 |
| Gef. | 60,39 | 5,89 | 19,50 | 7,39 |

(68) 4-[(3-Chlor-4-cyano-phenyl)amino]-6-[trans-4-hydroxycyclohexylamino]-pyrimido[5,4-d]pyrimidin
Schmelzpunkt: 244-247°C
$R_f$-Wert: 0,30 (Aluminiumoxid; Petrolether/Essigester/Methanol = 10:10:2)
Massenspektrum: $M^+$ = 395/397 (Cl)

(69) 4-[(4-Chlor-3-cyano-phenyl)amino]-6-[trans-4-hydroxycyclohexylamino)-pyrimido[5,4-d]pyrimidin
Schmelzpunkt: 250-252°C.
$R_f$-Wert: 0,57 (Kieselgel; Petrolether/Essigester/Methanol = 10:10:2)
Massenspektrum: $M^+$ = 395/397 (Cl)

(70) 4-[(3-Chlor-4-fluor-phenyl)amino]-6-[4-methylamino-cyclohexylamino]-pyrimido[5,4-d]pyrimidin
1:1-cis/trans-Mischung
Hergestellt aus den Verbindungen des Beispiels XXXII durch Umsetzung mit 4-Aminocyclohexanol, Oxidation mit Dess-Martin-Reagenz und anschließende reduktive Aminierung mit Methylamin und Natriumcyanoborhydrid.
Schmelzpunkt: 125-165°C
$R_f$-Wert: 0,40 (Aluminiumoxid; Petrolether/Essigester/Methanol/konz. Ammoniak = 10:10:2:0,05)
Massenspektrum: $M^+$ = 401/403 (Cl)

(71) 4-[(3-Chlor-4-fluor-phenyl)amino]-6-(cis-4-amino-3-methyl-1-piperidinyl)-pyrimido[5,4-d]pyrimidin
Hergestellt aus den Verbindungen der Beispiele XXXII und XXIX. Schmelzpunkt: 161-164°C
$R_f$-Wert: 0,25 (Kieselgel; Methylenchlorid/Methanol/konz. Ammoniak = 50:1:1)

(72) 4-[(3-Chlor-4-fluor-phenyl)amino]-6-(trans-4-amino-3-methyl-1-piperidinyl)-pyrimido[5,4-d]pyrimidin
Hergestellt aus den Verbindungen der Beispiele XXXII und XXIX (1).
Schmelzpunkt: 158°C
$R_f$-Wert: 0,20 (Kieselgel; Methylenchlorid/Methanol/konz. Ammoniak = 50:1:0,5)

(73) 4-[(3-Chlor-4-fluor-phenyl)amino]-6-[2-(4-acetyl-1-piperazinyl)-ethylamino]-pyrimido[5,4-d]pyrimidin
Hergestellt aus 4-[(3-Chlor-4-fluor-phenyl)amino]-6-[2-(1-piperazinyl)-ethylamino]-pyrimido[5,4-d]pyrimidin durch Umsetzung mit Acetanhydrid.
Schmelzpunkt: 196-198°C
$R_f$-Wert: 0,59 (Aluminiumoxid; Methylenchlorid/Methanol = 10:0,4)

(74) 4-[(3-Chlor-4-fluor-phenyl)amino]-6-(4-tetrahydropyranyloxy)-pyrimido[5,4-d]pyrimidin
Hergestellt aus den Verbindungen des Beispiels XXXII unter Verwendung von 4-Hydroxytetrahydropyran und metallischem Natrium. Schmelzpunkt: 220-222°C
$R_f$-Wert: 0,50 (Kieselgel; Petrolether/Essigester/Methanol = 10:10:1)

(75) 4-[(3-Chlor-4-fluor-ghenyl)amino]-6-(3-tetrahydrofuranyloxy)-pyrimido[5,4-d]pyrimidin
Hergestellt aus den Verbindungen des Beispiels XXXII unter Verwendung von 3-Hydroxytetrahydrofuran und metallischem Natrium. Schmelzpunkt: 163-165°C
$R_f$-Wert: 0,40 (Aluminiumoxid; Petrolether/Essigester/Methanol = 10:10:1)

(76) 4-[(3-Chlor-4-fluor-phenyl)amino]-6-[1-(hydroxymethylcyclopentylamino]-pyrimido[5,4-d]pyrimidin
Schmelzpunkt: 201-203°C
$R_f$-Wert: 0,39 (Kieselgel; Petrolether/Essigester/Methanol = 10:10:1)

(77) 4-[(3-Chlor-4-fluor-phenyl)amino]-6-[(4-aminocyclohexyl)-amino]-pyrimido[5,4-d]pyrimidin
Schmelzpunkt: 170-172°C
$R_f$-Wert: 0,35 (Aluminiumoxid; Petrolether/Essigester/Methanol = 10:10:2)

(78) 4-[(3-Chlor-4-fluor-phenyl)amino)-6-isopropylamino-pyrimido[5,4-d]pyrimidin
Schmelzpunkt: 230-234°C
$R_f$-Wert: 0,54 (Kieselgel; Petrolether/Essigester/Methanol = 10:5:1)

| | | | |
|---|---|---|---|
| Ber. | C 54,14 | H 4,24 | N 25,25 |
| Gef. | 54,32 | 4,29 | 25,14 |

(79) 4-[(3-Methylphenyl)amino]-6-[(1-(N,N-dimethylaminocarbonyl)-4-piperidinyl)amino]-pyrimido[5,4-d]pyrimidin
Hergestellt aus den Verbindungen der Beispiele XXXII und XII, nachfolgende Umsetzung mit Trifluoressigsäure und nachfolgende Umsetzung mit N,N-Dimethylcarbamoylchlorid.
Schmelzpunkt: 185-187°C
$R_f$-Wert: 0,42 (Kieselgel; Petrolether/Essigester/Methanol = 10:8:2)

(80) 4-[(3-Methylphenyl)amino]-6-[(1-formyl-4-piperidinyl)-amino]-pyrimido[5,4-d]pyrimidin
Hergestellt aus den Verbindungen der Beispiele XXXII und XII, nachfolgende Umsetzung mit Trifluoressigsäure und nachfolgende Umsetzung mit Ameisensäuremethylester.
Schmelzpunkt: 193-197°C
$R_f$-Wert: 0,53 (Kieselgel; Methylenchlorid/Methanol = 10:1)

| | | | |
|---|---|---|---|
| Ber. | C 62,79 | H 5,82 | N 26,98 |
| Gef. | 62,65 | 6,04 | 26,21 |

(81) 4-[(3-Chlor-4-fluor-phenyl)amino]-6-(4-piperidinyl-amino)-pyrimido[5,4-d]pyrimidin
Hergestellt aus den Verbindungen der Beispiele XXXII und XII und nachfolgende Umsetzung mit Trifluoressigsäure.
Schmelzpunkt: 239-243°C
$R_f$-Wert: 0,66 (Aluminiumoxid; Methylenchlorid/Methanol = 10:1)

(82) 4-[(3-Chlor-4-fluor-phenyl)amino]-6-[trans-4-carboxycyclohexylamino]-pyrimido[5,4-d]pyrimidin
Hergestellt aus den Verbindungen des Beispiels XXXII und trans-4-Aminocyclohexancarbonsäuremethylester und nachfolgende Verseifung mit Natronlauge.
Schmelzpunkt: >290°C

| | | | | |
|---|---|---|---|---|
| Ber. | C 54,75 | H 4,35 | N 20,16 | Cl 8,51 |
| Gef. | 54,49 | 4,69 | 19,56 | 8,48 |

(83) 4-[(3-Chlor-4-fluor-phenyl)amino]-6-[trans-4-(morpholinocarbonyl)cyclohexylamino]-pyrimido[5,4-d]pyrimidin
Hergestellt aus 4-[(3-Chlor-4-fluor-phenyl)amino]-6-[trans-4-carboxy-cyclohexylamino]-pyrimido[5,4-d]pyrimidin durch Umsetzung mit O-(Benzotriazol-1-yl)-N,N,N',N'-tetramethyluronium-tetrafluoroborat, Triethylamin und Morpholin. Schmelzpunkt: 221-225°C
$R_f$-Wert: 0,47 (Kieselgel; Petrolether/Essigester/Methanol = 10:8:3)

(84) 4-[(3-Chlor-4-fluor-phenyl)amino]-6-[trans-4-(pyrrolidinocarbonyl)cyclohexylamino]-pyrimido[5,4-d]pyrimidin
Hergestellt aus 4-[(3-Chlor-4-fluor-phenyl)amino]-6-[trans-4-carboxy-cyclohexylamino]-pyrimido[5,4-d]pyrimidin durch
Umsetzung mit O-(Benzotriazol-1-yl)-N,N,N',N'-tetramethyluronium-tetrafluoroborat, Triethylamin und Pyrrolidin.
Schmelzpunkt: 206-209°C
$R_f$-Wert: 0,52 (Kieselgel; Petrolether/Essigester/Methanol = 10:8:3)

(85) 4-[(3-Chlor-4-fluor-phenyl)amino]-6-[3-(morpholino)-1-propylamino]-pyrimido[5,4-d]pyrimidin
Schmelzpunkt: 157-159°C
$R_f$-Wert: 0,48 (Kieselgel; Petrolether/Essigester/Methanol = 10:10:3)

| | | | |
|---|---|---|---|
| Ber. | C 54,61 | H 5,07 | N 23,46 |
| Gef. | 54,40 | 5,25 | 23,30 |

(86) 4-[(3-Chlor-4-fluor-phenyl)amino]-6-[3-amino-1-pyrrolidinyl]-pyrimido[5,4-d]pyrimidin
Schmelzpunkt: 174-176°C
$R_f$-Wert: 0,54 (Aluminiumoxid; Methylenchlorid/Methanol = 15:1)

(87) 4-[(3-Chlor-4-fluor-phenyl)amino]-6-[4-(N-acetyl-N-methylamino)cyclohexylamino]-pyrimido[5,4-d]pyrimidin
Hergestellt aus den Verbindungen der Beispiele XXXII und XXII.
Schmelzpunkt: 195-197°C
$R_f$-Wert: 0,35 (Kieselgel; Petrolether/Essigester/Methanol = 10:10:2)

(88) 4-[(3-Chlor-4-fluor-phenyl)amino)-6-[4-tetrahydropyranylamino]-pyrimido[5,4-d]pyrimidin
Hergestellt aus den Verbindungen der Beispiele XXXII und XXIV.
Schmelzpunkt: 245-248°C
$R_f$-Wert: 0,47 (Kieselgel; Petrolether/Essigester = 3:10)

(89) 4-[(3-Methylphenyl)amino]-6-[(1-cyano-4-piperidinyl)-amino]-pyrimido[5,4-d]pyrimidin
Hergestellt aus den Verbindungen der Beispiele XXXII und XII, nachfolgende Umsetzung mit Trifluoressigsäure und nachfolgende Umsetzung mit Bromcyan.
Schmelzpunkt: 178-181°C
$R_f$-Wert: 0,61 (Kieselgel; Petrolether/Essigester/Methanol = 10:10:3)

(90) 4-[(3-Chlor-4-fluor-phenyl)amino]-6-(1-methyl-4-piperidinyl-amino)-pyrimido[5,4-d]pyrimidin
Schmelzpunkt: 175-177°C
$R_f$-Wert: 0,65 (Kieselgel; Petrolether/Essigester/Methanol = 10:10:2)

(91) 4-[(3-Chlor-4-fluor-phenyl)amino]-6-[(4-dimethylaminocyclohexyl)amino]-pyrimido[5,4-d]pyrimidin
Hergestellt aus den Verbindungen des Beispiels XXXII und 1,4-Diaminocyclohexan und anschließende reduktive Aminierung mit Formaldehyd und Natriumcyanoborhydrid.
Schmelzpunkt: 178-180°C (sintert ab 165°C)
$R_f$-Wert: 0,20 (Aluminiumoxid; Petrolether/Essigester/Methanol = 10:10:0,5)

(92) 4-[(3-Chlor-4-fluor-phenyl)amino]-6-(tert.butylamino)-pyrimido[5,4-d]pyrimidin
Schmelzpunkt: 265-267°C
$R_f$-Wert: 0,73 (Kieselgel; Petrolether/Essigester/Methanol = 10:10:1)

(93) 4-[(3-Chlor-4-fluor-phenyl)amino]-6-[3-amino-1-piperidinyl]-pyrimido[5,4-d]pyrimidin
Schmelzpunkt: 172-175°C
$R_f$-Wert: 0,33 (Kieselgel; Methylenchlorid/Methanol = 10:2)

(94) 4-[(3-Chlor-4-fluor-phenyl)amino]-6-[2-(1-piperazinyl)-ethylamino]-pyrimido[5,4-d]pyrimidin
Hergestellt aus den Verbindungen des Beispiels XXXII und 1-(2-Aminoethyl)piperazin.
Schmelzpunkt: 173-175°C
$R_f$-Wert: 0,53 (Kieselgel; Methylenchlorid/Methanol = 10:1)

(95) 4-[(4-Amino-5-brom-3-chlor-phenyl)amino]-6-[(trans-4-hydroxy-cyclohexyl)amino]-pyrimido[5,4-d]pyrimidin
Schmelzpunkt: 238-240°C
$R_f$-Wert: 0,50 (Aluminiumoxid; Petrolether/Essigester/Methanol = 10:10:2)

(96) 4-[(3-Chlor-4-fluor-phenyl)amino]-6-[(4-oxo-cyclohexyl)-amino]-pyrimido[5,4-d]pyrimidin
Hergestellt aus den Verbindungen des Beispiels XXXII durch Umsetzung mit 4-Aminocyclohexanol und anschließende Oxidation mit Dess-Martin-Reagenz.
Schmelzpunkt: 232-234°C
$R_f$-Wert: 0,48 (Kieselgel; Petrolether/Essigester/Methanol = 10:10:2)

(97) 4-[(3-Chlor-4-fluor-phenyl)amino]-6-[(cis-4-hydroxy-cyclohexyl)amino]-pyrimido[5,4-d]pyrimidin
Schmelzpunkt: 270-275°C
$R_f$-Wert: 0,47 (Aluminiumoxid; Methylenchlorid/Essigester/Methanol = 10:4:3)

(98) 4-[(3-Chlor-4-fluor-phenyl)amino]-6-[4-morpholino-cyclohexylamino]-pyrimido[5,4-d]pyrimidin
Hergestellt aus den Verbindungen des Beispiels XXXII durch Umsetzung mit 4-Aminocyclohexanol, Oxidation mit Dess-Martin-Reagenz und anschließende reduktive Aminierung mit Morpholin und Natriumcyanoborhydrid.
Schmelzpunkt: 233-235°C
$R_f$-Wert: 0,24 (Kieselgel; Petrolether/Essigester/Methanol = 10:10:2)

(99) 4-[(3-Chlor-4-fluor-phenyl)amino]-6-[4-(2-aminoethyl)-1-piperazinyl]-pyrimido[5,4-d]pyrimidin
Hergestellt aus den Verbindungen der Beispiele XXXII und XXV und anschließende Umsetzung mit 1N Kaliumhydrogensulfat-Lösung. Schmelzpunkt: 175-180°C
$R_f$-Wert: 0,35 (Aluminiumoxid; Methylenchlorid/Methanol = 10:1,5)

(100) 4-[(3-Chlor-4-fluor-phenyl)amino]-6-[4-(1-pyrrolidinyl)-cyclohexylamino]-pyrimido[5,4-d]pyrimidin
Hergestellt aus den Verbindungen des Beispiels XXXII durch Umsetzung mit 4-Aminocyclohexanol, Oxidation mit Dess-Martin-Reagenz und anschließende reduktive Aminierung mit Pyrrolidin und Natriumcyanoborhydrid.
Schmelzpunkt: 192-195°C
$R_f$-Wert: 0,38 (Aluminiumoxid; Petrolether/Essigester/Methanol = 10:10:1)

(101) 4-[(3-Methylphenyl)amino]-6-[2,6-dimethylmorpholino]-pyrimido[5,4-d]pyrimidin
1:1-cis-trans-Isomerengemisch
Schmelzpunkt: 123-129°C
$R_f$-Wert: 0,55 (Kieselgel; Petrolether/Essigester/Methanol = 10:8:2)

| | | | |
|---|---|---|---|
| Ber. | C 65,12 | H 6,33 | N 23,98 |
| Gef. | 64,96 | 6,20 | 23,95 |

(102) 4-(3-Chlor-4-fluor-phenyl)amino]-6-[trans-4-(2-(morpholinocarbonyl)ethyl)-cyclohexylamino]-pyrimido[5,4-d]pyrimidin Hergestellt aus den Verbindungen des Beispiels XXXII und X, Verseifung mit Natronlauge und Methanol und anschließende Umsetzung mit O-(Benzotriazol-1-yl)-N,N,N,N'-tetramethyluroniumtetrafluoroborat, Triethylamin und Morpholin.
Schmelzpunkt: 186-191°C
$R_f$-Wert: 0,48 (Kieselgel; Petrolether/Essigester/Methanol = 10:8:3)

| | | | | |
|---|---|---|---|---|
| Ber. | C 58,42 | H 5,69 | N 19,08 | Cl 6,90 |
| Gef. | 58,47 | 5,68 | 18,77 | 7,09 |

(103) 4-[(3-Chlor-4-fluor-phenyl)amino]-6-[3-piperidinyl-amino]-pyrimido[5,4-d]pyrimidin
Schmelzpunkt: 194-199°C
$R_f$-Wert: 0,62 (Aluminiumoxid; Methylenchlorid/Methanol = 10:1)

(104) 4-[(3-Chlor-4-fluor-phenyl)amino]-6-[4-acetylamino-1-piperidinyl]-pyrimido[5,4-d]pyrimidin
Hergestellt aus den Verbindungen des Beispiels XXXII durch Umsetzung mit 4-Amino-piperidin und nachfolgende Umsetzung mit Acetanhydrid.
Schmelzpunkt: 263-265°C
$R_f$-Wert: 0,63 (Aluminiumoxid; Methylenchlorid/Methanol = 20:1)

(105) 4-[(3-Chlor-4-fluor-phenyl)amino]-6-[4-(dimethylaminocarbonyl)-phenylamino]-pyrimido[5,4-d]pyrimidin

(106) 4-[(3-Chlor-4-fluor-phenyl)amino]-6-[4-(acetylaminophenylaminol-pyrimido[5,4-d]pyrimidin

(107) 4-[(3-Chlor-4-fluor-phenyl)amino)-6-[4-(dimethylaminocarbonylamino)-phenylamino]-pyrimido[5,4-d]pyrimidin

(108) 4-[(3-Chlor-4-fluor-phenyl)amino]-6-[4-(ethylaminocarbonylamino)-phenylamino]-pyrimido[5,4-d]pyrimidin

(109) 4-[(3-Chlor-4-fluor-phenyl)amino]-6-[4-(N-dimethylaminocarbonyl)-N-methyl-amino)-phenylamino]-pyrimido[-5,4-d]pyrimidin

(110) 4-[(3-Chlor-4-fluor-phenyl)amino]-6-[4-(3-oxo-1-piperazinyl)-cyclohexylamino]-pyrimido[5,4-d]pyrimidin

(111) 4-[(3-Chlor-4-fluor-phenyl)aminol-6-[4-(4-methyl-3-oxo-1-piperazinyl)-cyclohexylamino]-pyrimido[5,4-d]pyrimidin

(112) 4-[(3-Chlor-4-fluor-phenyl)amino]-6-[4-(methoxycarbonylamino)cyclohexylamino]-pyrimido[5,4-d]pyrimidin (cis/trans-Gemisch)
Hergestellt aus den Verbindungen des Beispiels XXXII durch Umsetzung mit cis/trans-1,4-Diamino-cyclohexan und nachfolgender Umsetzung mit Chlorameisensäuremethylester.
Schmelzpunkt: 230-235°C
$R_f$-Wert: 0,53 (Aluminiumoxid; Petrolether/Essigester/Methanol = 10:10:2)

(113) 4-[(3-Chlor-4-fluor-phenyl)amino)-6-[4-(1-piperidinyl)-cyclohexylamino]-pyrimido[5,4-d]pyrimidin

(114) 4-[(3-Chlor-4-fluor-phenyl)amino]-6-[4-(1-piperazinyl)-cyclohexylamino]-pyrimido[5,4-d]pyrimidin

(115) 4-(3-Chlor-4-fluor-phenyl)amino]-6-[4-(4-dimethylamino-1-piperidinyl)-cyclohexylamino]-pyrimido[5,4-d]pyrimidin Hergestellt aus den Verbindungen des Beispiels XXXII durch Umsetzung mit 4-Aminocyclohexanol, Oxidation mit Dess-Martin-Reagenz und anschließender reduktiver Aminierung mit 4-Dimethylamino-piperidin und Natriumcyanoborhydrid.
Schmelzpunkt: 175°C (Zers.)
$R_f$-Wert: 0,44 (Aluminiumoxid; Methylenchlorid/Essigester/Methanol = 10:5:1)

(116) 4-[(3-Chlor-4-fluor-phenyl)amino]-6-[4-ethylamino-cyclohexylamino]-pyrimido[5,4-d]pyrimidin

(117) 4-[(3-Chlor-4-fluor-phenyl)amino]-6-[4-(4-amino-1-piperidinyl)-cyclohexylamino]-pyrimido[5,4-d]pyrimidin

(118) 4-[(3-Chlor-4-fluor-phenyl)amino]-6-[4-cyano-cyclohexylamino]-pyrimido[5,4-d]pyrimidin

(119) 4-[(3-Chlor-4-fluor-phenyl)amino]-6-[4-(4-acetyl-1-piperazinyl)-cyclohexylamino]-pyrimido[5,4-d]pyrimidin

(120) 4- [(3-Chlor-4-fluor-phenyl)amino]-6-[4-(dimethylaminocarbonylamino)-cyclohexylamino]-pyrimido[5,4-d]pyrimidin

(121) 4-[(3-Chlor-4-fluor-phenyl)amino]-6-[4-(ethylaminocarbonylamino)-cyclohexylamino]-pyrimido[5,4-d]pyrimidin

(122) 4-[(3-Chlor-4-fluor-phenyl)amino)-6-[4-(benzoylamino)-cyclohexylamino]-pyrimido[5,4-d]pyrimidin

(123) 4-[(3-Chlor-4-fluor-phenyl)amino)-6-[4-(phenylsulfonylamino)-cyclohexylamino]-pyrimido[5,4-d]pyrimidin

(124) 4-[(3-Chlor-4-fluor-phenyl)amino]-6-[4-(phenylacetylamino)-cyclohexylamino]-pyrimido[5,4-d]pyrimidin

(125) 4-[(3-Chlor-4-fluor-phenyl)amino]-6-[4-(2-phenylpropionylamino)-cyclohexylamino]-pyrimido[5,4-d]pyrimidin

(126) 4-[(3-Chlor-4-fluor-phenyl)aminol-6-[4-(4-methyl-1-piperazinyl)carbonyl-cyclohexylamino]-pyrimido[5,4-d] pyrimidin Hergestellt aus 4-[(3-Chlor-4-fluor-phenyl)amino]-6-[trans-4-carboxy-cyclohexylamino]-pyrimido[5,4-d] pyrimidin durch Umsetzung mit O-(Benzotriazol-1-yl)-N,N,N',N'-tetramethyluronium-tetrafluoroborat, Triethylamin und 1-Methylpiperazin. Schmelzpunkt: 194-197°C
$R_f$-Wert: 0,45 (Aluminiumoxid; Methylenchlorid/Methanol = 10:0,3)

(127) 4-[(3-Chlor-4-fluor-phenyl)amino]-,6-[4-(1-piperazinyl)-carbonyl-cyclohexylamino]-pyrimido[5,4-d]pyrimidin Hergestellt aus 4-[(3-Chlor-4-fluor-phenyl)amino]-6-[trans-4-carboxy-cyclohexylamino]-pyrimido [5,4-d]pyrimidin durch Umsetzung mit O-(Benzotriazol-1-yl)-N,N,N',N'-tetramethyluronium-tetrafluoroborat, Triethylamin und Piperazin. Schmelzpunkt: 178-182°C
$R_f$-Wert: 0,47 (Aluminiumoxid; Methylenchlorid/Methanol = 10:0,3)

(128) 4-[(3-Chlor-4-fluor-phenyl)amino]-6-[trans-4-(cis/trans-2,6-dimethyl-morpholino)carbonyl-cyclohexylamino]-pyrimido-[5,4-d]pyrimidin
Hergestellt aus 4-[(3-Chlor-4-fluor-phenyl)amino]-6-[trans-4-carboxy-cyclohexylamino]-pyrimido[5,4-d]pyrimidin durch Umsetzung mit O-(Benzotriazol-1-yl)-N,N,N,N'-tetramethyluronium-tetrafluoroborat, Triethylamin und cis/trans-2,6-Dimethyl-morpholin.
Schmelzpunkt: 198-201°C
$R_f$-Wert: 0,40 und 0,33 (Kieselgel; Petrolether/Essigester/Methanol = 10:10:2)

(129) 4-[(3-Chlor-4-fluor-phenyl)amino]-6-[4-(thiomorpholino-carbonyl)-cyclohexylamino]-pyrimido[5,4-d]pyrimidin

(130) 4-[(3-Chlor-4-fluor-phenyl)amino]-6-[4-(S-oxido-thiomorpholino-carbonyl)-cyclohexylamino]-pyrimido[5,4-d] pyrimidin

(131) 4-[(3-Chlor-4-fluor-phenyl)amino]-6-[4-(S,S-dioxido-thiomorpholino-carbonyl)-cyclohexylamino]-pyrimido [5,4-d]pyrimidin

(132) 4-[(3-Chlor-4-fluor-phenyl)amino]-6-[4-(4-acetyl-1-piperazinyl)carbonyl-cyclohexylamino]-pyrimido[5,4-d] pyrimidin

(133) 4-[(3-Chlor-4-fluor-phenyl)amino]-F-[4-(1-piperidinylmethyl)-cyclohexylamino]-pyrimido[5,4-d]pyrimidin

(134) 4-[(3-Chlor-4-fluor-phenyl)amino]-6-[4-(1-pyrrolidinylmethyl)-cyclohexylamino]-pyrimido[5,4-d]pyrimidin

(135) 4-[(3-Chlor-4-fluor-phenyl)amino]-6-[4-(morpholino-methyl)-cyclohexylamino]-pyrimido[5,4-d]pyrimidin

(136) 4-[(3-Chlor-4-fluor-phenyl)amino]-6-[4-(1-piperazinylmethyl)-cyclohexylamino]-pyrimido[5,4-d]pyrimidin

(137) 4-[(3-Chlor-4-fluor-phenyl)amino]-6-[4-(4-methyl-2-piperazinyl-methyl)-cyclohexylamino]-pyrimido[5,4-d] pyrimidin

(138) 4-[(3-Chlor-4-fluor-phenyl)amino]-6-[4-(methylaminomethyl)-cyclohexylamino]-pyrimido[5,4-d]pyrimidin

(139) 4-[(3-Chlor-4-fluor-phenyl)amino]-6-[4-(ethylaminomethyl)-cyclohexylamino]-pyrimido[5,4-d]pyrimidin

(140) 4-[(3-Chlor-4-fluor-phenyl)amino]-6-[4-(dimethylaminomethyl)-cyclohexylamino]-pyrimido[5,4-d]pyrimidin

(141) 4-[(3-Chlor-4-fluor-phenyl)amino]-6-[1-(2-aminoethyl)-4-piperidinyl-amino]-pyrimido[5,4-d]pyrimidin

(142) 4-[(3-Chlor-4-fluor-phenyl)amino]-6-[1-(morpholinocarbonyl)-4-piperidinyl-amino]-pyrimido[5,4-d]pyrimidin

(143) 4-[(3-Chlor-4-fluor-phenyl)amino]-6-[1-ethyl-3-piperidinyl-amino]-pyrimido[5,4-d]pyrimidin

(144) 4-[(3-Chlor-4-fluor-phenyl)amino]-6-[3-amino-cyclohexylamino]-pyrimido[5,4-]pyrimidin

(145) 4-[(3-Chlor-4-fluor-phenyl)amino]-6-[3-dimethylamino-cyclohexylamino]-pyrimido[5,4-d]pyrimidin

(146) 4-[(3-Chlor-4-fluor-phenyl)amino]-6-[4-(2-aminoethyl)-cyclohexylamino]-pyrimido[5,4-d]pyrimidin

(147) 4-[(3-Chlor-4-fluor-phenyl)amino7-6-[4-S-oxido-tetrahydrothiopyranyl-amino]-pyrimido[5,4-d] pyrimidin

(148) 4-[(3-Chlor-4-fluor-phenyl)amino]-6-[4-S,S-dioxido-tetrahydrothiopyranyl-amino)-pyrimido[5,4-d]pyrimidin

(149) 4-[(3-Chlor-4-fluor-phenyl)amino]-6-[4-(1-piperidinyl)-1-piperidinyl]-pyrimido[5,4-d]pyrimidin

(150) 4-[(3-Chlor-4-fluor-phenyl)amino]-6-[4-(1-piperazinyl)-1-piperidinyl]-pyrimido[5,4-d]pyrimidin
Hergestellt aus den Verbindungen des Beispiels XXXII durch Umsetzung mit 4-Hydroxypiperidin, Oxidation mit Dess-Martin-Reagenz, anschließender reduktiver Aminierung mit 1-tert.Butoxycarbonyl-piperazin und Natriumcyanoborhydrid und anschließender Umsetzung mit Trifluoressigsäure.
Schmelzpunkt: 178-180°C
$R_f$-Wert: 0,35 (Aluminiumoxid; Petrolether/Essigester/Methanol = 10:5:3)

(151) 4-[(3-Chlor-4-fluor-phenyl)amino]-6-[4-(4-methyl-1-piperazinyl)-1-piperidinyl]-pyrimido[5,4-d]pyrimidin
Hergestellt aus den Verbindungen des Beispiels XXXII durch Umsetzung mit 4-Hydroxypiperidin, Oxidation mit Dess-Martin-Reagenz und anschließender reduktiver Aminierung mit 1-Methylpiperazin und Natriumcyanoborhydrid.
Schmelzpunkt: 163-165°C
$R_f$-Wert: 0,52 (Aluminiumoxid; Petrolether/Essigester/Methanol = 10:10:1)

(152) 4-[(3-Chlor-4-fluor-phenyl)amino]-6-[4-(4-dimethylamino-1-piperidinyl)-1-piperidinyl]-pyrimido[5,4-d]pyrimidin Hergestellt aus den Verbindungen des Beispiels XXXII durch Umsetzung mit 4-Hydroxypiperidin, Oxidation mit Dess-Martin-Reagenz und anschließender reduktiver Aminierung mit 4-Dimethylamino-piperidin und Natriumcyanoborhydrid.
Schmelzpunkt: 160-165°C
$R_f$-Wert: 0,30 (Aluminiumoxid; Petrolether/Essigester/Methanol 10:10:2)

(153) 4-[(3-Chlor-4-fluor-phenyl)amino]-6-[4-methylamino-1-piperidinyl]-pyrimido[5,4-d]pyrimidin
Hergestellt aus der Verbindung des Beispiels XXXII durch Umset zung mit 4-Hydroxypiperidin, Oxidation mit Dess-Martin-Reagenz und anschließender reduktiver Aminierung mit Methylamin und Na triumcyanoborhydrid.
Schmelzpunkt: 174-176°C
$R_f$-Wert: 0,40 (Aluminiumoxid; Petrolether/Essigester/Methanol = 10:10:4)

(154) 4-[(3-Chlor-4-fluor-phenyl)amino]-6-[4-ethylamino-1-piperidinyl]-pyrimido[5,4-d]pyrimidin

(155) 4-[(3-Chlor-4-fluor-phenyl)amino]-6-[4-(morpholinocarbonylamino)-1-piperidinyl]-pyrimido[5,4-d]pyrimidin

(156) 4-[(3-Chlor-4-fluor-phenyl)amino]-6-[4-(4-amino-1-piperidinyl)-1-piperidinyl]-pyrimido[5,4-d]pyrimidin

(157) 4-[(3-Chlor-4-fluor-phenyl)amino]-6-[4-methoxy-1-piperidinyl]-pyrimido[5,4-d]pyrimidin

(158) 4-[(3-Chlor-4-fluor-phenyl)amino]-6-[4-(2-oxo-1-pyrrolidinyl)-1-piperidinyl]-pyrimido[5,4-d]pyrimidin

(159) 4-[(3-Chlor-4-fluor-phenyl)amino]-6-[4-(4-hydroxy-1-piperidinyl)-1-piperidinyl]-pyrimido[5,4-d]pyrimidin

(160) 4-[(3-Chlor-4-fluor-phenyl)amino]-6-[4-(4-methylamino-1-piperidinyl)-1-piperidinyl]-pyrimido[5,4-d]pyrimidin

(161) 4-[(3-Chlor-4-fluor-phenyl)amino]-6-[4-(3-oxo-1-piperazinyl)-1-piperidinyl]-pyrimido[5,4-d]pyrimidin

(162) 4-[(3-Chlor-4-fluor-phenyl)amino]-6-[4-(4-methyl-3-oxo-1-piperazinyl)-1-piperidinyl]-pyrimido[5,4-d]pyrimidin

(163) 4-[(3-Chlor-4-fluor-phenyl)amino]-6-[4-(N-acetyl-N-methyl-amino)-1-piperidinyl]-pyrimido[5,4-d)pyrimidin

(164) 4-[(3-Chlor-4-fluor-phenyl)amino]-6-[4-(N-methyl-N-methylsulfonyl-amino)-1-piperidinyl]-pyrimido[5,4-d]pyrimidin Hergestellt aus der Verbindung 153 des Beispiels 1 durch Umsetzung mit Methansulfonsäurechlorid und Triethylamin. Schmelzpunkt: 225-227°C
$R_f$-Wert: 0,53 (Aluminiumoxid; Petrolether/Essigester/Methanol = 10:10:1)

| | | | |
|---|---|---|---|
| Ber. | C 48,97 | H 4,54 | N 21,04 |
| Gef. | 49,07 | 4,59 | 20,75 |

(165) 4-[(3-Chlor-4-fluor-phenyl)amino]-6-[4-(2-methoxycarbonyl-ethyl)-1-piperidinyl]-pyrimido[5,4-d]pyrimidin

(166) 4-[(3-Chlor-4-fluor-phenyl)amino]-6-[4-(2-carboxy-ethyl)-1-piperidinyl]-pyrimido[5,4-d]pyrimidin

(167) 4-[(3-Chlor-4-fluor-phenyl)amino]-6-[4-(2-(morpholinocarbonyl)-ethyl)-1-piperidinyl]-pyrimido[5,4-d]pyrimidin

(168) 4-[(3-Chlor-4-fluor-phenyl)amino]-6-[4-(2-amino-ethyl)-1-piperidinyll-pyrimido[5,4-d]pyrimidin

(169) 4-[(3-Chlor-4-fluor-phenyl)amino]-6-[4-(2-aminocarbonylethyl)-1-piperidinyl]-pyrimido[5,4-d]pyrimidin

(170) 4-[(3-Chlor-4-fluor-phenyl)amino]-6-[4-(2-methylaminocarbonyl-ethyl)-1-piperidinyl]-pyrimido[5,4-d]pyrimidin

(171) 4-[(3-Chlor-4-fluor-phenyl)amino]-6-[4-(2-dimethylaminocarbonyl-ethyl)-1-piperidinyl)-pyrimido[5,4-d]pyrimidin

(172) 4-[(3-Chlor-4-fluor-phenyl)amino]-6-[4-(2-(pyrrolidinocarbonyl) -ethyl) -1-piperidinyl] -pyrimido[5,4-d)pyrimidin

(173) 4-[(3-Chlor-4-fluor-phenyl)amino]-6-[4-(carboxy-methyloxy) -1-piperidinyl] -pyrimido[5,4-d]pyrimidin

(174) 4-[(3-Chlor-4-fluor-phenyl)amino]-6-[4-(methoxycarbonylmethyloxy)-1-piperidinyl]-pyrimido[5,4-d]pyrimidin

(175) 4-[(3-Chlor-4-fluor-phenyl)amino]-6-[4-(aminocarbonylmethyloxy)-1-piperidinyl]-pyrimido[5,4-d]pyrimidin

(176) 4-[(3-Chlor-4-fluor-phenyl)amino]-6-[4-(methylaminocarbonyl-methyloxy) -1-piperidinyl] -pyrimido [5,4-d] pyrimidin

(177) 4-[(3-Chlor-4-fluor-phenyl)amino]-6-[4-(dimethylaminocarbonyl-methyloxy)-1-piperidinyl]-pyrimido[5,4-d] pyrimidin

(178) 4-[(3-Chlor-4-fluor-phenyl)amino]-6-[4-(morpholinocarbonyl-methyloxy)-1-piperidinyl]-pyrimido[5,4-d)pyrimidin

(179) 4-[(3-Chlor-4-fluor-phenyl)amino]-6-[4-((1-pyrrolidinyl)-carbonyl-methyloxy)-1-piperidinyl]-pyrimido[5,4-d] pyrimidin

(180) 4-[(3-Chlor-4-fluor-phenyl)amino]-6-[2-aminomethyl-1-piperidinyl]-pyrimido[5,4-d]pyrimidin

(181) 4-[(3-Chlor-4-fluor-phenyl)amino]-6-[2-(1-pyrrolidinyl)-methyl-1-piperidinyl]-pyrimido[5,4-d]pyrimidin

(182) 4-[(3-Chlor-4-fluor-phenyl)amino]-6-[2-dimethylaminomethyl-1-piperidinyl]-pyrimido[5,4-d]pyrimidin

(183) 4-[(3-Chlor-4-fluor-phenyl)amino]-6-[4-amino-4-methyl-1-piperidinyl]-pyrimido[5,4-d]pyrimidin
Hergestellt aus den Verbindungen der Beispiele XXXII und der Verbindung 2 des Beispiels XXXVI.
Schmelzpunkt: 188-190°C
$R_f$-Wert: 0,50 (Kieselgel; Methylenchlorid/Methanol/konz.Ammoniak = 9:1:0,1)

(184) 4-[(3-Chlor-4-fluor-phenyl)amino]-6-[endo-3-amino-8-azabicyclo[3.2.1]-8-octyl]-pyrimido[5,4-d]pyrimidin
Hergestellt aus den Verbindungen der Beispiele XXXII und XXXVI. Schmelzpunkt: 238-240°C
$R_f$-Wert: 0,34 (Kieselgel; Methylenchlorid/Methanol/konz.Amanoniak = 9:1:0,1)

(185) 4-[(3-Chlor-4-fluor-phenyl)amino]-6-[3-methylamino-8-azabicyclo[3.2.1]-8-octyl]-pyrimido[5,4-d]pyrimidin

(186) 4-[(3-Chlor-4-fluor-phenyl)amino]-6-[3-dimethylamino-8-azabicyclo[3.2.1]-8-octyl]-pyrimido[5,4-d]pyrimidin

(187) 4-[(3-Chlor-4-fluor-phenyl)amino]-6-[endo-3-acetylamino-8-aza-bicyclo[3.2.1]-8-octyl]-pyrimido[5,4-d]pyrimidin Hergestellt aus der Verbindung 184 des Beispiels 1 durch Umsetzung mit Acetanhydrid und Triethylamin.
Schmelzpunkt: 214-216°C (sintert ab 130°C, wird dann wieder fest)
$R_f$-Wert: 0,53 (Kieselgel; Methylenchlorid/Methanol/konz.Ammoniak = 9:1:0,1)

(188) 4-[(3-Chlor-4-fluor-phenyl)amino]-6-[3-acetylaminomethyl-1-piperidinyl]-pyrimido[5,4-d]pyrimidin

(189) 4-[(3-Chlor-4-fluor-phenyl)amino]-6-[3-methylsulfonylaminomethyl-1-piperidinyl]-pyrimido[5,4-d]pyrimidin

(190) 4-[(3-Chlor-4-fluor-phenyl)amino]-6-[4-ethylaminomethyl-1-piperidinyl]-pyrimido[5,4-d]pyrimidin

(191) 4-[(3-Chlor-4-fluor-phenyl)amino]-6-[4-methylaminomethyl-1-piperidinyl]-pyrimido[5,4-d]pyrimidin

(192) 4-[(3-Chlor-4-fluor-phenyl)amino]-6-[4-(1-piperidinyl)methyl-1-piperidinyl]-pyrimido[5,4-d]pyrimidin

(193) 4-[(3-Chlor-4-fluor-phenyl)amino]-6-[4-(1-piperazinyl)methyl-1-piperidinyl]-pyrimido[5,4-d]pyrimidin

(194) 4-[(3-Chlor-4-fluor-phenyl)amino]-6-[4-(4-methyl-1-piperidinyl)methyl-1-piperidinyl]-pyrimido[5,4-d]pyrimidin Hergestellt aus den Verbindungen der Beispiele XXXII und XXXIV, nachfolgender Oxidation mit Dess-Martin-Reagenz und anschließender reduktiver Aminierung mit 1-Methylpiperazin und Natriumcyanoborhydrid.
Schmelzpunkt: 138-140°C
$R_f$-Wert: 0,55 (Aluminiumoxid; Petrolether/Essigester/Methanol = 10:10:1)

(195) 4-[(3-Chlor-4-fluor-phenyl)amino]-6-[4-(morpholinomethyl)-1-piperidinyl]-pyrimido[5,4-d]pyrimidin

(196) 4-[(3-Chlor-4-fluor-phenyl)amino]-6-[4-(1-pyrrolidinyl)-methyl-1-piperidinyl]-pyrimido[5,4-d]pyrimidin

(197) 4-[(3-Chlor-4-fluor-phenyl)aminol-6-[4-dimethylaminomethyl-1-piperidinyl]-pyrimido[5,4-d]pyrimidin

(198) 4-[(3-Chlor-4-fluor-phenyl)amino]-6-[4-acetylaminomethyl-1-piperidinyl]-pyrimido[5,4-d]pyrimidin

(199) 4-[(3-Chlor-4-fluor-phenyl)amino]-6-[4-methylsulfonylaminomethyl-1-piperidinyl]-pyrimido[5,4-d]pyrimidin

(200) 4-[(3-Chlor-4-fluor-phenyl)amino]-6-[4-cyanomethyl-1-piperidinyl]-pyrimido[5,4-d]pyrimidin

(201) 4-[(3-Chlor-4-fluor-phenyl)amino]-6-[4-(1-piperazinyl)-carbonyl-1-piperidinyl]-pyrimido[5,4-d]pyrimidin

(202) 4-[(3-Chlor-4-fluor-phenyl)amino]-6-[4-(4-methyl-1-piperazinyl)carbonyl-1-piperidinyl]-pyrimido[5,4-d]pyrimidin

(203) 4-[(3-Chlor-4-fluor-phenyl)amino]-6-[4-(2-oxo-1-imidazolidinyl)-1-piperidinyl]-pyrimido[5,4-d]pyrimidin

(204) 4-[(3-Chlor-4-fluor-phenyl)amino]-6-[4-(3-methyl-2-oxo-1-imidazolidinyl)-1-piperidinyl]-pyrimido[5,4-d]pyrimidin

(205) 4-[(3-Chlor-4-fluor-phenyl)amino]-6-[2-(3-oxo-1-piperazinyl)-ethylamino]-pyrimido[5,4-d]pyrimidin

(206) 4-[(3-Chlor-4-fluor-phenyl)amino]-6-[2-(4-methyl-3-oxo-1-piperazinyl)-ethylamino)-pyrimido[5,4-d]pyrimidin

(207) 4-[(3-Chlor-4-fluor-phenyl)amino)-6-[2-cyano-ethylamino)-pyrimido[5,4-d]pyrimidin

(208) 4-[(3-Chlor-4-fluor-phenyl)amino]-6-[2-(1-methyl-2-piperidinyl)-ethylamino]-pyrimido[5,4-d]pyrimidin

(209) 4-[(3-Chlor-4-fluor-phenyl)amino]-6-[N-methyl-N-(2-(1-methyl-2-piperidinyl)-ethyl)amino]-pyrimido[5,4-d] pyrimidin

(210) 4-[(3-Chlor-4-fluor-phenyl)amino]-6-[2-(4-piperidinyl)-ethylamino]-pyrimido[5,4-d]pyrimidin

(211) 4-[(3-Chlor-4-fluor-phenyl)amino]-6-[2-(2-oxo-1-imidazolidinyl)-ethylamino]-pyrimido[5,4-d]pyrimidin

(212) 4-[(3-Chlor-4-fluor-phenyl)amino]-6-[2-(3-methyl-2-oxo-1-imidazolidinyl)-ethylamino]-pyrimido[5,4-d]pyrimidin

(213) 4-[(3-Chlor-4-fluor-phenyl)amino]-6-[2-(1-acetyl-4-piperidinyl)-ethylamino]-pyrimido{5,4-d]pyrimidin

(214) 4-[(3-Chlor-4-fluor-phenyl)amino]-6-[2-(1-methoxycarbonyl-4-piperidinyl)-ethylamino]-pyrimido[5,4-d]pyrimidin

(215) 4-[(3-Chlor-4-fluor-phenyl)amino]-6-[2-(4-amino-cyclohexyl)-ethylamino)-pyrimido[5,4-d]pyrimidin

(216) 4-[(3-Chlor-4-fluor-phenyl)amino]-6-[3-acetylamino-propylamino]-pyrimido[5,4-d]pyrimidin

(217) 4-[(3-Chlor-4-fluor-phenyl)amino]-6-[3-methylsulfonylamino-propylamino]-pyrimido[5,4-d]pyrimidin

(218) 4-[(3-Chlor-4-fluor-phenyl)amino]-6-[3-methoxycarbonylamino-propylamino]-pyrimido[5,4-d]pyrimidin

(219) 4-[(3-Chlor-4-fluor-phenyl)amino]-6-[3-(morpholinocarbonylamino)-propylamino]-pyrimido[5,4-d]pyrimidin

(220) 4-[(3-Chlor-4-fluor-phenyl)amino]-6-[1-piperazinyl-carbonyl-methylamino]-pyrimido[5,4-d] pyrimidin

(221) 4-[(3-Chlor-4-fluor-phenyl)amino]-6-[4-methyl-1-piperazinyl-carbonyl-methylamino]-pyrimido[5,4-d]pyrimidin

(222) 4-[(3-Chlor-4-fluor-phenyl)amino]-6-[4-acetyl-1-piperazinyl-carbonyl-rnethylamino]-pyrimido[5,4-d]pyrimidin

(223) 4-[(3-Chlor-4-fluor-phenyl)amino]-6-[1-carboxy-ethylamino]-pyrimido[5,4-d]pyrimidin

(224) 4-[(3-Chlor-4-fluor-phenyl)amino]-6-[1-methoxycarbonylethylamino]-pyrimido[5,4-d]pyrimidin

(225) 4-[(3-Chlor-4-fluor-phenyl)amino]-6-[1-aminocarbonylethylamino]-pyrimido[5,4-d]pyrimidin

(226) 4-[(3-Chlor-4-fluor-phenyl)amino]-6-[1-methylaminocarbonyl-ethylamino]-pyrimido[5,4-d]pyrimidin

(227) 4-[(3-Chlor-4-fluor-phenyl)amino]-6-[1-dimethylaminocarbonyl-ethylamino]-pyrimido[5,4-d]pyrimidin

(228) 4-[(3-Chlor-4-fluor-phenyl)amino]-6-[1-(1-pyrrolidinylcarbonyl)-ethylamino]-pyrimido[5,4-d]pyrimidin

(229) 4-[(3-Chlor-4-fluor-phenyl)amino]-6-[1-(morpholinocarbonyl)-ethylamino]-pyrimido[5,4-d]pyrimidin

(230) 4-[(3-Chlor-4-fluor-phenyl)amino]-6-[1-(1-piperazinylcarbonyl)-ethylamino]-pyrimido[5,4-d]pyrimidin

(231) 4-[(3-Chlor-4-fluor-phenyl)amino]-6-[1-(4-methyl-1-piperazinyl-carbonyl)-ethylamino]-pyrimido[5,4-d]pyrimidin

(232) 4-[(3-Chlor-4-fluor-phenyl)amino]-6-[1-(4-acetyl-1-piperazinyl-carbonyl)-ethylamino]-pyrimido[5,4-d]pyrimidin

(233) 4-[(3-Chlor-4-fluor-phenyl)amino]-6-[2-(1-piperazinylcarbonyl)-2-propylamino]-pyrimido[5,4-]pyrimidin

(234) 4-[(3-Chlor-4-fluor-phenyl)amino]-6-[2-(4-methyl-1-piperazinyl-carbonyl)-2-propylamino]-pyrimido[5,4-d]pyrimidin

(235) 4-[(3-Chlor-4-fluor-phenyl)amino]-6-[2-(4-acetyl-1-piperazinyl-carbonyl)-2-propylamino]-pyrimido[5,4-d]pyrimidin

(236) 4-[(3-Chlor-4-fluor-phenyl)amino]-6-[1-(morpholinocarbonyl)-3-pyrrolidinyl-amino]-pyrimido[5,4-d]pyrimidin

(237) 4-[(3-Chlor-4-fluor-phenyl)amino)-6-[3-amino-cyclopentylamino]-pyrimido[5,4-d]pyrimidin

(238) 4-[(3-Chlor-4-fluor-phenyl)amino]-6-[3-methoxycarbonylcyclopentylamino]-pyrimido[5,4-d]pyrimidin

(239) 4-[(3-Chlor-4-fluor-phenyl)amino]-6-[3-carboxy-cyclopentylamino]-pyrimido[5,4-d]pyrimidin

(240) 4-[(3-Chlor-4-fluor-phenyl)amino]-6-[3-(morpholinocarbonyl)-cyclopentylamino]-pyrimido[5,4-d]pyrimidin

(241) 4-[(3-Chlor-4-fluor-phenyl)amino]-6-[1-ethyl-2-pyrrolidinyl-methylamino]-pyrimido[5,4-d]pyrimidin

(242) 4-[(3-Chlor-4-fluor-phenyl)amino]-6-[1-ethyl-3-pyrrolidinyl-methylamino]-pyrimido[5,4-d]pyrimidin

(243) 4-[(3-Chlor-4-fluor-phenyl)amino]-6-[(3-aminomethylcyclopentyl)-methylamino]-pyrimido[5,4-d]pyrimidin

(244) 4-[(3-Chlor-4-fluor-phenyl)amino)-6-[3-tetrahydrofurylmethylamino7-pyrimido[5,4-d)pyrimidin

(245) 4-[(3-Chlor-4-fluor-phenyl)amino]-6-[3-methylamino-1-pyrrolidinyl]-pyrimido[5,4-d]pyrimidin

(246) 4-[(3-Chlor-4-fluor-phenyl)amino]-6-[3-ethylamino-1-pyrrolidinyl]-pyrimido[5,4-d]pyrimidin

(247) 4-[(3-Chlor-4-fluor-phenyl)amino)-6-[1-(morpholinocarbonyl)-4-piperidinyl-methylamino]-pyrimido[5,4-d]pyrimidin

(248) 4-[(3-Chlor-4-fluor-phenyl)amino]-6-[1-acetyl-4-piperidinyl-methylamino]-pyrimido[5,4-d]pyrimidin

(249) 4-[(3-Chlor-4-fluor-phenyl)amino]-6-[1-methyl-4-piperidinyl-methylamino]-pyrimido[5,4-d]pyrimidin

(250) 4-[(3-Chlor-4-fluor-phenyl)amino]-6-[3-piperidinyl-methylamino]-pyrimido[5,4-d]pyrimidin

(251) 4-[(3-Chlor-4-fluor-phenyl)amino]-6-[2-piperidinyl-methylamino]-pyrimido[5,4-d]pyrimidin

(252) 4-[(3-Chlor-4-fluor-phenyl)amino]-6-[1-amino-2-propylamino]-pyrimido[5,4-d]pyrimidin

(253) 4-[(3-Chlor-4-fluor-phenyl)amino]-6-[4-(morpholinocarbonyl)-1-piperazinyl]-pyrimido[5,4-d]pyrimidin

(254) 4-[(3-Chlor-4-fluor-phenyl)amino]-6-[N-methyl-N-(4-piperidinyl)amino]-pyrimido[5,4-d]pyrimidin

(255) 4-[(3-Cyano-phenyl)amino]-6-[N-methyl-N-(1-methyl-4-piperidinyl)amino]-pyrimido[5,4-d]pyrimidin
Schmelzpunkt: 199-204°C
$R_f$-Wert: 0,49 (Aluminiumoxid; Essigester/Petrolether/Methanol = 10:3:0,4)

(256) 4-[(3-Trifluormethoxy-phenyl)amino]-6-[N-methyl-N-(1-methyl-4-piperidinyl)amino]-pyrimido[5,4-d]pyrimidin

Schmelzpunkt: 118-120°C
$R_f$-Wert: 0,68 (Aluminiumoxid; Essigester/Petrolether/Methanol = 10:10:2)

(257) 4-[(3-Trifluormethyl-phenyl)amino]-6-[N-methyl-N-(1-methyl-4-piperidinyl)amino]-pyrimido[5,4-d]pyrimidin
Schmelzpunkt: 129-132°C
$R_f$-Wert: 0,50 (Aluminiumoxid; Essigester/Petrolether = 10:3)

(258) 4-[(3,4-Difluor-phenyl)amino]-6-[N-methyl-N-(1-methyl-4-piperidinyl)amino]-pyrimido[5,4-d]pyrimidin
Schmelzpunkt: 196-198°C
$R_f$-Wert: 0,59 (Aluminiumoxid; Essigester/Petrolether/Methanol = 10:5:0.4)

(259) 4-[(3-Nitro-phenyl)amino]-6-[N-methyl-N-(1-methyl-4-piperidinyl)amino]-pyrimido[5,4-d]pyrimidin
Schmelzpunkt: 199-203°C
$R_f$-Wert: 0,36 (Kieselgel; Methylenchlorid/Methanol = 10:1)

(260) 4-[(3-Bthinyl-phenyl)amino]-6-[N-methyl-N-(1-methyl-4-piperidinyl)amino]-pyrimido[5,4-d)pyrimidin
Schmelzpunkt: 162-165°C
$R_f$-Wert: 0,64 (Kieselgel; Petrolether/Essigester/Methanol = 10:5:1)

(261) 4-[(4-Amino-3-nitro-phenyl}amino]-6-[N-methyl-N-(1-methyl-4-piperidinyl)amino]-pyrimido[5,4-d]pyrimidin
Schmelzpunkt: 242-246°C (sintert ab 220°C)
$R_f$-Wert: 0,63 (Kieselgel; Methylenchlorid/Methanol/konz. Ammoniaklösung = 10:2:0.05)

(262) 4-[(4-Chlor-3-nitro-phenyl)amino]-6-[N-methyl-N-(1-methyl-4-piperidinyl)amino]-pyrimido[5,4-d]pyrimidin
Schmelzpunkt: 210-215°C
$R_f$-Wert: 0,36 (Kieselgel; Methylenchlorid/Methanol = 10:1)

(263) 4-[(3-Chlor-4-fluor-phenyl)amino]-6-[exo-3-amino-8-azabicyclo[3.2.1]-8-octyl]-pyrimido[5,4-d]pyrimidin
Hergestellt aus den Verbindungen der Beispiele XXXII und XXXVI. Schmelzpunkt: 178-180°C
$R_f$-Wert: 0,41 (Kieselgel; Methylenchlorid/Methanol/konz.Ammoniak = 9:1:0,1)

(264) 4-[(3-Trifluormethoxy-phenyl)amino]-6-[trans-4-hydroxycyclohexylamino]-pyrimido[5,4-d]pyrimidin
Schmelzpunkt: 224-226°C
$R_f$-Wert: 0,41 (Kieselgel; Petrolether/Essigester/Methanol = 10:10:2)

(265) 4-[(3-Chlor-4-fluor-phenyl)amino]-6-[1-amino-2-methyl-2-propylamino]-pyrimido[5,4-d]pyrimidin
Hergestellt aus den Verbindungen der Beispiele XXXII und der Verbindung 1 des Beispiels XII und nachfolgender Umsetzung mit Trifluoressigsäure.
Schmelzpunkt: 196-201°C
$R_f$-Wert: 0,43 (Aluminiumoxid; Methylenchlorid/Methanol = 10:1)

(266) 4-[(4-Phenoxy-phenyl)amino)-6-[1-piperidinyl]-pyrimido-[5,4-d]pyrimidin
Schmelzpunkt: 175°C
$R_f$-Wert: 0,84 (Kieselgel; Petrolether/Essigester/Methanol = 10:8:2)

(267) 4-[(4-Phenoxy-phenyl)amino]-6-morpholino-pyrimido-[5,4-d]pyrimidin
Schmelzpunkt: 212°C
$R_f$-Wert: 0,69 (Kieselgel; Petrolether/Essigester/Methanol = 10:8:2)

(268) 4-[(4-Phenoxy-phenyl)amino]-6-[trans-4-hydroxy-cyclohexylamino]-pyrimido[5,4-d]pyrimidin
Schmelzpunkt: 206°C
$R_f$-Wert: 0,36 (Kieselgel; Petrolether/Essigester/Methanol = 10:8:2)

(269) 4-[(4-Benzyloxy-phenyl)amino)-6-[trans-4-hydroxy-cyclohexylamino]-pyrimido[5,4-d]pyrimidin
Schmelzpunkt: 217°C
$R_f$-Wert: 0,37 (Kieselgel; Petrolether/Essigester/Methanol = 10:8:2)

(270) 4- [(4-Phenoxy-phenyl) amino) -6- [1-methyl=4-piperidinylamino)-pyrimido[5,4-d]pyrimidin

Schmelzpunkt: 157°C
$R_f$-Wert: 0,58 (Aluminiumoxid; Methylenchlorid/Essigester/Methanol = 10:3:1)

(271) 4-[(4-Benzyloxy-phenyl)amino)-6-[1-piperidinyl)-pyrimido-[5,4-d]pyrimidin
Schmelzpunkt: 165°C
$R_f$-Wert: 0,60 (Kieselgel; Petrolether/Essigester/Methanol = 10:5:1)

(272) 4-[(4-Benzyloxy-phenyl)amino]-6-morpholino-pyrimido-[5,4-d)pyrimidin
Schmelzpunkt: 152°C
$R_f$-Wert: 0,51 (Kieselgel; Petrolether/Essigester/Methanol = 10:8:2)

(273) 4-[(4-Benzyloxy-phenyl)amino]-6-[2-hydroxy-ethylamino]-pyrimido[5,4-d)pyrimidin
Schmelzpunkt: 216°C

(274) 4-[(4-Benzyloxy-phenyl)amino]-6-[tetrahydrofurfurylamino]-pyrimido[5,4-d]pyrimidin
Schmelzpunkt: 178°C

(275) 4-[(4-Benzyloxy-phenyl)amino]-6-[trans-4-dimethylaminocyclohexylamino)-pyrimido[5,4-d]pyrimidin
Hergestellt aus den Verbindungen der Beispiele XXXII und XLVI. Schmelzpunkt: 155°C
$R_f$-Wert: 0,56 (Aluminiumoxid; Methylenchlorid/Essigester/Methanol = 10:3:1)

(276) 4-[(4-Benzyloxy-phenyl)amino]-6-[4-amino-1-piperidinyl]-pyrimido[5,4-d]pyrimidin
Schmelzpunkt: 150°C (Zers., sintert ab 136°C)
$R_f$-Wert: 0,26 (Aluminiumoxid; Methylenchlorid/Essigester/Methanol = 10:3:1)

(277) (3'S)-4-[3-Chlor-phenylamino]-6-[(3'-chinuclidinyl)-amino]pyrimido[5,4-d]pyrimidin
Schmelzpunkt: 129-132°C
$R_f$-Wert: 0,26 (Aluminiumoxid; Methylenchlorid/Essigester/Methanol = 10:3:1)

(278) 4-[(3-Chlor-4-fluor-phenyl)amino]-6-[1-dimethylaminocarbonyl-4-piperidinylamino]-pyrimido [5,4-d] pyrimidin
Hergestellt aus 4-[(3-Chlor-4-fluor-phenyl)amino]-6-[1-tert.butyloxycarbonyl-4-piperidinylamino] -pyrimido [5,4-d] pyrimidin durch Umsetzung mit Trifluoressigsäure und anschließender Um-. setzung mit Dimethylcarbamoylchlorid und Triethylamin. Schmelzpunkt: 187-188°C
$R_f$-Wert: 0,59 (Aluminiumoxid; Petrolether/Essigester/Methanol = 10:10:2)

(279) rac.-4-[(3-Chlor-4-fluor-phenyl)amino]-6-[1-methyl-3-piperidinylamino]-pyrimido[5,4-d]pyrimidin
Hergestellt aus den Verbindungen der Beispiele XXXII und XLVII, nachfolgender Umsetzung mit Trifluoressigsäure und anschließender Umsetzung mit Natriumcyanoborhydrid und Formaldehyd. Schmelzpunkt: 142-146°C (sintert ab 125)
$R_f$-Wert: 0,27 (Kieselgel; Petrolether/Essigester/Methanol = 10:10:4)

(280) 4-[(4-Benzyloxy-3-chlor-phenyl)amino]-6-[trans-4-dimethylamino-cyclohexylamino]-pyrimido[5,4-d]pyrimidin Hergestellt aus den Verbindungen der Beispiele XXXII und XLII. Schmelzpunkt: 154-156°C
$R_f$-Wert: 0,40 (Aluminiumoxid; Petrolether/Essigester/Methanol = 10:10:2)

(281) 4-[(4-Phenoxy-3-chlor-phenyl)amino]-6-[trans-4-dimethylamino-cyclohexylamino]-pyrimido[5,4-d]pyrimidin
Hergestellt aus den Verbindungen der Beispiele XXXII und XLII. Schmelzpunkt: 126-128°C
$R_f$-Wert: 0,48 (Aluminiumoxid; Petrolether/Essigester/Methanol = 10:10:0,5)

(282) (3'S)-4-[(3-Chlor-4-fluor-phenyl)amino]-6-[(3'-chinuclidinyl)amino]pyrimido[5,4-d]pyrimidin
Schmelzpunkt: 171-173°C
$R_f$-Wert: 0,38 (Aluminiumoxid; Petrolether/Essigester/Methanol = 10:10:2)
$[\alpha]_D^{20}$ = -38,2 (c = 1,0 in Methylenchlorid/Methanol = 2:1)

(283) (3'R)-4-((3-Chlor-4-fluor-phenyl)amino]-6-[(3'-chinuclidinyl)amino]pyrimido[5,4-d]pyrimidin
Schmelzpunkt: 162-164°C
$R_f$-Wert: 0,39 (Aluminiumoxid; Petrolether/Essigester/Methanol = 10:10:2)

$[a]_D^{20}$ = +36,6 (c = 1,0 in Methylenchlorid/Methanol = 2:1)

Beispiel 2

| Dragées mit 75 mg Wirksubstanz | |
|---|---|
| 1 Dragéekern enthält: | |
| Wirksubstanz | 75,0 mg |
| Calciumphosphat | 93,0 mg |
| Maisstärke | 35,5 mg |
| Polyvinylpyrrolidon | 10,0 mg |
| Hydroxypropylmethylcellulose | 15,0 mg |
| Magnesiumstearat | 1,5 mg |
| | 230,0 mg |

Herstellung:

Die Wirksubstanz wird mit Calciumphosphat, Maisstärke, Polyvinylpyrrolidon, Hydroxypropylmethylcellulose und der Hälfte der angegebenen Menge Magnesiumstearat gemischt. Auf einer Tablettiermaschine werden Preßlinge mit einem Durchmesser von ca. 13 mm hergestellt, diese werden auf einer geeigneten Maschine durch ein Sieb mit 1,5 mm-Maschenweite gerieben und mit der restlichen Menge Magnesiumstearat vermischt. Dieses Granulat wird auf einer Tablettiermaschine zu Tabletten mit der gewünschten Form gepreßt.

| | |
|---|---|
| Kerngewicht | 230 mg |
| Stempel | 9 mm, gewölbt |

Die so hergestellten Dragéekerne werden mit einem Film überzogen, der im wesentlichen aus Hydroxypropylmethylcellulose besteht. Die fertigen Filmdragées werden mit Bienenwachs geglänzt.

| | |
|---|---|
| Dragéegewicht | 245.mg. |

Beispiel 3

| Tabletten mit 100 mg Wirksubstanz | |
|---|---|
| Zusammensetzung: | |
| 1 Tablette enthält: | |
| Wirksubstanz | 100,0 mg |
| Milchzucker | 80,0 mg |
| Maisstärke | 34,0 mg |
| Polyvinylpyrrolidon | 4,0 mg |
| Magnesiumstearat | 2,0 mg |
| | 220,0 mg |

Herstellungverfahren:

Wirkstoff, Milchzucker und Stärke werden gemischt und mit einer wäßrigen Lösung des Polyvinylpyrrolidons gleichmäßig befeuchtet. Nach Siebung der feuchten Masse (2,0 mm-Maschenweite) und Trocknen im Hordentrockenschrank bei 50°C wird erneut gesiebt (1,5 mm-Maschenweite) und das Schmiermittel zugemischt. Die preßfertige Mischung wird zu Tabletten verarbeitet.

| | |
|---|---|
| Tablettengewicht | 220 mg |
| Durchmesser | 10 mm, biplan mit beidseitiger Facette und einseitiger Teilkerbe. |

Beispiel 4

| Tabletten mit 150 mg Wirksubstanz | |
|---|---|
| Zusammensetzung: | |
| 1 Tablette enthält: | |
| Wirksubstanz | 150,0 mg |
| Milchzucker pulv. | 89,0 mg |
| Maisstärke | 40,0 mg |
| Kolloide Kieselgelsäure | 10,0 mg |
| Polyvinylpyrrolidon | 10,0 mg |
| Magnesiumstearat | 1,0 mg |
| | 300,0 mg |

Herstellung:

Die mit Milchzucker, Maisstärke und Kieselsäure gemischte Wirksubstanz wird mit einer 20%igen wäßrigen Polyvinylpyrrolidonlösung befeuchtet und durch ein Sieb mit 1,5 mm-Maschenweite geschlagen.
Das bei 45°C getrocknete Granulat wird nochmals durch dasselbe Sieb gerieben und mit der angegebenen Menge Magnesiumstearat gemischt. Aus der Mischung werden Tabletten gepreßt.

| | |
|---|---|
| Tablettengewicht | 300 mg |
| Stempel | 10 mm, flach |

Beispiel 5

| Hartgelatine-Kapseln mit 150 mg Wirksubstanz | | |
|---|---|---|
| 1 Kapsel enthält: | | |
| Wirkstoff | | 150,0 mg |
| Maisstärke getr. | ca. | 180,0 mg |
| Milchzucker pulv. | ca. | 87,0 mg |
| Magnesiumstearat | | 3,0 mg |
| | ca. | 420,0 mg |

Herstellung:

Der Wirkstoff wird mit den Hilfsstoffen vermengt, durch ein Sieb von 0,75 mm-Maschenweite gegeben und in einem geeigneten Gerät homogen gemischt.
Die Endmischung wird in Hartgelatine-Kapseln der Größe 1 abgefüllt.

| | |
|---|---|
| Kapselfüllung | ca. 320 mg |
| Kapselhülle | Hartgelatine-Kapsel Größe 1. |

Beispiel 6

| Suppositorien mit 150 mg Wirksubstanz | |
|---|---|
| 1 Zäpfchen enthält: | |
| Wirkstoff | 150,0 mg |
| Polyäthylenglykol 1500 | 550,0 mg |
| Polyäthylenglykol 6000 | 460,0 mg |
| Polyoxyäthylensorbitanmonostearat | 840,0 mg |
| | 2 000,0 mg |

Herstellung:

Nach dem Aufschmelzen der Suppositorienmasse wird der Wirkstoff darin homogen verteilt und die Schmelze in vorgekühlte Formen gegossen.

Beispiel 7

| Suspension mit 50 mg Wirksubstanz | | |
|---|---|---|
| 100 ml Suspension enthalten: | | |
| Wirkstoff | | 1,00 g |
| Carboxymethylcellulose-Na-Salz | | 0,10 g |
| p-Hydroxybenzoesäuremethylester | | 0,05 g |
| p-Hydroxybenzoesäurepropylester | | 0,01 g |
| Rohrzucker | | 10,00 g |
| Glycerin | | 5,00 g |
| Sorbitlösung 70%ig | | 20,00 g |
| Aroma | | 0,30 g |
| Wasser dest. | ad | 100 ml |

Herstellung:

Dest. Wasser wird auf 70°C erhitzt. Hierin wird unter Rühren p-Hydroxybenzoesäuremethylester und -propylester sowie Glycerin und Carboxymethylcellulose-Natriumsalz gelöst. Es wird auf Raumtemperatur abgekühlt und unter Rühren der Wirkstoff zugegeben und homogen dispergiert. Nach Zugabe und Lösen des Zuckers, der Sorbitlösung und des Aromas wird die Suspension zur Entlüftung unter Rühren evakuiert.
5 ml Suspension enthalten 50 mg wirkstoff.

Beispiel 8

| Ampullen mit 10 mg Wirksubstanz | | |
|---|---|---|
| Zusammensetzung: | | |
| Wirkstoff | | 10,0 mg |
| 0,01 n Salzsäure s.q. | | |
| Aqua bidest | ad | 2,0 ml |

Herstellung:

Die Wirksubstanz wird in der erforderlichen Menge 0,01 n HCl gelöst, mit Kochsalz isotonisch gestellt, sterilfiltriert und in 2 ml Ampullen abgefüllt.

Beispiel 9

| Ampullen mit 50 mg Wirksubstanz | | |
|---|---|---|
| Zusammensetzung: | | |
| Wirkstoff<br>0,01 n Salzsäure s.q.<br>Aqua bidest | <br><br>ad | 50,0 mg<br><br>10,0 ml |

Herstellung:

Die Wirksubstanz wird in der erforderlichen Menge 0,01 n HCl gelöst, mit Kochsalz isotonisch gestellt, sterilfiltriert und in 10 ml Ampullen abgefüllt.

**Patentansprüche**

**1.** Pyrimido[5,4-d]pyrimidine der allgemeinen Formel

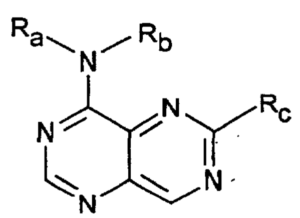

, (I)

in der

$R_a$ ein Wasserstoffatom,

$R_b$ eine 3-Chlor-phenyl-, 3-Chlor-4-fluor-phenyl-, 3-(1,1,2,2-Tetrafluorethoxy)-phenyl-, 4-Amino-3,5-dibromphenyl-, 4-Amino-3,5-dichlorphenyl-, 4-(Benzyl)phenyl-, 3-(Benzyloxy)phenyl-, 4-(Benzyloxy)phenyl-, 3-(Hydroxymethyl)-phenyl-, 4-Biphenylyl-, 3-Phenoxyphenyl-, 4-Phenoxy-phenyl-, 3-Trifluormethoxyphenyl-, 3-Cyano-phenyl-, 3-Trifluormethylphenyl-, 3,4-Difluorphenyl-, 3-Nitrophenyl-, 3-Ethinylphenyl-, 4-Amino-3-nitrophenyl-, 4-Chlor-3-nitrophenyl-, 4-(Benzyloxy)-3-chlor-phenyl-,3-Chlor-4-phenoxy-phenyl-, 3-Chlor-4-cyanophenyl- oder 4-Chlor-3-cyano-phenylgruppe

$R_c$ eine in 4-Position durch eine 4-Hydroxyphenylgruppe und zusätzlich in 2-Position durch eine Methylgruppe substituierte 1-Pyrrolidinylgruppe,

eine 1-Piperidinylgruppe, die gegebenenfalls in der 4-Position durch eine Amino-, Methylamino-, Hydroxy-, Formylamino-, Methoxycarbonylamino-, N-Methyl-N-methylsulfonyl-amino-, Aminomethyl-, Dimethylaminomethyl-, Morpholino-, 1-Pyrrolidinyl-, 1-Piperazinyl-, 1-Methyl-4-piperazinyl-, (1-Methyl-4-piperazinyl)-methyl-, 4-Dimethylamino-1-piperidinyl-, 4-Piperidinyl- oder 1-Methyl-4-piperidinylgruppe substituiert ist,

eine 4-Amino-3-methyl-1-piperidinylgruppe,

eine 4-Amino-4-methyl-1-piperidinylgruppe,

eine in der 3-Position durch eine Aminomethyl-, Aminocarbonyloder Aminocarbonylmethyl-gruppe substituierte 1-Piperidinylgruppe,

eine 1-Azacycloheptyl- oder 4-Amino-1-azacycloheptylgruppe,

eine Morpholinogruppe,

eine in 4-Position durch eine 2-Methoxyphenyl-, 3-Methoxyphenyl- oder 4-Methoxyphenylgruppe substituierte 1-Piperazinylgruppe,

eine 1-Homopiperazinyl- oder 4-Methyl-1-homopiperazinylgruppe,

eine in 3-Position durch eine Amino- oder Acetylaminogruppe substituierte 8-Aza-bicyclo[3.2.1]-8-octylgruppe oder

eine ($R_4NR_5$)-Gruppe, in der

$R_4$ ein Wasserstoffatom, eine Methyl- oder Ethylgruppe,

$R_5$ ein Wasserstoffatom,

eine Methylgruppe, die durch eine 3-Tetrahydrofuryl-, 4-Piperidinyl-, 1-Methyl-4-piperidinyl-, 1-tert.Butyloxycarbonyl-4-piperidinyl- oder 4-Chinuclidinylgruppe substituiert ist,

eine Ethylgruppe, die in 2-Position durch eine Hydroxy-, Amino-, 4-tert.Butyloxycarbonyl-1-piperazinyl- oder 4-(Morpholinocarbonyl)-1-piperazinylgruppe substituiert ist,

eine 2,2-Dimethoxy-ethylgruppe,

eine 1-Propylgruppe, die in 2-Position durch eine Aminogruppe und gegebenenfalls zusätzlich in 2-Position durch eine Methylgruppe substituiert ist,

eine 1-Propylgruppe, die in 3-Position durch eine Aminogruppe substituiert ist,

eine 2-Propylgruppe, die in 1-Position durch eine Phenoxy-, 4-Aminophenyl-, 1-Piperidinyl- oder Diethylaminogruppe substituiert ist,

eine 2-Propylgruppe, die in 1-Position durch eine Aminogruppe und zusätzlich in 2-Position durch eine Methylgruppe substituiert ist,

eine 4-Amino-butyl- oder eine 5-Aminopentylgruppe,

eine Cyclohexylgruppe, die in 4-Position durch eine Hydroxy-, Dimethylamino-, 1-Methyl-4-piperazinyl-, 1-Piperazinyl-carbonyl-, 1-Methyl-4-piperazinyl-carbonyl-, 4-Dimethylamino-1-piperidinyl-,Carboxy-, Morpholinocarbonyl-, (1-Pyrrolidinyl)carbonyl-, Methoxycarbonyl-, Aminomethyl-, Methylamino-, Methoxycarbonylamino-, 2-(Morpholinocarbonyl)ethyl- oder 2-(1-Pyrrolidinylcarbonyl)ethyl-gruppe substituiert ist,

eine Cyclohexylmethylgruppe, die im Cyclohexylteil in 4-Position durch eine Amino-, Aminomethyl- oder Benzyloxycarbonylaminogruppe oder in 3-Position durch eine Aminomethylgruppe substituiert ist,

eine 1-Methyl-3-piperidinylgruppe,

eine 4-Piperidinylgruppe, die in der 1-Position durch eine Cyano-, Methyl-, tert.Butyloxycarbonyl-, (N,N-Dimethylamino)carbonyl- oder Methoxycarbonylgruppe substituiert ist,

eine 4-Aminobenzylgruppe,

eine 3-Chinuclidinyl-, 1-Benzyl-4-(azacycloheptyl)-,

1-tert.Butyloxycarbonyl-4-(azacycloheptyl)- oder 4-(Azacycloheptyl)gruppe darstellen,

mit der Maßgabe bedeuten, daß die Verbindungen

4-[(4-Amino-3,5-dibrom-phenyl)amino]-6-[(trans-4-hydroxy-cyclohexyl)amino]-pyrimido[5,4-d]pyrimidin,

4-[(4-Amino-3,5-dichlor-phenyl)amino]-6-[(trans-4-hydroxycyclohexyl)-amino]-pyrimido[5,4-d]pyrimidin,

4-[(4-Amino-3-nitro-phenyl)amino]-6-[(trans-4-hydroxycyclohexyl)-amino]-pyrimido[5,4-d]pyrimidin,

4-[(4-Amino-3-nitrophenyl)amino]-6-(morpholino)-pyrimido[5,4-d]pyrimidin,

4-[(4-Chlor-3-nitro-phenyl)amino]-6-[(trans-4-hydroxycyclohexyl)-amino]-pyrimido[5,4-d]pyrimidin,

sowie die Verbindungen, in denen

die $R_aNR_b$-Gruppe eine (3-Chlorphenyl)amino-, (3-Nitrophenyl)-amino- oder (3-Ethinylphenyl)aminogruppe darstellt, wenn $R_c$ gleichzeitig eine Tetrahydrofurfurylamino-, Morpholino-, 1-Methyl-4-piperidinylamino-, N-(4-Hydroxy-cyclohexyl)-N-methylamino-, 4-Hydroxy-cyclohexylamino-, 4-Dimethylamino-cyclohexylamino-, trans-4-Carboxy-cyclohexylamino-, trans-4-(1-Pyrrolidinyl)carbonyl-cyclohexylamino- oder trans-4-Morpholinocarbonyl-cyclohexylaminogruppe darstellt,

und die Verbindungen, in denen

die $R_aNR_b$-Gruppe eine (3-Chlor-4-fluor-phenyl)aminogruppe darstellt, wenn $R_c$ gleichzeitig eine 1-Methyl-3-piperidinylamino-, Tetrahydrofurfurylamino-, 3-(Methoxycarbonylamino)-1-propylamino-, N-Methyl-N-(2-hydroxyethyl)amino-, 4-Amino-1-piperidinyl-, Morpholino-, 1-Methyl-4-piperidinylamino-, 4-Hydroxy-cyclohexylamino-, 4-Dimethylamino-cyclohexylamino-, N-(4-Hydroxy-cyclohexyl)-N-methylamino-, trans-4-Carboxy-cyclohexylamino-, trans-4-(2-(Morpholinocarbonyl)ethyl)-cyclohexylamino-, trans-4-(1-Pyrrolidinyl)carbonyl-cyclohexylaminooder trans-4-Morpholinocarbonyl-cyclohexylaminogruppe darstellt, ausgenommen sind,

deren Tautomeren, deren Stereoisomere und deren Salze.

**2.** Folgende Pyrimido[5,4-d]pyrimidine der allgemeinen Formel I gemäß Anspruch 1:

4-[(3-Chlor-4-fluor-phenyl)amino]-6-[4-methoxycarbonylamino-1-piperidinyl]-pyrimido[5,4-d]pyrimidin,

4-[(3-Chlor-4-fluor-phenyl)amino]-6-[(3-chinuclidinyl)amino]-pyrimido[5,4-d]pyrimidin,

4-[(3-Chlor-4-fluor-phenyl)amino]-6-[4-formylamino-1-piperidinyl]-pyrimido[5,4-d]pyrimidin,

4-[(3-Chlor-4-fluor-phenyl)amino]-6-[4-[aminomethyl)-1-piperidinyl]-pyrimido[5,4-d]pyrimidin,

4-[(3-Chlor-4-fluor-phenyl)amino]-6-[1-methoxycarbonyl-4-piperidinyl-amino]-pyrimido[5,4-d]pyrimidin,

4-[(3-Chlor-4-fluor-phenyl)amino]-6-[3-amino-propylamino]-pyrimido[5,4-d]pyrimidin,

4-[(3-Chlor-4-fluor-phenyl)amino]-6-[4-amino-benzylamino]-pyrimido[5,4-d]pyrimidin,

4-[(4-Amino-3,5-dichlor-phenyl)amino]-6-[trans-4-(morpholinocarbonyl)cyclohexylamino]-pyrimido[5,4-d]pyrimidin,

4-[(4-Amino-3,5-dichlor-phenyl)amino]-6-[trans-4-(pyrrolidinocarbonyl)cyclohexylamino]-pyrimido[5,4-d]pyrimidin,

4-[(3-Chlor-4-fluor-phenyl)amino]-6-[4-(4-piperidinyl)-1-piperidinyl]-pyrimido[5,4-d]pyrimidin,

4-[(3-Chlor-4-fluor-phenyl)amino]-6-[4-(aminomethyl)-cyclohexyl-methyl-amino]-pyrimido[5,4-d]pyrimidin,

4-[(3-Chlor-4-fluor-phenyl)amino]-6-[4-chinuclidinyl-methylamino]-pyrimido[5,4-d]pyrimidin,

4-[(3-Chlor-4-fluor-phenyl)amino]-6-[2-amino-2-methyl-1-propylamino]-pyrimido[5,4-d]pyrimidin,

4-[(3-Chlor-4-fluor-phenyl)amino]-6-[N-methyl-N-(1-methyl-4-piperidinyl)-amino]-pyrimido[5,4-d]pyrimidin,

4-[(3-Chlor-4-fluor-phenyl)amino]-6-[4-piperidinyl-methyl-amino]-pyrimido[5,4-d]pyrimidin,

4-[(3-Chlor-4-fluor-phenyl)amino]-6-[4-(morpholino)-1-piperidinyl] -pyrimido [5,4-d]pyrimidin,

4-[(3-Chlor-4-fluor-phenyl)amino]-6-[4-(1-pyrrolidinyl)-1-piperidinyl]-pyrimido[5,4-d]pyrimidin,

4-[(3-Chlor-4-fluor-phenyl)amino]-6-[3-(aminomethyl)cyclohexylmethyl-amino]-pyrimido[5,4-d]pyrimidin,

4-[(3-Chlor-4-fluor-phenyl)amino)-6-[4-(1-methyl-4-piperidinyl)-1-piperidinyl]-pyrimido[5,4-d]pyrimidin,

4-[(3-Chlor-4-fluor-phenyl)amino]-6-[4-(1-methyl-4-piperazinyl)-cyclohexylamino]-pyrimido[5,4-d]pyrimidin,

4-[(3-Chlor-4-fluor-phenyl)amino]-6-[4-hydroxy-1-piperidinyl]-pyrimido[5,4-d]pyrimidin,

4-[(3-Chlor-4-fluor-phenyl)amino]-6-[(1-cyano-4-piperidinyl)-amino]-pyrimido[5,4-d]pyrimidin,

4-[(3-Chlor-4-fluor-phenyl)amino]-6-[(1-methyl-4-piperidinyl)-methylamino]-pyrimido[5,4-d]pyrimidin,

4-[(3-Chlor-4-fluor-phenyl)amino]-6-[2-(4-(morpholinocarbonyl)-1-piperazinyl)-ethylamino]-pyrimido[5,4-d] pyrimidin,

4-[(3-Chlor-4-fluor-phenyl)amino]-6-[4-amino-1-azacycloheptyl]-pyrimido[5,4-d]pyrimidin,

4-[(3-Chlor-4-fluor-phenyl)amino]-6-[4-(azacycloheptyl)-amino]-pyrimido[5,4-d]pyrimidin,

4-[(3-Chlor-4-fluor-phenyl)amino]-6-[trans-4-aminomethyl-cyclohexylamino]-pyrimido[5,4-d]pyrimidin,

4-[(3-Chlor-4-fluor-phenyl)amino]-6-[3-aminomethyl-1-piperidinyl]-pyrimido[5,4-d]pyrimidin,

4-[(3-Chlor-4-fluor-phenyl)amino]-6-[4-(1-methyl-4-piperidinyl)-1-piperidinyl)-pyrimido[5,4-d]pyrimidin,

4-[(3-Chlor-4-fluor-phenyl)amino]-6-[4-amino-4-methyl-1-piperidinyl]-pyrimido[5,4-d]pyrimidin,

4-[(3-Chlor-4-fluor-phenyl)amino]-6-[endo-3-acetylamino-8-azabicyclo[3.2.1]-8-octyl]-pyrimido[5,4-d]pyrimidin,

4-[(3-Chlor-4-fluor-phenyl)amino]-6-[4-(4-amino-1-piperidinyl)-methyl-1-piperidinyl]-pyrimido[5,4-d]pyrimidin,

4-[(4-Benzyloxy-phenyl)amino]-6-[trans-4-dimethylamino-cyclohexylamino]-pyrimido[5,4-d]pyrimidin,

(3'S)-4-[3-Chlor-phenylamino]-6-[(3'-chinuclidinyl)amino]pyrimido[5,4-d]pyrimidin

4-[(3-Chlor-4-fluor-phenyl)amino)-6-[1-dimethylaminocarbonyl-4-piperidinylamino]-pyrimido[5,4-d]pyrimidin,

(3'S)-4-[(3-Chlor-4-fluor-phenyl)amino]-6-[(3'-chinuclidinyl)amino]pyrimido[5,4-d]pyrimidin,

(3'R)-4-[(3-Chlor-4-fluor-phenyl)amino]-6-[(3'-chinuclidinyl)-amino]pyrimido[5,4-d]pyrimidin

4-[(3-Chlor-4-fluor-phenyl)amino]-6-(1-methyl-4-piperidinylamino)-pyrimido[5,4-d]pyrimidin,

4-[(3-Chlor-4-fluor-phenyl)amino]-6-[(4-dimethylaminocyclohexyl)amino]-pyrimido[5,4-d]pyrimidin

und deren Salze.

3. Physiologisch verträgliche Salze der Verbindungen nach einem der Ansprüche 1 bis 2 mit anorganischen oder organischen Säuren oder Basen.

4. Arzneimittel, enthaltend eine Verbindung nach einem der Ansprüche 1 bis 2 oder ein physiologisch verträgliches Salz gemäß Anspruch 3 neben gegebenenfalls einem oder mehreren inerten Trägerstoffen und/oder Verdünnungsmitteln.

5. Verwendung einer Verbindung nach einem der Ansprüche 1 bis 3 zur Herstellung eines Arzneimittels, das zur Behandlung von benignen oder malignen Tumoren, insbesondere Tumoren epithelialen und neuroepithelialen Ursprungs, der Metastasierung sowie der abnormen Proliferation vaskulärer Endothelzellen (Neoangiogenese), geeignet ist.

6. Verfahren zur Herstellung eines Arzneimittels gemäß Anspruch 4, **dadurch gekennzeichnet, daß** auf nichtchemischem Wege eine Verbindung nach einem der Ansprüche 1 bis 3 in einen oder mehrere inerte Trägerstoffe und/oder Verdünnungsmittel eingearbeitet wird.

7. Verfahren zur Herstellung der Verbindungen der allgemeinen Formel I gemäß den Ansprüchen 1 bis 3, **dadurch gekennzeichnet, daß**

a) eine Verbindung der allgemeinen Formel

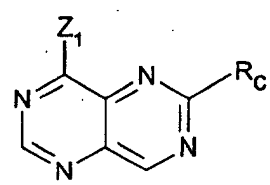

, (II)

in der

$R_c$ wie in den Ansprüchen 1 bis 3 definiert ist und
$Z_1$ eine Austrittsgruppe darstellt, mit einem Amin der allgemeinen Formel

$$H\text{-}(R_aNR_b)\ , \qquad (III)$$

in der
$R_a$ und $R_b$ wie in den Ansprüchen 1 bis 3 definiert sind; umgesetzt wird oder

b) zur Herstellung von Verbindungen der allgemeinen Formel I, in der $R_c$ einen der für $R_c$ in den Ansprüchen 1 bis 3 erwähnten. über ein Sauerstoff- oder Stickstoffatom mit dem Pyrimido[5,4-d]pyrimidin verknüpften. Reste darstellt, eine Verbindung der allgemeinen Formel

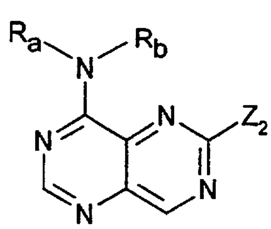

, (IV)

in der

$R_a$ und $R_b$ wie in den Ansprüchen 1 bis 3 definiert sind und $Z_2$ eine Austrittsgruppe darstellt, mit einer Verbindung der allgemeinen Formel

$$H - R_c , \qquad (V)$$

in der

$R_c$ die für $R_c$ in den Ansprüchen 1 bis 3 erwähnten über ein Sauerstoff- oder Stickstoffatom mit dem Pyrimido[5,4-d]pyrimidin verknüpften Reste darstellt, umgesetzt wird und

gewünschtenfalls eine so erhaltene Verbindung der allgemeinen Formel I, die eine Amino-, Alkylamino- oder Iminogruppe enthält, mittels Acylierung oder Sulfonylierung in eine entsprechende Acyl- oder Sulfonylverbindung der allgemeinen Formel I übergeführt wird und/oder

eine so erhaltene Verbindung der allgemeinen Formel I, die eine Amino-, Alkylamino- oder Iminogruppe enthält, mittels Alkylierung oder reduktiver Alkylierung in eine entsprechende Alkylverbindung der allgemeinen Formel I übergeführt wird und/oder

eine so erhaltene Verbindung der allgemeinen Formel I, die eine Carboxygruppe enthält, mittels Veresterung in einen entsprechenden Ester der allgemeinen Formel I übergeführt wird und/oder

eine so erhaltene Verbindung der allgemeinen Formel I, die eine Carboxy- oder Estergruppe enthält, mittels Amidierung in ein entsprechendes Amid der allgemeinen Formel I übergeführt wird und/oder

eine so erhaltene Verbindung der allgemeinen Formel I, die eine primäre oder sekundäre Hydroxygruppe enthält, mittels Oxidation in eine entsprechende Carbonylverbindung der allgemeinen Formel I übergeführt wird und/oder

erforderlichenfalls ein bei den vorstehend beschriebenen Umsetzungen verwendeter Schutzrest wieder abgespalten wird und/oder

gewünschtenfalls eine so erhaltene Verbindung der allgemeinen Formel I in ihre Stereoisomere aufgetrennt wird und/oder

eine so erhaltene Verbindung der allgemeinen Formel I, die eine Carboxygruppe enthält, mittels Veresterung in einen entsprechenden Ester der allgemeinen Formel I übergeführt wird und/oder

eine so erhaltene Verbindung der allgemeinen Formel I, die eine Carboxy- oder Estergruppe enthält, mittels Amidierung in ein entsprechendes Amid der allgemeinen Formel I übergeführt wird und/oder

eine so erhaltene Verbindung der allgemeinen Formel I, die eine primäre oder sekundäre Hydroxygruppe enthält, mittels Oxidation in eine entsprechende Carbonylverbindung der allgemeinen Formel I übergeführt wird und/oder

erforderlichenfalls ein bei den vorstehend beschriebenen Umsetzungen verwendeter Schutzrest wieder abgespalten wird und/oder

gewünschtenfalls eine so erhaltene Verbindung der allgemeinen Formel I in ihre Stereoisomere aufgetrennt wird und/oder

eine so erhaltene Verbindung der allgemeinen Formel I in ihre Salze, insbesondere für die pharmazeutische Anwendung in ihre physiologisch verträgliche Salze übergeführt wird.

## Claims

**1.** Pyrimido[5,4-d]pyrimidines of the general formula

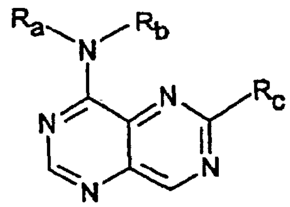

, (I)

wherein

$R_a$ denotes a hydrogen atom,

$R_b$ denotes a 3-chlorophenyl, 3-chloro-4-fluorophenyl, 3-(1,1,2,2-tetrafluoroethoxy)phenyl, 4-amino-3,5-dibromophenyl, 4-amino-3,5-dichlorophenyl, 4-(benzyl)phenyl, 3-(benzyloxy)phenyl, 4-(benzyloxy)phenyl, 3-(hydroxymethyl)phenyl, 4-biphenylyl, 3-phenoxyphenyl, 4-phenoxyphenyl, 3-trifluoromethoxyphenyl, 3-cyanophenyl, 3-trifluoromethylphenyl, 3,4-difluorophenyl, 3-nitrophenyl, 3-ethynylphenyl, 4-amino-3-nitrophenyl, 4-chloro-3-nitrophenyl, 4-(benzyloxy)-3-chlorophenyl, 3-chloro-4-phenoxyphenyl, 3-chloro-4-cyanophenyl or 4-chloro-3-cyanophenyl group,

$R_c$ denotes a 1-pyrrolidinyl group which is substituted in position 4 by a 4-hydroxyphenyl group and additionally in position 2 by a methyl group,

a 1-piperidinyl group which is optionally substituted in position 4 by an amino, methylamino, hydroxyl, formylamino, methoxycarbonylamino, N-methyl-N-methylsulphonylamino, aminomethyl, morpholino, 1-pyrrolidinyl, 1-piperazinyl, 1-methyl-4-piperazinyl, (1-methyl-4-piperazinyl)methyl, 4-dimethylamino-1-piperidinyl, 4-piperidinyl or 1-methyl-4-piperidinyl group,

a 4-amino-3-methyl-1-piperidinyl group,

a 4-amino-4-methyl-1-piperidinyl group,

a 1-piperidinyl group which is substituted in position 3 by an aminomethyl, aminocarbonyl or aminocarbonylmethyl group,

a 1-azacycloheptyl or 4-amino-1-azacycloheptyl group,

a morpholino group,

a 1-piperazinyl group which is substituted in position 4 by a 2-methoxyphenyl, 3-methoxyphenyl or 4-methoxyphenyl group,

a 1-homopiperazinyl or 4-methyl-1-homopiperazinyl group,

an 8-azabicyclo[3.2.1]-8-octyl group which is substituted in position 3 by an amino or acetylamino group or

an ($R_4NR_5$) group in which

$R_4$ represents a hydrogen atom, a methyl or ethyl group,

$R_5$ represents a hydrogen atom,

a methyl group which is substituted by a 3-tetrahydrofuryl, 4-piperidinyl, 1-methyl-4-piperidinyl, 1-tert-butyloxycarbonyl-4-piperidinyl or 4-quinuclidinyl group,

an ethyl group which is substituted in position 2 by a hydroxyl, amino, 4-tert-butyloxycarbonyl-1-piperazinyl or 4-(morpholinocarbonyl)-1-piperazinyl group,

a 2,2-dimethoxyethyl group,

a 1-propyl group which is substituted in position 2 by an amino group and optionally additionally in position 2 by a methyl group,

a 1-propyl group which is substituted in position 3 by an amino group,

a 2-propyl group which is substituted in position 1 by a phenoxy, 4-aminophenyl, 1-piperidinyl or diethylamino group,

a 2-propyl group which is substituted in position 1 by an amino group and additionally in position 2 by a methyl group,

a 4-aminobutyl or a 5-aminopentyl group,

a cyclohexyl group which is substituted in position 4 by a hydroxyl, dimethylamino, 1-methyl-4-piperazinyl, 1-piperazinylcarbonyl, 1-methyl-4-piperazinylcarbonyl, 4-dimethylamino-1-piperidinyl, carboxyl, morpholinocarbonyl,

(1-pyrrolidinyl)carbonyl, methoxycarbonyl, aminomethyl, methylamino, methoxycarbonylamino, 2-(morpholinocarbonyl)ethyl or

2-(1-pyrrolidinylcarbonyl)ethyl group,

a cyclohexylmethyl group which is substituted in the cyclohexyl moiety in position 4 by an amino, aminomethyl or benzyloxycarbonylamino group or in position 3 by an aminomethyl group,

a 1-methyl-3-piperidinyl group,

a 4-piperidinyl group which is substituted in position 1 by a cyano, methyl, tert-butyloxycarbonyl, (N,N-dimethylamino)carbonyl or methoxycarbonyl group,

a 4-aminobenzyl group,

a 3-quinuclidinyl, 1-benzyl-4-(azacycloheptyl), 1-tert-butyloxycarbonyl-4-(azacycloheptyl) or 4-(azacycloheptyl) group,

with the proviso that the compounds

4-[(4-amino-3,5-dibromophenyl)amino]-6-[(trans-4-hydroxycyclo-hexyl)amino]pyrimido[5,4-d]pyrimidine,

4-[(4-amino-3,5-dichlorophenyl)amino]-6-[(trans-4-hydroxycyclo-hexyl)aminolpyrimido[5,4-d]pyrimidine,

4-[(4-amino-3-nitrophenyl)amino]-6-[(trans-4-hydroxycyclohexyl)amino]pyrimido[5,4-d]pyrimidine,

4-[(4-amino-3-nitrophenyl)amino]-6-(morpholino)pyrimido[5,4-d]pyrimidine,

4-[(4-chloro-3-nitrophenyl)amino]-6-[(trans-4-hydroxycyclohexyl)amino]pyrimido[5,4-d]pyrimidine,

and the compounds wherein

the $R_aNR_b$ group represents a (3-chlorophenyl) amino, (3-nitrophenyl)amino or (3-ethynylphenyl)amino group if $R_c$ simultaneously represents a tetrahydrofurfurylamino, morpholino, 1-methyl-4-piperidinylamino, N-(4-hydroxycyclohexyl)-N-methylamino, 4-hydroxycyclohexylamino, 4-dimethylaminocyclohexylamino, trans-4-carboxycyclohexylamino, trans-4-(1-pyrrolidinyl)carbonylcyclohexylamino or trans-4-morpholinocarbonylcyclohexylamino group,

and the compounds wherein

the $R_aNR_b$ group represents a (3-chloro-4-fluorophenyl)amino group if $R_c$ simultaneously represents a 1-methyl-3-piperidinylamino,tetrahydrofurfurylamino,3-(methoxycarbonylamino)-1-propylamino,N-methyl-N-(2-hydroxyethyl)amino, 4-amino-1-piperidinyl, morpholino, 1-methyl-4-piperidinylamino, 4-hydroxycyclohexylamino, 4-dimethylaminocyclohexylamino, N-(4-hydroxycyclohexyl)-N-methylamino, trans-4-carboxycyclohexylamino, trans-4-(2-(morpholinocarbonyl)ethyl)cyclohexylamino, trans-4-(1-pyrrolidinyl)carbonylcyclohexylamino or trans-4-morpholinocarbonylcyclohexylamino group, are excepted,

their tautomers, their stereoisomers and their salts.

**2.** The following pyrimido[5,4-d]pyrimidines of the general formula I according to Claim 1:

4-[(3-chloro-4-fluorophenyl)amino]-6-[4-methoxycarbonylamino-1-piperidinyl]pyrimido[5,4-d]pyrimidine,

4-[(3-chloro-4-fluorophenyl)amino]-6-[(3-quinuclidinyl)-amino]pyrimido[5,4-d]pyrimidine,

4-[(3-chloro-4-fluorophenyl)amino]-6-[4-formylamino-1-piperidinyl]pyrimido[5,4-d]pyrimidine,

4-[(3-chloro-4-fluorophenyl)amino]-6-[4-(aminomethyl)-1-piperidinyl]pyrimido[5,4-d]pyrimidine,

4-[(3-chloro-4-fluorophenyl)amino]-6-[1-methoxycarbonyl-4-piperidinylamino]pyrimido[5,4-d]pyrimidine,

4-[(3-chloro-4-fluorophenyl)amino]-6-[3-aminopropylamino]pyrimido[5,4-d]pyrimidine,

4-[(3-chloro-4-fluorophenyl)amino]-6-[4-aminobenzylamino]pyrimido[5,4-d]pyrimidine,

4-[(4-amino-3,5-dichlorophenyl)amino]-6-[trans-4-(morpholinocarbonyl)cyclohexylamino]pyrimido[5,4-d]pyrimidine,

4-[(4-amino-3,5-dichlorophenyl)amino]-6-[trans-4-(pyrrolidinocarbonyl)cyclohexylamino]pyrimido[5,4-d]pyrimidine,

4-[(3-chloro-4-fluorophenyl)amino]-6-[4-(4-piperidinyl)-1-piperidinyl]pyrimido[5,4-d]pyrimidine,

4-[(3-chloro-4-fluorophenyl)amino]-6-[4-(aminomethyl)-cyclohexylmethylamino]pyrimido[5,4-d]pyrimidine,

4-[(3-chloro-4-fluorophenyl)amino]-6-[4-quinuclidinylmethylamino]pyrimido[5,4-d]pyrimidine,

4-[(3-chloro-4-fluorophenyl)amino]-6-[2-amino-2-methyl-1-propylamino]pyrimido[5,4-d)pyrimidine,

4-[(3-chloro-4-fluorophenyl)amino]-6-[N-methyl-N-(1-methyl-4-piperidinyl)amino]pyrimido[5,4-d]pyrimidine,

4-[(3-chloro-4-fluorophenyl)amino]-6-[4-piperidinylmethylamino]pyrimido[5,4-d]pyrimidine,

4-[(3-chloro-4-fluorophenyl)amino]-6-[4-(morpholino)-1-piperidinyl]pyrimido[5,4-d]pyrimidine,

4-[(3-chloro-4-fluorophenyl)amino]-6-[4-(1-pyrrolidinyl)-1-piperidinyl]pyrimido[5,4-d]pyrimidine,

4-[(3-chloro-4-fluorophenyl)amino]-6-[3-(aminomethyl)-cyclohexylmethylamino]pyrimido[5,4-d]pyrimidine,

4-[(3-chloro-4-fluorophenyl)amino]-6-[4-(1-methyl-4-piperidinyl)-1-piperidinyl]pyrimido[5,4-d]pyrimidine,

4- [ (3-chloro-4-fluorophenyl) amino] -6- (4- (1-methyl-4-piperazinyl)cyclohexylamino)pyrimido[5,4-d]pyrimidine,

4-[(3-chloro-4-fluorophenyl)amino]-6-[4-hydroxy-1-piperidinyl]pyrimido[5,4-d]pyrimidine,

4-[(3-chloro-4-fluorophenyl)amino]-6-[(1-cyano-4-piperidinyl)amino]pyrimido[5,4-d]pyrimidine,

4-[(3-chloro-4-fluorophenyl)amino]-6-[(1-methyl-4-piperidinyl)methylamino]pyrimido[5,4-d]pyrimidine,

4-[(3-chloro-4-fluorophenyl)amino]-6-[2-(4-(morpholinocarbonyl)-1-piperazinyl)ethylamino]pyrimido[5,4-d]pyrimidine,

4-[(3-chloro-4-fluorophenyl)amino]-6-[4-amino-1-azacycloheptyl]pyrimido[5,4-d]pyrimidine,

4-[(3-chloro-4-fluorophenyl)amino]-6-(4-(azacycloheptyl)-amino]pyrimido[5,4-d]pyrimidine,

4-[(3-chloro-4-fluorophenyl)amino]-6-[trans-4-aminomethyl-cyclohexylamino]pyrimido[5,4-d]pyrimidine,

4-[(3-chloro-4-fluorophenyl)amino)-6-[3-aminomethyl-1-piperidinyl]pyrimido[5,4-d]pyrimidine,

4-[(3-chloro-4-fluorophenyl)amino)-6-[4-(1-methyl-4-piperidinyl)-1-piperidinyl)pyrimido[5,4-d]pyrimidine,

4-[(3-chloro-4-fluorophenyl)amino]-6-[4-amino-4-methyl-1-piperidinyl]pyrimido[5,4-d]pyrimidine,

4-[(3-chloro-4-fluorophenyl)amino]-6-[endo-3-acetylamino-8-azabicyclo[3.2.1]-8-octyl]pyrimido[5,4-d]pyrimidine,

4-[(3-chloro-4-fluorophenyl)amino]-6-[4-(4-amino-1-piperidinyl)methyl-1-piperidinyl]pyrimido[5,4-d]pyrimidine,

4-[(4-benzyloxyphenyl)amino]-6-[trans-4-dimethylaminocyclohexylamino]pyrimido[5,4-d]pyrimidine,

(3'S)-4-[3-chlorophenylamino]-6-[(3'-quinuclidinyl)-amino]pyrimido[5,4-d]pyrimidine,

4-[(3-chloro-4-fluorophenyl)amino]-6-[1-dimethylaminocarbonyl-4-piperidinylamino]pyrimido[5,4-d]pyrimidine,

(3'S)-4-[(3-chloro-4-fluorophenyl)amino]-6-[(3'-quinuclidinyl)-amino]pyrimido[5,4-d]pyrimidine,

(3'R)-4-[(3-chloro-4-fluorophenyl)amino]-6-[(3'-quinuclidinyl)amino]pyrimido[5,4-d]pyrimidine,

4-[(3-chloro-4-fluorophenyl)amino)-6-[1-methyl-4-piperidinylamino]pyrimido[5,4-d]pyrimidine

4-[(3-Chloro-4-fluorophenyl)amino]-6-[trans-4-dimethylaminocyclohexylamino]pyrimido[5,4-d]pyrimidine

and the salts thereof.

**3.** Physiologically acceptable salts of the compounds according to at least one of Claims 1 and 2 with inorganic or organic acids or bases.

**4.** Pharmaceutical composition containing a compound according to at least one of Claims 1 and 2 or a physiologically acceptable salt according to Claim 3 optionally together with one or more inert excipients and/or diluents.

**5.** Use of a compound according to at least one of Claims 1 to 3 for preparing a pharmaceutical composition which is suitable for the treatment of benign or malignant tumours, in particular tumours of epithelial and neuroepithelial origin, of metastasis and of abnormal proliferation of vascular endothelial cells (neoangiogenesis).

**6.** Process for preparing a pharmaceutical composition according to Claim 4, **characterized in that** a compound according to at least one of Claims 1 to 3 is incorporated into one or more inert excipients and/or diluents by non-chemical means.

**7.** Process for preparing the compounds of general formula I according to Claims 1 to 3, **characterized in that**

a) a compound of the general formula

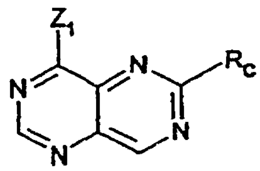

, (II)

wherein as in Claims 1 to 3, and

$R_c$ is defined as in Claims 1 to 3, and
$Z_1$ represents a leaving group, is reacted with an amine of the general formula

$$H\text{- }(R_aNR_b)\ , \qquad (III)$$

wherein
$R_a$ and $R_b$ are defined as in Claims 1 to 3, or

b) to prepare compounds of the general formula I in which $R_c$ represents one of the radicals mentioned for $R_c$ in Claims 1 to 3 linked via an oxygen or nitrogen atom to the pyrimido[5,4-d)pyrimidine, a compound of the general formula

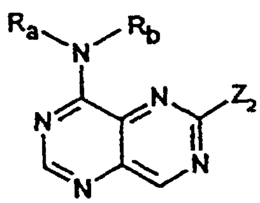

, (IV)

wherein

$R_a$ and $R_b$ are defined as in Claims 1 to 3 and

$Z_2$ represents a leaving group, is reacted with a compound of the general formula

$$H - R_c\ , \qquad (V)$$

wherein

$R_c$ represents the radicals mentioned for $R_c$ in Claims 1 to 3 linked via an oxygen or nitrogen atom to the pyrimido[5,4-d]pyrimidine, and

if required a compound of the general formula I obtained in this way and containing an amino, alkylamino or imino group is converted by acylation or sulphonylation into a corresponding acyl or sulphonyl compound of the general formula I and/or

a compound of the general formula I obtained in this way and containing an amino, alkylamino or imino group is converted by alkylation or reductive alkylation into a corresponding alkyl compound of the general formula I and/or

a compound of the general formula I obtained in this way and containing a carboxyl group is converted by esterification into a corresponding ester of the general formula I and/or

a compound of the general formula I obtained in this way and containing a carboxyl or ester group is converted by amidation into a corresponding amide of the general formula I and/or

a compound of the general formula I obtained in this way and containing a primary or secondary hydroxyl group is converted by oxidation into a corresponding carbonyl compound of the general formula I and/or

if necessary a protecting group used in the reactions described above is eliminated again and/or

if required a compound of the general formula I obtained in this way is fractioned into its stereoisomers and/or

a compound of the general formula I obtained in this way is converted into its salts, in particular for pharmaceutical use into its physiologically acceptable salts.

**Revendications**

**1.** Pyrimido[5,4-d]pyrimidines de formule générale

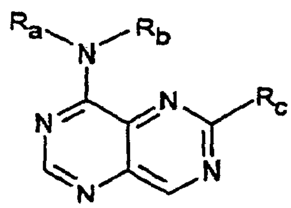

(I)

où

$R_a$ représente un atome d'hydrogène,
$R_b$ représente un groupe 3-chlorophényle, 3-chloro-4-fluorophényle, 3-(1,1,2,2-tétrafluoroéthoxy)phényle, 4-amino-3,5-dibromophényle, 4-amino-3,5-dichlorophényle, 4-(benzyl)phényle, 3-(benzyloxy)phényle, 4-(benzyloxy)-phényle, 3-(hydroxyméthyl)phényle, 4-biphénylyle, 3-phénoxyphényle, 4-phénoxyphényle, 3-trifluorométhoxyphényle, 3-cyanophényle, 3-trifluorométhylphényle, 3,4-difluorophényle, 3-nitrophényle, 3-éthynylphényle, 4-amino-3-nitrophényle, 4-chloro-3-nitrophényle, 4-(benzyloxy)-3-chlorophényle, 3-chloro-4-phénoxyphényle, 3-chloro-4-cyanophényle ou 4-chloro-3-cyanophényle,
$R_c$ représente un groupe 1-pyrrolidinyle substitué en position 4 par un groupe 4-hydroxyphényle et en outre en position 2 par un groupe méthyle,
un groupe 1-pipéridinyle qui est éventuellement substitué en position 4 par un groupe amino, méthylamino, hydroxyle, formylamino, méthoxycarbonylamino, N-méthyl-N-méthylsulfonylamino, aminométhyle, diméthylaminométhyle, morpholino, 1-pyrrolidinyle, 1-pipérazinyle, 1-méthyl-4-pipérazinyle, (1-méthyl-4-pipérazinyl) méthyle, 4-diméthylamino-1-pipéridinyle, 4-pipéridinyle ou 1-méthyl-4-pipéridinyle,
un groupe 4-amino-3-méthyl-1-pipéridinyle,
un groupe 4-amino-4-méthyl-1-pipéridinyle,
un groupe 1-pipéridinyle substitué en position 3 par un groupe aminométhyle, aminocarbonyle ou aminocarbonylméthyle,
un groupe 1-azacycloheptyle ou 4-amino-1-azacycloheptyle,
un groupe morpholino,
un groupe 1-pipérazinyle substitué en position 4 par un groupe 2-méthoxyphényle, 3-méthoxyphényle ou 4-méthoxyphényle,
un groupe 1-homopipérazinyle ou 4-méthyl-1-homopipérazinyle,
un groupe 8-aza-bicyclo[3.2.1]-8-octyle substitué en position 3 par un groupe amino ou acétylamino ou
un groupe ( $R_4NR_5$ ) où

$R_4$ représente un atome d'hydrogène, un groupe méthyle ou éthyle,
$R_5$ représente un atome d'hydrogène,
un groupe méthyle qui est substitué par un groupe 3-tétrahydrofuryle, 4-pipéridinyle, 1-méthyl-4-pipéridinyle, 1-tert.butyloxycarbonyl-4-pipéridinyle ou 4-quinuclidinyle,
un groupe éthyle qui est substitué en position 2 par un groupe hydroxyle, amino, 4-tert.butyloxycarobnyl-1-pipérazinyle ou 9-(morpholinocarbonyl)-1-pipérazinyle,
un groupe 2,2-diméthoxyéthyle,
un groupe 1-propyle qui est substitué en position 2 par un groupe amino et éventuellement en outre en position 2 par un groupe méthyle,
un groupe 1-propyle qui est substitué en position 3 par un groupe amino,
un groupe 2-propyle qui est substitué en position 1 par un groupe phénoxy, 4-aminophényle, 1-pipéridinyle ou diéthylamino,
un groupe 2-propyle qui est substitué en position 1 par un groupe amino et en outre en position 2 par un groupe méthyle,
un groupe 4-aminobutyle ou un groupe 5-aminopentyle,
un groupe cyclohexyle qui est substitué en position 4 par un groupe hydroxyle, diméthylamino, 1-méthyl-4-pipérazinyle, 1-pipérazinyl-carbonyle, 1-méthyl-4-pipérazinylcarbonyle, 4-diméthylamino-1-pipéridinyle, carboxyle, morpholinocarbonyle, (1-pyrrolidinyl)-carbonyle, méthoxycarbonyle, aminométhyle, méthylamino, méthoxycarbonylamino, 2-(morpholinocarbonyl)éthyle ou 2-(1-pyrrolidinylcarbonyl)éthyle,
un groupe cyclohexylméthyle qui est substitué dans la partie cyclohexyle en position 4 par un groupe amino-, aminométhyle ou benzyloxycarbonylamino ou en position 3 par un groupe aminométhyle,
un groupe 1-méthyl-3-pipéridinyle,
un groupe 4-pipéridinyle qui est substitué en position 1 par un groupe cyano, méthyle, tert.butyloxycarbonyle, (N,N-diméthylamino)carbonyle ou méthoxycarbonyle,
un groupe 4-aminobenzyle,
un groupe 3-quinuclidinyle, 1-benzyl-4-(azacycloheptyle), 1-tert.butyloxycarbonyl-4-(azacycloheptyle) ou 4-(azacycloheptyle),
à condition que les composés
4-[(4-amino-3,5-dibromo-phényl)amino]-6-[(trans-4-hydroxy-cyclohexyl)amino)-pyrimido[5,4-d]pyrimidine,
4-[(4-amino-3,5-dichloro-phényl)amino]-6-[(trans-4-hydroxycyclohexyl)-amino]-pyrimido[5,4-d]pyrimidine,
4-[(4-amino-3-nitro-phényl)amino]-6-[(trans-4-hydroxycyclohexyl)-amino)-pyrimido[5,4-d]pyrimidine,
4-[(4-amino-3-nitrophényl)amino]-6-(morpholino)-pyrimido[5,4-d]pyrimidine,
4-[(4-chloro-3-nitro-phényl)amino]-6-[(trans-4-hydroxycyclohexyl)-amino]-pyrimido[5,4-d]pyrimidine,

ainsi que les composés dans lesquels

le groupe $R_aNR_b$ représente un groupe (3-chlorophényl)amino, (3-nitrophényl)amino ou (3-éthynylphényl)amino soient exclus quand $R_c$ représente simultanément un groupe tétrahydrofurfurylamino, morpholino, 1-méthyl-4-pipéridinylamino, N-(4-hydroxycyclohexyle), N-méthylamino, 4-hydroxy-cyclohexylamino, 4-diméthylamino-cyclohexylamino, trans-4-carboxycyclohexylamino, trans-4-(1-pyrrolidinyl)carbonylcyclohexylamino ou trans-4-morpholinocarbonylcyclohexylamino,

et que les composés dans lesquels

le groupe $R_aNR_b$ représente un groupe (3-chloro-4-fluorophényl)amino soient exclus quand $R_c$ représente simultanément un groupe 1-méthyl-3-pipéridinylamino, tétrahydrofurfurylamino, 3-(méthoxycarbonylamino)-1-propylamino, N-méthyl-N-(2-hydroxyéthyl)amino, 4-amino-1-pipéridinyle, morpholino, 1-méthyl-4-pipéridinylamino, 4-hydroxy-cyclohexylamino, 4-diméthylamino-cyclohexylamino, N-(4-hydroxy-cyclohexyl)-N-méthylamino, trans-4-carboxy-cyclohexylamino, trans-4-(2-(morpholinocarbonyl)éthyl)-cyclohexylamino, trans-4-(1-pyrrolidinyl)carbonyl-cyclohexylamino ou trans-4-morpholinocarbonyl-cyclohexylamino,

leurs tautomères, leurs stéréoisomères et leurs sels.

**2.** Pyrimido[5,4-d]pyrimidines de formule générale I selon la revendication 1 suivantes :

4-[(3-chloro-4-fluoro-phényl)amino]-6-(4-méthoxycarbonylaminol-pipéridinyl]-pyrimido[5,4-d]pyrimidine,

4-[(3-chloro-4-fluoro-phényl)amino]-6-[(3-quinuclidinyl)amino]pyrimido[5,4-d]pyrimidine,

4-[(3-chloro-4-fluoro-phényl)amino]-6-[4-formylamino-1-pipéridinyl]-pyrimido[5,4-d]pyrimidine,

4-[(3-chloro-4-fluoro-phényl)amino]-6-[4-(aminométhyl)-1-pipéridinyl]-pyrimido[5,4-d]pyrimidine,

4-[(3-chloro-4-fluoro-phényl)amino]-6-[1-méthoxycarbonyl-4-pipéridinyl-amino]-pyrimido[5,4-d]-pyrimidine,

4-[(3-chloro-4-fluoro-phényl)amino]-6-[3-aminopropylamino]-pyrimido[5,4-d]pyrimidine,

4-[(3-chloro-4-fluoro-phényl)amino]-6-[4-aminobenzylamino]-pyrimido[5,4-d]pyrimidine,

4-[(4-amino-3,5-dichloro-phényl)amino]-6-[trans-4-(morpholinocarbonyl)cyclohexylamino]-pyrimido[5,4-d]-pyrimidine,

4-[(4-amino-3,5-dichloro-phényl)amino]-6-[trans-4-(pyrrolidinocarbonyl)cyclohexylamino]-pyrimido[5,4-d]-pyrimidine,

4-[(3-chloro-4-fluoro-phényl)amino]-6-[4-(4-pipéridinyl)-1-pipéridinyl]-pyrimido[5,4-d]pyrimidine,

4-[(3-chloro-4-fluoro-phényl)amino]-6-[4-(aminométhyl)-cyclohexyl-méthyl-amino]-pyrimido[5,4-d]pyrimidine,

4-[(3-chloro-4-fluoro-phényl)amino]-6-[4-quinuclidinylméthylamino]-pyrimido[5,4-d]pyrimidine,

4-[(3-chloro-4-fluoro-phényl)amino]-6-[2-amino-2-méthyl-1-propylamino]-pyrimido[5,4-d]pyrimidine,
4-[(3-chloro-4-fluoro-phényl)amino]-6-[N-méthyl-N-(1-méthyl-4-pipéridinyl)-amino]-pyrimido[5,4-d]pyrimidine,

4-[(3-chloro-4-fluoro-phényl)amino]-6-[4-pipéridinylméthyl-amino]-pyrimido[5,4-d]pyrimidine,

4-[(3-chloro-4-fluoro-phényl)amino]-6-[4-(morpholino)-1-pipéridinyl]-pyrimido[5,4-d]pyrimidine,

4-[(3-chloro-4-fluoro-phényl)amino]-6-[4-(1-pyrrolidinyl)-1-pipéridinyl]-pyrimido[5,4-d]pyrimidine,

4-[(3-chloro-4-fluoro-phényl)amino]-6-[3-(aminométhyl)cyclohexylméthyl-amino]-pyrimido[5,4-d]-pyrimidine,

4-[(3-chloro-4-fluoro-phényl)amino]-6-[4-(1-méthyl-4-pipéridinyl)-1-pipéridinyl]-pyrimido[5,4-d]pyrimidine,

4-[(3-chloro-4-fluoro-phényl)amino]-6-[4-(1-méthyl-4-pipérazinyl)-cyclohexylamino]-pyrimido[5,4-d]-pyrimidine,

4-[(3-chloro-4-fluoro-phényl)amino]-6-[4-hydroxy-1-pipéridinyl]pyrimido[5,4-d]pyrimidine,

4-[(3-chloro-4-fluoro-phényl)amino]-6-[(1-cyano-4-pipéridinyl)amino]-pyrimido[5,4-d]pyrimidine,

4-[(3-chloro-4-fluoro-phényl)amino]-6-[(1-méthyl-4-pipéridinyl)méthylamino]-pyrimido[5,4-d]pyrimidine,

4-[(3-chloro-4-fluoro-phényl)amino]-6-[2-(4-(morpholinocarbonyl)]-pipérazinyl)-éthylamino]-pyrimido[5,4-d] pyrimidine,

4-[(3-chloro-4-fluoro-phényl)amino]-6-[4-amino-1-azacycloheptyl]pyrimido[5,4-d]pyrimidine,

4-[(3-chloro-4-fluoro-phényl)amino]-6-[4-(azacycloheptyl)-amino]pyrimido[5,4-d]pyrimidine,

4-[(3-chloro-4-fluoro-phényl)amino]-6-[trans-4-aminométhyl-cyclohexylamino)-pyrimido[5,4-d]pyrimidine,

4-[(3-chloro-4-fluoro-phényl)amino]-6-[3-aminométhyl-1-pipéridinyl]-pyrimido[5,4-d]pyrimidine,

4-[(3-chloro-4-fluoro-phényl)amino]-6-[4-(1-méthyl-4-pipéridinyl)-1-pipéridinyl]-pyrimido[5,4-d]pyrimidine,

4-[(3-chloro-4-fluoro-phényl)amino]-6-[4-amino-4-méthyl-1-pipéridinyl]-pyrimido[5,4-d]pyrimidine,

4-[(3-chloro-4-fluoro-phényl)amino]-6-[endo-3-acetylamino-8-azabicyclo[3.2.1]-8-octyl]-pyrimido[5,4-d]pyrimidine,

4-[(3-chloro-4-fluoro-phényl)amino]-6-[4-(4-amino-1-pipéridinyl)méthyl-1-pipéridinyl]-pyrimido[5,4-d]-pyrimidine,

4-[(4-benzyloxy-phényl)amino]-6-[trans-4-diméthylaminocyclohexylamino]-pyrimido[5,4-d]pyrimidine,

(3'S)-4-[3-chloro-phénylamino]-6-[(3'-quinuclidinyl)amino]pyrimido[5,4-d]pyrimidine,

4-[(3-chloro-4-fluoro-phényl)amino]-6-[1-diméthylaminocarbonyl-4-pipéridinylamino]-pyrimido[5,4-d]-pyrimidine,

(3'S)-4-[(3-chloro-4-fluoro-phényl)amino)-6-[(3'-quinuclidinyl)amino]pyrimido[5,4-d]pyrimidine,

(3'R)-4-[(3-chloro-4-fluoro-phényl)amino]-6-[(3'-quinuclidinyl)amino]pyrimido[5,4-d]pyrimidine,

4-[(3-chloro-4-fluoro-phényl)amino]-6-(1-méthyl-4-pipéridinylamino)-pyrimido[5,4-d]pyrimidine,

4-[(3-chloro-4-fluoro-phényl)amino]-6-[(4-diméthylaminocyclohexyl)amino]-pyrimido[5,4-d]pyrimidine

et leurs sels.

**3.** Sels physiologiquement acceptables des composés selon l'une des revendications 1 à 2 avec des acides ou des bases inorganiques ou organiques.

**4.** Médicament contenant un composé selon l'une des revendications 1 à 2 ou un sel physiologiquement acceptable selon la revendication 3 outre éventuellement un ou plusieurs supports et/ou diluants inertes.

**5.** Utilisation d'un composé selon l'une des revendications 1 à 3 pour la préparation d'un médicament qui convient pour le traitement des tumeurs bénignes ou malignes, en particulier des tumeurs d'origine épithéliale et neuroépithéliale, de la formation de métastases ainsi que de la prolifération anormale des cellules endothéliales vasculaires (néoangiogenèse).

**6.** Procédé de préparation d'un médicament selon la revendication 4 **caractérisé en ce que**, par voie non chimique, un composé selon l'une des revendications 1 à 3 est incorporé dans un ou plusieurs supports et/ou diluants inertes.

**7.** Procédé de préparation des composés de formule générale I selon les revendications 1 à 3 **caractérisé en ce que**

a) un composé de formule générale

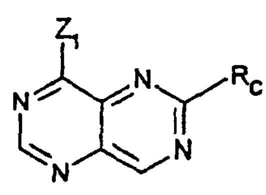

(II)

où

$R_c$ est défini comme dans les revendications 1 à 3 et
$Z_1$ représente un groupe partant est mis à réagir avec une amine de formule générale

$$H\text{-}(R_aNR_b) \qquad (III)$$

où
$R_a$ et $R_b$ sont définis comme dans les revendications 1 à 3 ou

b) pour la préparation de composés de formule générale I où $R_c$ représente l'un des restes cités pour $R_c$ dans les revendications 1 à 3 liés à la pyrimido[5,4-d]-pyrimidine par le biais d'un atome d'oxygène ou d'azote, un composé de formule générale

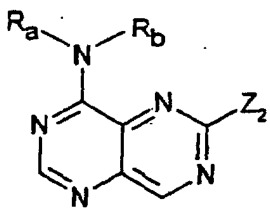

(IV)

où

$R_a$ et $R_b$ sont définis comme dans les revendications 1 à 3 et $Z_2$ représente un groupe partant, est mis à réagir avec un composé de formule générale

$$H - R_c \qquad (V)$$

où
$R_c$ représente les restes cités pour $R_c$ dans les revendications 1 à 3 liés à la pyrimido[5,4-d]pyrimidine par le biais d'un atome d'oxygène ou d'azote, et
si on le souhaite, un composé de formule générale I ainsi obtenu, qui contient un groupe amino, alkylamino ou imino, est converti par acylation ou sulfonylation en un composé acyle ou sulfonyle de formule générale I correspondant et/ou
un composé de formule générale I ainsi obtenu, qui contient un groupe amino, alkylamino ou imino, est converti par alkylation ou alkylation réductrice en un composé alkyle de formule générale I correspondant et/ou
un composé de formule générale I ainsi obtenu, qui contient un groupe carboxyle, est converti par estérification en un ester de formule générale I correspondant et/ou
un composé de formule générale I ainsi obtenu, qui contient un groupe carboxyle ou ester, est converti par amidation en un amide de formule générale I correspondant et/ou
un composé de formule générale I ainsi obtenu, qui contient un groupe hydroxyle primaire ou secondaire, est converti par oxydation en un composé carbonyle de formule générale I correspondant et/ou
si nécessaire, un reste protecteur utilisé lors des réactions décrites précédemment est clivé et/ou
si on le souhaite, un composé de formule générale I ainsi obtenu est résolu en ses stéréoisomères et/ou
un composé de formule générale I ainsi obtenu, qui contient un groupe carboxyle, est converti par estérification en un ester de formule générale I correspondant et/ou
un composé de formule générale I ainsi obtenu, qui contient un groupe carboxyle ou ester, est converti par amidation en un amide de formule générale I correspondant et/ou
un composé de formule générale I ainsi obtenu, qui contient un groupe hydroxyle primaire ou secondaire, est converti par oxydation en un composé carbonyle de formule générale I correspondant et/ou
si nécessaire, un reste protecteur utilisé lors des réactions décrites précédemment est clivé et/ou
si on le souhaite, un composé de formule générale I ainsi obtenu est résolu en ses stéréoisomères et/ou
un composé de formule générale I ainsi obtenu est converti en ses sels, en particulier pour l'utilisation pharmaceutique en ses sels physiologiquement acceptables.